(19) Europäisches Patentamt
European Patent Office
Office européen des brevets

(11) EP 1 250 323 B1

(12) **EUROPEAN PATENT SPECIFICATION**

(45) Date of publication and mention of the grant of the patent:
**01.01.2014 Bulletin 2014/01**

(21) Application number: **01921764.5**

(22) Date of filing: **17.01.2001**

(51) Int Cl.:
*C07D 207/325* (2006.01) *C07D 207/333* (2006.01)
*C07D 207/335* (2006.01) *C07D 207/34* (2006.01)
*A61K 31/404* (2006.01) *A61P 3/00* (2006.01)
*A61P 3/06* (2006.01)

(86) International application number:
**PCT/IN2001/000005**

(87) International publication number:
**WO 2001/053257 (26.07.2001 Gazette 2001/30)**

(54) **Compounds having hypolipidemic and hypocholesterolemic activities, process for their preparation and pharmaceutical compositions containing them**

Verbindungen mit hypolipidemischen und hypocholesterolemischen Wirkungen, Verfahren zu ihrer Herstellung und diese Verbindungen enthaltende pharmazeutische Zusammensetzungen

Composés hypolipidémiques et hypocholesterolémiques, leur procédé de préparation, et compositions pharmaceutiques les contenant

(84) Designated Contracting States:
**AT BE CH CY DE DK ES FI FR GB GR IE IT LI LU MC NL PT SE TR**
Designated Extension States:
**AL LT LV MK RO SI**

(30) Priority: **19.01.2000 IN 5700**

(43) Date of publication of application:
**23.10.2002 Bulletin 2002/43**

(73) Proprietor: **CADILA HEALTHCARE LIMITED**
**Ahmedebad 380 015,**
**Gujarat (IN)**

(72) Inventors:
- **LOHRAY, Braj, Bhushan**
  **R & DCtr,**
  **Zydus Tower**
  **Ahmedabad 380 015,**
  **Gujarat (IN)**
- **LOHRAY, Vidya, Bhushan,**
  **R & D Ctr.,Zydus Tower**
  **Ahmedabad 380 015,**
  **Gujarat (IN)**
- **BAROT, Vijay, Kumar, G.,**
  **R & D Ctr., Zydus Tower**
  **Ahmedabad 380 015,**
  **Gujarat (IN)**

(74) Representative: **Spencer, Michael David et al**
**Bromhead Johnson**
**Sovereign House**
**212-224 Shaftesbury Avenue**
**London WC2H 8HQ (GB)**

(56) References cited:
**EP-A- 0 041 711** **EP-A- 0 114 632**
**EP-A- 0 133 247** **EP-A- 0 163 559**
**EP-A- 0 507 696** **EP-A- 0 903 343**
**WO-A-00/23451** **WO-A-94/12165**
**WO-A-99/08501** **WO-A-99/19313**
**WO-A-99/20614** **DD-A- 86 826**
**GB-A- 2 141 709** **US-A- 3 980 089**
**US-A- 4 224 330** **US-A- 4 229 352**
**US-A- 4 232 038** **US-A- 4 235 777**
**US-A- 4 252 724** **US-A- 4 410 534**
**US-A- 4 505 920** **US-A- 5 418 242**

- **L. LEDERER ET AL.: "Synthese von Pyrrolderivaten" BERICHTE DER DEUTSCHEN CHEMISCHEN GESELLSCHAFT, vol. 18, 1885, pages 2591-9, XP002186358**
- **R. A. JONES: "The Chemistry of Pyrroles" 1977 , ACADEMIC PRESS , LONDON, NEW YORK, SAN FRANCISCO XP002186359 page 77 -page 79**
- **H. R. CHRISTEN: "Grundlagen der Organischen Chemie" 1982 , OTTO SALLE VERLAG, VERLAG SAUERLÄNDER , FRANKFURT AM MAIN, DE; AARAU, CH XP002186360 page 858 -page 859**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 250 323 B1

## Description

### Field of Invention

The present invention relates to novel hypolipidemic and hypocholesterolemic compounds, their derivatives, their analogs, their tautomeric forms, their stereoisomers, their polymorphs, their pharmaceutically acceptable salts, their pharmaceutically acceptable solvates and pharmaceutically acceptable compositions containing them.

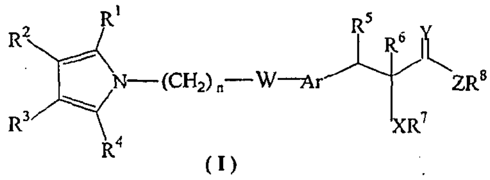

(I)

More particularly, the present invention relates to novel β-aryl-α-substituted propanoic acids of the general formula (I), their derivatives, their analogs, their tautomeric forms, their stereoisomers, their polymorphs, their pharmaceutically acceptable salts, their pharmaceutically acceptable solvates, pharmaceutically acceptable compositions containing them, their preparation, novel intermediates in their preparation and the use of these compounds in medicine.

The present invention also relates to a process for the preparation of the above said novel compounds, their derivatives, their analogs, their tautomeric forms, their stereoisomers, their polymorphs, their pharmaceutically acceptable salts, their pharmaceutically acceptable solvates, novel intermediates and pharmaceutical compositions containing them.

The compounds of general formula (I) lower total cholesterol (TC), low-density lipoproteins (LDL), triglycerides and free fatty acids and increase high-density lipoproteins. The compounds of the general formula (I) are useful in the treatment of metabolic disorders categorized, as Syndrome X. The characteristic features of Syndrome X include initial insulin resistance leading to hyperinsulinemia, dyslipidemia and impaired glucose tolerance, which can further progress to non-insulin dependent diabetes mellitus (Type 2 diabetes), characterized by hyperglycemia which may lead to diabetic complications.

The compounds of the general formula (I) are useful in the treatment and/or prophylaxis of diseases such as obesity, hyperlipidemia, hyperglycemia especially type 2 diabetes, hypertension, cardiovascular diseases especially atherosclerosis. Also, the compounds of the general formula (I) are useful in treating renal diseases which may be associated with type 2 diabetes, for example, diabetic nephropathy, glomerulonephritis glomerular sclerosis, nephrotic syndrome, hypertensive nephrosclerosis and end stage renal diseases, for the prevention or retardation of the progression of microalbuminuria to albuminuria. The compounds of the present invention are useful in the treatment and/or prophylaxis of psoriasis, polycystic ovarian syndrome, osteoporosis, inflammation and inflammatory bowel diseases, arteriosclerosis, xanthoma, pancreatic, myotonic dystrophy, disorders due to endothelial cell activation and hyperlipidemia. The compounds of the present invention are useful in the treatment of the above mentioned diseases in combination or along with one or more hypoglycemic, antihyperglycemic, hypolipidemic, hypolipoproteinemic agents such as statins, glitazones, sulfonyl urea's, fibric acid derivatives, α-glycosidase inhibitors or antioxidants.

### Background of the invention

The present invention relates to compounds represented by the general formula (I) having utility as hypocholesterolemic, hypolipidemic, hypolipoproteinemic, antiobesity and antihyperglycemic agents; methods for their preparation and methods for their use and pharmaceutical compositions containing them.

Hyperlipidemia has been recognized as the major risk factor in causing cardiovascular diseases due to atherosclerosis. Atherosclerosis and other such peripheral vascular diseases affect the quality of life of a large population in the world. During the treatment, emphasis is laid on lowering the elevated plasma cholesterol, low-density lipoprotein and plasma triglycerides as an important step to prevent occurrence of cardiovascular diseases. The details of etiology in atherosclerosis and coronary artery diseases is discussed by Ross and Glomset [New Engl. J. Med. 295, 369 - 377 (1976)]. Plasma cholesterol is generally esterified with various serum lipoproteins and numerous studies suggest an inverse relation between serum HDL-cholesterol concentration and cardiovascular disease. Numerous studies have indicated increased risk for occurrence of coronary artery diseases due to elevated LDL and VLDL-cholesterol levels [Stampfer et al. N. Engl. J. Med. 325, 373-381(1991)]. In other studies protective effects of HDL against progression of atherosclerosis are illustrated. Thus, HDL is a crucial factor in treating diseases with increased levels of cholesterol [Miller et. al. Br. Med. J. 282, 1741-1744(1981); Picardo et al., Arteriosclerosis, 6, 434-441 (1986); Macikinnon et al., J. Biol. Chem. 261, 2548-2552 (1986)].

Diabetes affects a large population and this condition is associated with a number of other complications.

Usually, the disease is associated with other disease conditions such as obesity, hyperlipidemia, hypertension and angina. It is a well-recognized fact that improper treatment can aggravate impaired glucose tolerance and insulin resistance, leading to frank diabetes. Presently, sulfonamides and biguanides along with insulin are agents of choice in treatment of diabetes. The limitations to this therapy include mild to severe hypoglycemia, which may lead to coma or in some cases may lead to death, which are mainly due to unsatisfactory glycaemic control. Recently, thiazolidinediones class of drugs having insulin-sensitizing action is being used in combination with other anti-diabetic agents, which includes troglitazone, rosiglitazone and pioglitazone. These are useful in the treatment of diabetes, and affect lipid metabolism. Patients with insulin resistance and type 2 diabetes often have raised triglycerides and low HDL-cholesterol concentrations and therefore, have greater risk of cardiovascular diseases. Thiazolidinediones are reported to have potential to induce tumor and are to cause hepatic dysfunction, which may lead to liver failure. Presently, there is need for safe and effective drug, to treat insulin resistance, diabetes and hyperlipidemia.

Obesity is another major health problem being associated with increased morbidity and morality. It is a metabolic disorder, in which excess of fat is accumulated in the body. Although, the etiology of obesity is unclear, the general feature includes excess of calorie intake than that is consumed. Various therapies such as dieting, exercise, appetite suppression, inhibition of fat absorption etc. have been used to combat obesity. However, the need for more efficient therapies to treat this abnormality is essential as obesity is closely related to several diseases such as coronary heart disease, stroke, diabetes, gout, osteroarthritis, hyperlipidemia and reduced fertility. It also leads to social and psychological problems.

Peroxisome Proliferator Activated Receptor (PPAR) is a member of the steroid/ retinoid/ thyroid hormone receptor family. PPARoc, PPARγ and PPARδ have been identified as subtypes of PPARs. PPARγ activation has been found to play a central role in initiating and regulating adipocyte differentiation [Endocrinology 135, 798-800, (1994)] and energy homeostasis, [Cell, 83, 803-812, (1995)]. During adipocyte differentiation, several highly specialized proteins are induced, which are being involved in lipid storage and metabolism. However, exact link from PPARγ activation to changes in glucose metabolism and decrease in insulin resistance in muscle has not been clear. PPARα is involved in stimulating β-oxidation of fatty acids [Trends Endocrine. Metabolism, 4, 291-296 (1993)] resulting in plasma circulating free fatty acid reduction [Current. Biol. 5, 618-621, 1995)]. Recently, role of PPARγ activation in the terminal differentiation of adipocyte precursors has been implicated in the treatment of cancer. [Cell, 79, 1147-1156 (1994); Cell, 79, 377-389 (1996); Molecular Cell, 465-470, (1998); Carcinogenesis, 1949-1953 (1998); Proc. Natl. Acad. Sci., 94, 237-241 (1997); Cancer Research, 58, 3344-3352, (1998)]. Since PPARγ is expressed in certain cells consistently, PPARγ agonists would lead to nontoxic chemotherapy.

Leptin is a protein when bound to leptin receptors is involved in sending satiety signal to the hypothalamus. Leptin resistance would therefore lead to excess food in-take, reduced energy expenditure, obesity, impaired glucose tolerance and diabetes. It has been reported that insulin sensitizers lower plasma leptin concentration [Proc. Natl. Acad. Sci. 93,5793-5796, (1996): WO 98/02159)].

PPAR α agonists have been found useful in the treatment of obesity (WO 97/36579). Dual PPAR α and γ agonists have been suggested to be useful for Syndrome X (WO 97/25042). PPAR γ agonists and HMG-CoA reductase inhibitors have exhibited synergism and indicated the usefulness of the combination in the treatment of atherosclerosis and xanthoma (EP 0753 298).

A number of compounds have been reported to be useful in the treatment of hyperlipidemia, hypercholesterolemia and hyperglycemia [US 5,306,726 US 5,985,884, US 6,054,453, EP 90 3343, PCT publications Nos. WO 91/19702, WO 94/01420, WO 94/13650, WO 95/03038, WO 95/17394, WO 96/04260, WO 96/04261, WO 96/33998, WO 97/25042, WO 97/36579, WO 98/28534, WO 99/08501, WO 99/16758, WO 99/19313, WO99/20614, WO 00/23417, WO 00/23445, WO 00/23451].

A few β-aryl-α-hydroxypropanoic acids, their derivatives, and their analogs have been reported to be useful in the treatment of hyperglycemia and hypercholesterolemia. Some of such compounds described in the prior art are outlined below :

1. U. S. patent 5,306,726 and WO 91/19702 disclose several 3-aryl-2-hydroxypropionic acid derivatives of general formulae (II) and (III) as hypolipidemic and hypoglycemic agents. Examples of these compounds are shown in the formulae (IV) and (V).

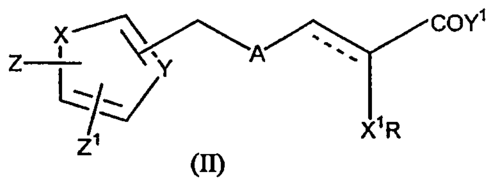
(II)

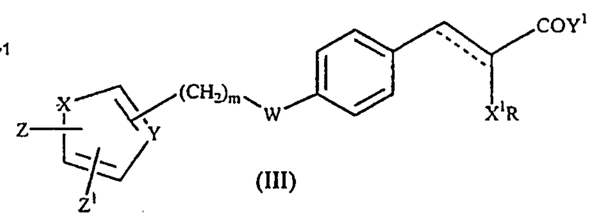
(III)

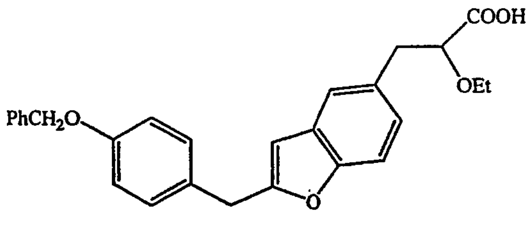

(IV)

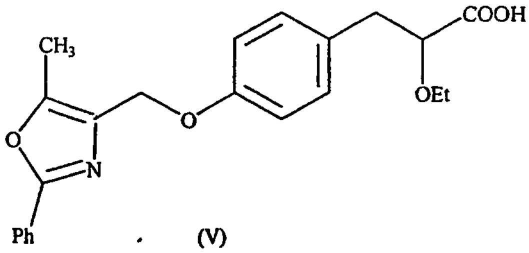

(V)

2. International patent applications, WO 95/03038 and WO 96/04260 disclose compounds of formula (VI) wherein, $R^a$ represents 2-benzoxazolyl or 2-pyridyl and $R^b$ represent $CF_3$, $CH_2OCH_3$ or $CH_3$. A typical example is (S)-3-[4-[2-[N-(2-benzoxazolyl)N-methylamino]ethoxy]phenyl]-2-(2,2,2-trifluoroethoxy)propanoic acid (VII).

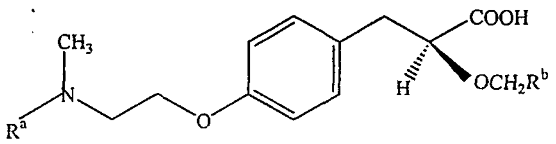

(VI)

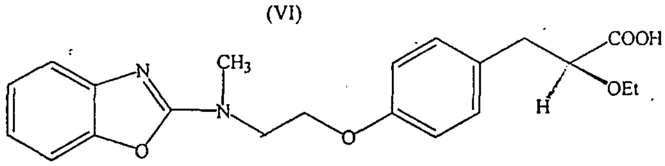

(VII)

3. International patent applications, WO 94/13650 and WO 94/01420 and WO/95/177394 disclose the compounds of general formula (VIII) wherein,

$$A^1\text{-}X\text{-}(CH_2)_n\text{-}O\text{-}A^2\text{-}A^3\text{-}YR^2 \qquad \text{(VIII)}$$

$A^1$ represents aromatic heterocycle moiety, $A^2$ represents substituted benzene ring and $A^3$ represents moiety of formula $(CH_2)_m$-CH-($OR^1$), wherein $R^1$ represents alkyl groups, m is integer of 1-5; X represents substituted or unsubstituted N; Y represents C=O or C=S, $R^2$ represents $OR^3$ where $R^3$ may be hydrogen, alkyl, aralkyl, or aryl group and n is integer of 2-6. An example of these compounds is shown in formula (IX).

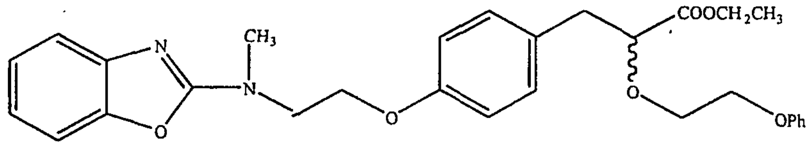

(IX)

4. International patent application, WO 00/23,445, WO 00/23,417 and WO 00/23,451 disclose cyclic compounds of the formula (X) useful in treatment of diabetes and obesity. A typical example of these compounds is shown formulae (XI) and (XII).

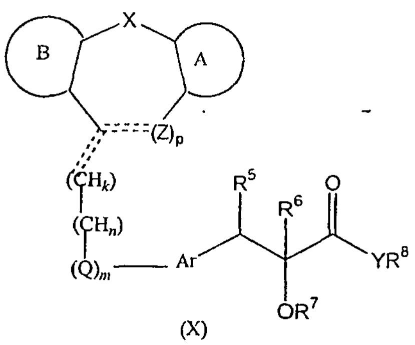

(X)

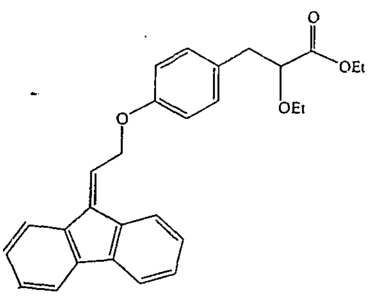

(XI)

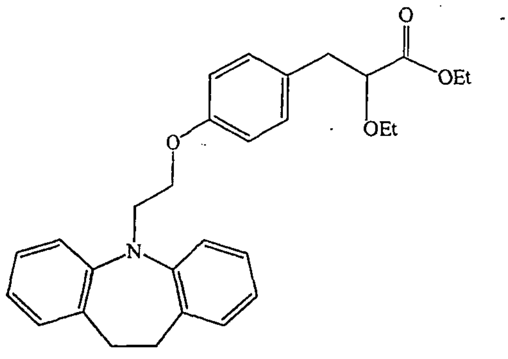

(XII)

5. WO 00/540,414, WO 99/16,758, WO 99/19,313 report compounds of general formula (XIII), (XIV), (XV) and (XVI) which reduce glucose, cholesterol and triglycerides.

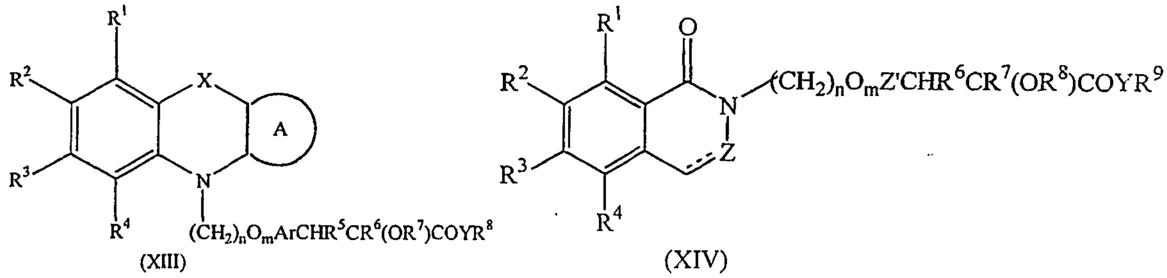

(XIII) (XIV)

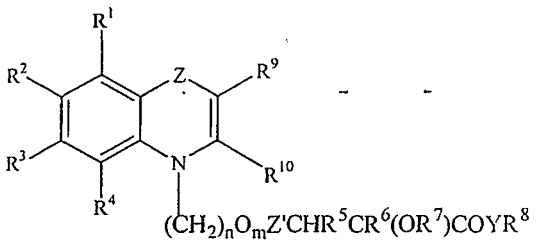

(XV)

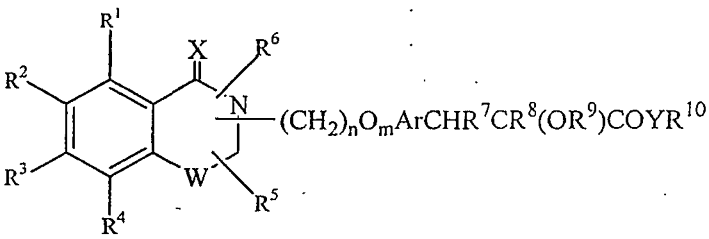

(XIV)

## Summary of the Invention

The objective of this invention is to develop novel compounds represented by the general formula (I) having utility as hypocholesterolemic, hypolipidemic, hypolipoproteinemic, anti-obesity and antihyperglycemic agents which may have additional body weight lowering effect and beneficial effect in treatment and/or prophylaxis of diseases caused by hyperlipidemia, coronary artery diseases, diseases classified under syndrome X and atherosclerosis.

The main objective of the present invention is to provide novel β-aryl-α-substituted propanoic acids represented by the general formula (I), their derivatives, their analogs, their tautomeric forms, their stereoisomers, their polymorphs, their pharmaceutically acceptable salts, their pharmaceutically acceptable solvates, and pharmaceutical compositions containing them or their mixtures.

Another objective of the present invention is to provide novel β-aryl-α-substituted propanoic acids represented by the general formula (I), their derivatives, their analogs, their tautomeric forms, their stereoisomers, their polymorphs, their pharmaceutically acceptable salts, their pharmaceutically acceptable solvates, and pharmaceutical compositions containing them or their mixtures having enhanced activities, without toxic effects or with reduced toxic effect.

Yet another objective of this invention is to provide a process for the preparation of novel β-aryl-α-substituted propanoic acids represented by the general formula (I), their derivatives, their analogs, their tautomeric forms, their stereoisomers, their polymorphs, their pharmaceutically acceptable salts, their pharmaceutically acceptable solvates.

Still another objective of the present invention is to provide pharmaceutical compositions containing compounds of the general formula (I), their derivatives, their analogs, their tautomeric forms, their stereoisomers, their polymorphs, their pharmaceutically acceptable salts, their pharmaceutically acceptable solvates or their mixtures in combination with suitable carriers, solvents, diluents and other media normally employed in preparing such compositions.

A further objective of the present invention is to provide novel intermediates and a process for their preparation.

## Detailed Description of the Invention

Accordingly the present invention is directed to a compound as described in Claim 1. Further advantageous features and embodiments are described in Claims 2 to 11.

Suitable groups represented by $R^1$, $R^2$, $R^3$ and $R^4$ may be selected from hydrogen, halogen atom such as fluorine, chlorine, bromine or iodine; perhaloalkyl particularly, perhalo($C_1$-$C_6$)alkyl such as fluoromethyl, difluoromethyl, trifluoromethyl, trifluoroethyl, fluoroethyl, difluoroethyl; hydroxy, thio, amino, nitro, cyano, formyl, amidino, guanidino groups; substituted or unsubstituted ($C_1$-$C_{12}$) alkyl group, especially, linear or branched ($C_1$-$C_8$)alkyl group, such as methyl, ethyl, *n*-propyl, *iso*-propyl, *n*-butyl, *iso*-butyl, *t*-butyl, *n*-pentyl, *iso*-pentyl, hexyl, *iso*-hexyl, heptyl, octyl; cyclo ($C_3$-$C_7$)alkyl group such as cyclopropyl, cyclobutyl, cpclopentyl, cyclohexyl, cycloheptyl, the cycloalkyl group may be substituted; cyclo($C_3$-$C_7$)alkenyl group such as cyclopentenyl, cyclohexenyl, cycloheptenyl, cycloheptadienyl, cycloheptatrienyl, the cycloalkenyl group may be substituted; ($C_1$-$C_{12}$)alkoxy, especially, ($C_1$-$C_6$)alkoxy group such as methoxy, ethoxy, propyloxy, butyloxy, *iso*-propyloxy, which may be substituted; cyclo($C_3$-$C_7$)alkoxy group such as cyclopropyloxy, cyclobutyloxy, cyclopentyloxy, cyclohexyloxy, cycloheptyloxy, the cycloalkoxy group may be substituted; aryl group such as phenyl or naphthyl, the aryl group may be substituted; aryloxy group such as phenoxy, naphthyloxy, the aryloxy group may be substituted; aralkyl group such as benzyl, phenethyl, $C_6H_5CH_2CH_2CH_2$, naphthylmethyl, the aralkyl group may be substituted and the substituted aralkyl is a group such as $CH_3C_6H_4CH_2$, Hal-$C_6H_4CH_2$, $CH_3OC_6H_4CH_2$, $CH_3OC_6H_4CH_2CH_2$; aralkoxy group such as benzyloxy, phenethyloxy, naphthylmethyloxy, phenylpropyloxy, the aralkoxy group may be substituted; heterocyclyl groups such as aziridinyl, pyrrolidinyl, morpholinyl, piperidinyl, piperazinyl, the heterocyclyl group may be substituted; heteroaryl group such as pyridyl, thienyl, furyl, pyrrolyl, oxazolyl, thiazolyl, imidazolyl, oxadiazolyl, tetrazolyl, benzopyranyl, benzofuranyl, the heteroaryl group may be substituted; heterocyclo($C_1$-$C_6$) alkyl, such as pyrrolidinealkyl, piperidinealkyl, morpholinealkyl, thiomorpholinealkyl, oxazolinealkyl, the heterocyclo ($C_1$-$C_6$)alkyl group may be substituted; heteroaralkyl group such as furanmethyl, pyridinemethyl, oxazolemethyl, oxazolethyl, the heteroaralkyl group may be substituted; heteroaryloxy, heteroaralkoxy, heteroocycloalkoxy, heterocyclylalkoxy wherein heteroaryl, heteroaralkyl, heterocycloalkyl and heterocyclylalkyl moieties are as defined earlier and may be substituted; acyl group such as acetyl, propionyl or benzoyl, the acyl group may be substituted; acyloxy group such as MeCOO, EtCOO, PhCOO which may optionally be substituted; acylamino groups such as $CH_3CONH$, $C_2H_5CONH$, $C_3H_7CONH$, $C_6H_5CONH$ which may be substituted; ($C_1$-$C_6$)monoalkylamino group such as $CH_3NH$, $C_2H_5NH$, $C_3H_7NH$, $C_6H_{13}NH$, which may be substituted; ($C_1$-$C_6$)dialkylamino group such as $N(CH_3)_2$, $CH_3(C_2H_5)N$, which may be substituted; arylamino group such as $C_6H_5HN$, $CH_3(C_6H_5)N$, $C_6H_4(CH_3)NH$, $NHC_6H_4$-Hal, which may be substituted; aralkylamino group such as $C_6H_5CH_2NH$, $C_6H_5CH_2CH_2NH$, $C_6H_5CH_2NCH_3$, which may be substituted; hydroxy($C_1$-$C_6$) alkyl which may be substituted; amino($C_1$-$C_6$)alkyl which may be substituted; mono($C_1$-$C_6$)alkylamino($C_1$-$C_6$)alkyl, di ($C_1$-$C_6$)alkylamino($C_1$-$C_6$)alkyl group which may be substituted; alkoxyalkyl group such as methoxymethyl, ethoxymethyl, methoxyethyl, ethoxyethyl, which may be substituted; aryloxyalkyl group such as $C_6H_5OCH_2$, $C_6H_5OCH_2CH_2$, naphthyloxymethyl, which may be substituted; aralkoxyalkyl group such as $C_6H_5CH_2OCH_2$, $C_6H_5CH_2OCH_2CH_2$, which may be substituted; ($C_1$-$C_6$)alkylthio, thio($C_1$-$C_6$)alkyl which may be substituted; alkoxycarbonylamino group such as $C_2H_5OCONH$, $CH_3OCONH$ which may be substituted; aryloxycarbonylamino group such as $C_6H_5OCONH$, $C_6H_5OCONCH_3$, $C_6H_5OCONC_2H_5$, $C_6H_4CH_3OCONH$, $C_6H_4(OCH_3)OCONH$ which may be substituted; aralkoxycarbonylamino group such as $C_6H_5CH_2OCONH$, $C_6H_5CH_2CH_2OCONH$, $C_6H_5CH_2OCON(CH_3)$, $C_6H_5CH_2OCON(C_2H_5)$, $C_6H_4CH_3CH_2OCONH$, $C_6H_4OCH_3CH_2OCONH$, which may be substituted; aminocarbonylamino group; ($C_1$-$C_6$)alkylaminocarbonylamino group, di($C_1$-$C_6$)alkylaminocarbonylamino group, ($C_1$-$C_6$)alkylamidino group, ($C_1$-$C_6$)alkylguanidino, di($C_1$-$C_6$)alkylguanidinogroups, hydrazino and hydroxylamino groups; carboxylic acid or its derivatives such as amides, like $CONH_2$, alkylaminocarbonyl like MeNHCO, $Me_2NCO$, EtNHCO, $Et_2NCO$, arylaminocarbonyl like PhNHCO, NapthNHCO, aralkylaminocarbonyl such as $PhCH_2NHCO$, $PhCH_2CH_2NHCO$, heteroarylaminocarbonyl and heteroaralkylamino carbonyl groups where the heteroaryl groups are as defined earlier, heterocyclylaminocarbonyl where the heterocyclyl

group is as defined earlier; carboxylic acid derivatives such as esters, wherein the ester moieties are alkoxycarbonyl such as methoxycarbonyl or ethoxycarbonyl, which may be substituted; aryloxycarbonyl group such as unsubstituted or substituted phenoxycarbonyl, naphthyloxycarbonyl; aralkoxycarbonyl group such as benzyloxycarbonyl, phenethyloxycarbonyl, naphthylmethoxycarbonyl, heteroaryloxycarbonyl, heteroaralkoxycarbonyl, wherein the heteroaryl group is as defined earlier, heterocycloxycarbonyl where heterocycle is as defined earlier and these carboxylic acid derivatives may be substituted; sulfonic acid or its derivatives such as $SO_2NH_2$, $SO_2NHMe$, $SO_2NMe_2$, $SO_2NHCF_3$, $SO_2NHCO(C_1-C_6)$alkyl, $SO_2NHCO$aryl where the aryl group is as defined earlier and the sulfonic acid derivatives may be substituted; phosphonic acid and its derivatives such as $P(O)(OH)_2$, $P(O)(O\ C_1-C_6\ alkyl)_2$, $P(O)(O\ aryl)_2$.

When the groups represented by $R^1$, $R^2$, $R^3$ and $R^4$ are substituted, the substituents are selected from halogen, hydroxy, nitro or unsubstituted or substituted groups selected from alkyl, cycloalkyl, alkoxy, cycloalkoxy, aryl, aralkyl, aralkoxyalkyl, heterocyclyl, heteroaryl, heteroaralkyl, acyl, acyloxy, hydroxyalkyl, amino, acylamino, arylamino, aminoalkyl, aryloxy, aralkoxy, alkylamino, alkoxyalkyl, alkylthio, thioalkyl groups, carboxylic acid or its derivatives, or sulfonic acid or its derivatives or phosphonic acid or its derivatives.

Preferably the substituents on the pyrrole namely, $R^1$ to $R^4$ represent halogen atom such as fluorine, chlorine, bromine; hydroxy group, optionally halogenated groups selected from alkyl group such as methyl, ethyl, n-propyl, isopropyl or n-butyl; cycloalkyl group such as cyclopropyl, cyclobutyl, cyclopentyl, cyclohexyl which may be substituted ; aryl group such as phenyl which may be substituted; aralkyl group such as benzyl which may be substituted; $(C_1-C_3)$ alkoxy, benzyloxy, acyl or acyloxy groups; heterorayl such as thienyl, pyrrolyl, furyl, pyridyl, pyrimidinyl, imidazolyl, indolyl, oxazolyl, groups which may be substituted; carboxylic acid and its derivatives.

Suitable cyclic structures formed by the two adjacent groups $R^2$ and $R^3$ together with the carbon atoms to which they are attached contain 5 to 6 ring atoms which may optionally contain one or more heteroatoms selected from oxygen, nitrogen or sulfur and optionally contain one or more double bonds. The cyclic structure may be optionally substituted phenyl, pyridyl, furanyl, thienyl, pyrrolyl, imidazolyl, pyrimidinyl, pyrazinyl. Suitable substituents on the cyclic structure formed by $R^2$ and $R^3$ together with the adjacent carbon atoms to which they are attached include oxo, hydroxy, halogen atom such as chlorine, bromine and iodine; nitro, cyano, amino, formyl, $(C_1-C_3)$alkyl, $(C_1-C_3)$alkoxy, thioallcyl, alkylthio phenyl or benzyl groups.

n is an integer ranging from 1-8. It is preferred that n be 1 to 4.

Suitable groups represented by Ar include substituted or unsubstituted groups selected from divalent phenylene, naphthylene, pyridyl, quinolinyl, benzofuryl, dihydrobenzofuryl, benzopyranyl, indolyl, indolinyl, azaindolyl, azaindolinyl, pyrazolyl, benzothiazolyl, benzoxazolyl. The substituents on the group represented by Ar may be selected from substituted or unsubstituted linear or branched $(C_1-C_6)$alkyl, $(C_1-C_3)$alkoxy, halogen, haloalkyl, haloalkoxy, acyl, amino, acylamino, thio or carboxylic or sulfonic acids and their derivatives or phosphonic acid and their derivatives.

It is preferred that Ar represents substituted or unsubstituted divalent phenylene, naphthylene, benzofuryl, indolyl, indolinyl, quinolinyl, azaindolyl, azaindolinyl, benzothiazolyl or benzoxazolyl groups.

It is more preferred that Ar is represented by divalent phenylene or naphthylene, which may be unsubstituted or substituted by halogen, methyl, halomethyl, methoxy or halomethoxy groups.

Suitable $R^5$ includes hydrogen, lower alkyl groups such as methyl, ethyl or propyl; hydroxy, $(C_1-C_3)$alkoxy, halogen atom such as fluorine, chlorine, bromine, or iodine; aralkyl such as benzyl, phenethyl, which may be unsubstituted or substituted or $R^5$ together with $R^6$ represent a bond.

Suitable $R^6$ may be hydrogen, lower alkyl groups such as methyl, ethyl or propyl; hydroxy, $(C_1-C_3)$alkoxy; halogen atom such as fluorine, chlorine, bromine, iodine; acyl group such as linear or branched $(C_2-C_{10})$ acyl group such as acetyl, propanoyl, butanoyl, pentanoyl, benzoyl; aralkyl such as benzyl, phenethyl, which may be unsubstituted or substituted or together with $R^5$ forms a bond.

When $R^5$ or $R^6$ represent substituted aralkyl, the preferred substitutents are hydroxy, halogen, alkyl and alkoxy.

It is preferred that $R^5$ or $R^6$ represent hydrogen atom or $R^5$ and $R^6$ together represent a bond.

Suitable groups represented by $R^7$ may be selected from hydrogen, substituted or unsubstituted, linear or branched $(C_1-C_{16})$alkyl, preferably $(C_1-C_{12})$alkyl group such as methyl, ethyl, *n*-propyl, *iso*-propyl, *n*-butyl, *iso*-butyl, pentyl, hexyl, octyl; $(C_3-C_7)$cycloalkyl group such as cyclopropyl, cyclobutyl, cyclopentyl, cyclohexyl, the cycloalkyl group may be substituted; aryl group such as phenyl, naphthyl, the aryl group may be substituted; heteroaryl group such as pyridyl, thienyl, furyl, the heteroaryl group may be substituted; heteroaralkyl group such as furanemethyl pyridinemethyl, oxazolemethyl, oxazolethyl, the heteroaralkyl group may be substituted; aralkyl group wherein the alkyl moiety may contain $C_1-C_6$ atoms such as benzyl and phenethyl etc, wherein the aryl moiety may be substituted; heterocyclyl group such as aziridinyl, pyrrolidinyl, piperidinyl, the heterocyclyl group may be substituted; $(C_1-C_6)$alkoxy$(C_1-C_6)$alkyl group such as methoxymethyl, ethoxymethyl, methoxyethyl, ethoxypropyl, the alkoxyalkyl group may be substituted; substituted or unsubstituted, linear or branched $(C_2-C_{16})$acyl group such as acetyl, propanoyl, butanoyl, benzoyl, octanoyl, decanoyl; $(C_1-C_6)$alkoxycarbonyl, the alkyl group may be substituted; aryloxycarbonyl such as phenoxycarbonyl, naphthyloxycarbonyl, the aryl group may be substituted; $(C_1-C_6)$alkylaminocarbonyl, the alkyl group may be substituted; arylaminocarbonyl such as PhNHCO, or naphthylaminocarbonyl, the aryl moiety may be substituted. The substituents may be selected

from the group consisting of halogen, hydroxy, nitro, or unsubstituted/substituted groups selected from alkyl, cycloalkyl, alkoxy, cycloalkoxy, aryl, aralkyl, aralkoxyalkyl, heterocyclyl, heteroaryl, heteroaralkyl, acyl, acyloxy, hydroxyalkyl, amino, acylamino, arylamino, aminoalkyl, aryloxy, alkoxycarbonyl, alkylamino, alkoxyalkyl, alkylthio, thioalkyl groups, carboxylic acid or its derivatives, or sulfonic acid or its derivatives.

Suitable groups represented by $R^8$ may be selected from hydrogen, substituted or unsubstituted, linear or branched $(C_1\text{-}C_{16})$alkyl, preferably $(C_1\text{-}C_{12})$alkyl group such as methyl, ethyl, *n*-propyl, *iso*-propyl, *n*-butyl, *iso*-butyl, pentyl, hexyl, octyl; $(C_3\text{-}C_7)$cycloalkyl such as cyclopropyl, cyclopentyl, cyclohexyl, the cycloalkyl group may be substituted; aryl group such as phenyl, naphthyl, the aryl group may be substituted; heteroaryl group such as pyridyl, thienyl, furyl, the heteroaryl group may be substituted; heteroaralkyl group such as furanemethyl, pyridinemethyl, oxazolemethyl, oxazolethyl, the heteroaralkyl group may be substituted; aralkyl group such as benzyl and phenethyl, the aralkyl group may be substituted; heterocyclyl group such as aziridinyl, pyrrolidinyl, piperidinyl, the heterocyclyl group may be substituted. The substituents on $R^8$ may be selected from halogen, hydroxy, nitro or unsubstituted or substituted groups selected from alkyl, cycloalkyl, alkoxy, cycloalkoxy, aryl, aralkyl, aralkoxyalkyl, heterocyclyl, heteroaryl, heteroaralkyl, acyl, acyloxy, hydroxyalkyl, amino, acylamino, arylamino, aminoalkyl aryloxy, aralkoxy, alkoxycarbonyl, alkylamino, alkoxyalkyl, alkylthio, thioalkyl groups, carboxylic acid or its derivatives, or sulfonic acid or its derivatives.

Z represents oxygen or $NR^{10}$.

Suitable groups represented by $R^{10}$ may be selected from hydrogen, substituted or unsubstituted, linear or branched $(C_1\text{-}C_{16})$alkyl, preferably $(C_1\text{-}C_{12})$alkyl; hydroxy$(C_1\text{-}C_6)$alkyl; aryl group such as phenyl, naphthyl; aralkyl group such as benzyl and phenethyl; heterocyclyl group such as aziridinyl, pyrrolidinyl, piperidinyl; heteroaryl group such as pyridyl, thienyl, furyl; or heteroaralkyl group such as furanemethyl, pyridinemethyl, oxazolemethyl, oxazolethyl.

The cyclic structure formed by $R^8$ and $R^{10}$ together with the carbon atoms to which they are attached may be a substituted or unsubstituted 5 or 6 membered cyclic structure containing carbon atoms which may optionally contain one or two heteroatoms selected from oxygen, nitrogen or sulfur. The cyclic structure may contain one or more double bonds.

Suitable ring structures formed by $R^8$ and $R^{10}$ together may be selected from pyrrolidinyl, piperidinyl, morpholinyl, piperazinyl, oxazolinyl, diazolinyl. Suitable substituents on the cyclic structure formed by $R^8$ and $R^{10}$ taken together may be selected from halogen, hydroxy, alkyl, oxo, aralkyl.

For any $R^1$, $R^2$, $R^3$, $R^4$, $R^5$, $R^6$, $R^7$, $R^8$, $R^9$, $R^{10}$ and Ar that may be substituted, the substituents are as defined anywhere in this specification.

Suitable n is an integer ranging from 1 to 6, preferably n represents an integer 2 to 4.

Pharmaceutically acceptable salts forming part of this invention are intended to define salts of the carboxylic acid moiety such as alkali metal salts like Li, Na, and K salts; alkaline earth metal salts like Ca and Mg salts; salts of organic bases such as lysine, arginine, guanidine, diethanolamine, choline, tromethamine and the like; ammonium or substituted ammonium salts and aluminum salts. Salts may be acid addition salts which defines sulfates, nitrates, phosphates, perchlorates, borates, hydrohalides, acetates, tartrates, maleates, citrates, succinates, palmoates, methanesulfonates, benzoates, salicylates, hydroxynaphthoates, benzenesulfonates, ascorbates, glycerophosphates, ketoglutarates. Pharmaceutically acceptable solvates may be hydrates or comprising other solvents of crystallization such as alcohols.

Particularly useful compounds according to the present invention includes:

(±) Ethyl 3-{4-[2-(pyrrol-1-yl)ethoxy]phenyl}-2-ethoxypropanoate
(+) Ethyl 3-{4-[2-(pyrrol-1-yl)ethoxy]phenyl}-2-ethoxypropanoate
(-) Ethyl 3-{4-[2-(pyrrol-1-yl)ethoxy]phenyl}-2-ethoxypropanoate
(±) Ethyl 3-{4-[2-(2,5-dimethylpyrrol-1-yl)ethoxy]phenyl}-2-ethoxypropanoate
(+) Ethyl 3-{4-[2-(2,5-dimethylpyrrol-1-yl)ethoxy]phenyl}-2-ethoxypropanoate
(-) Ethyl 3-{4-[2-(2,5-dimethylpyrrol-1-yl)ethoxy]phenyl}-2-ethoxypropanoate
(±) Ethyl 3-{4-[2-(2,5-diisopropyl-3-phenylpyrrol-1-yl)ethoxy]phenyl}-2-ethoxypropanoate
(+) Ethyl 3-{4-[2-(2,5-diisopropyl-3-phenylpyrrol-1-yl)ethoxy]phenyl}-2-ethoxypropanoate
(-) Ethyl 3-{4-[2-(2,5-diisopropyl-3-phenylpyrrol-1-yl)ethoxy]phenyl}-2-ethoxypropanoate
(±) Ethyl 3-(4-{2-[2-isopropyl-5-(4-methoxyphenyl)pyrrol-1-yl]ethoxy}phenyl)- 2-ethoxypropanoate
(+) Ethyl 3-(4-{2-[2-isopropyl-5-(4-methoxyphenyl)pyrrol-1-yl]ethoxy}phenyl)- 2-ethoxypropanoate
(-) Ethyl 3-(4-{2-[2-isopropyl-5-(4-methoxyphenyl)pyrrol-1-yl]ethoxy}phenyl)- 2-ethoxypropanoate
(±) Ethyl 3-(4-{2-[2-(4-fluorophenyl)-5-isopropylpyrrol-1-yl]ethoxy}phenyl)- 2-ethoxypropanoate
(+) Ethyl 3-(4-{2-[2-(4-fluorophenyl)-5-isopropylpyrrol-1-yl]ethoxy}phenyl)- 2-ethoxypropanoate
(-) Ethyl 3-(4-{2-[2-(4-fluorophenyl)-5-isopropylpyrrol-1-yl]ethoxy}phenyl)- 2-ethoxypropanoate
(±) Ethyl 3-(4-{2-[2-(4-fluorophenyl)-5-isopropyl-3-phenylpyrrol-1-yl]ethoxy}phenyl)- 2-ethoxypropanoate
(+) Ethyl 3-(4-{2-[2-(4-fluorophenyl)-5-isopropyl-3-phenylpyrrol-1-yl]ethoxy}phenyl)- 2-ethoxypropanoate
(-) Ethyl 3-(4-{2-[2-(4-fluorophenyl)-5-isopropyl-3-phenylpyrrol-1-yl]ethoxy}phenyl)- 2-ethoxypropanoate

(±) Ethyl 3-(4-{2-[2-(4-fluorophenyl)-5-phenylpyrrol-1-yl]ethoxy}phenyl)- 2-ethoxypropanoate
(+) Ethyl 3-(4-{2-[2-(4-fluorophenyl)-5-phenylpyrrol-1-yl]ethoxy}phenyl)- 2-ethoxypropanoate
(-) Ethyl 3-(4-{2-[2-(4-fluorophenyl)-5-phenylpyrrol-1-yl]ethoxy}phenyl)- 2-ethoxypropanoate
(±) Ethyl 3-[4-[2-(2-phenyl-3-carbethoxy-5-(4-fluorophenyl)pyrrol-1-yl)ethoxy]phenyl-2-ethoxypropanoate
(+) Ethyl 3-[4-[2-(2-phenyl-3-carbethoxy-5-(4-fluorophenyl)pyrrol-1-yl)ethoxy]phenyl-2-ethoxypropanoate
(-) Ethyl 3-[4-[2-(2-phenyl-3-carbethoxy-5-(4-fluorophenyl)pyrrol-1-yl)ethoxy]phenyl-2-ethoxypropanoate
(±) Ethyl 3-(4-{2-[2-(4-fluorophenyl)-5-isopropyl-3-phenyl-4-phenylcarbamoylpyrrol-1-yl]ethoxy}phenyl)- 2-ethoxypropanoate
(+) Ethyl 3-(4-{2-[2-(4-fluorophenyl)-5-isopropyl-3-phenyl-4-phenylcarbamoylpyrrol-1-yl]ethoxy}phenyl)- 2-ethoxypropanoate
(-) Ethyl 3-(4-{2-[2-(4-fluorophenyl)-5-isopropyl-3-phenyl-4-phenylcarbamoylpyrrol-1-yl]ethoxy}phenyl)-2-ethoxypropanoate
(±) Ethyl 3-(4-{3-[2-(4-fluorophenyl)-5-isopropyl-3-phenyl-4-phenylcarbamoylpyrrol-1-yl]propoxy}phenyl)- 2-ethoxypropanoate
(+) Ethyl 3-(4-{3-[2-(4-fluorophenyl)-5-isopropyl-3-phenyl-4-phenylcarbamoylpyrrol-1-yl]propoxy}phenyl)-2-ethoxypropanoate
(-) Ethyl 3-(4-{3-[2-(4-fluorophenyl)-5-isopropyl-3-phenyl-4-phenylcarbamoylpyrrol-1-yl]propoxy}phenyl)-2-ethoxypropanoate
(±) Ethyl 3-{4-[2-(2-isopropyl-5-methylpyrrol-1-yl)ethoxy]phenyl}-2-ethoxypropanoate (+) Ethyl 3-{4-[2-(2-isopropyl-5-methylpyrrol-1-yl)ethoxy]phenyl}-2-ethoxypropanoate
(-) Ethyl 3-{4-[2-(2-isopropyl-5-methylpyrrol-1-yl)ethoxy]phenyl}-2-ethoxypropanoate
(±) 3-{4-[2-(pyrrol-1-yl)ethoxy]phenyl}-2-ethoxypropanoic acid and its pharmaceutically acceptable salts
(+) 3-{4-[2-(pyrrol-1-yl)ethoxy]phenyl}-2-ethoxypropanoic acid and its pharmaceutically acceptable salts
(-) 3-{4-[2-(pyrrol-1-yl)ethoxy]phenyl}-2-ethoxypropanoic acid and its pharmaceutically acceptable salts
(±) 3-{4-[2-(2,5-dimethylpyrrol-1-yl)ethoxy]phenyl}-2-etboxypropanoic acid and its pharmaceutically acceptable salts
(+) 3-{4-[2-(2,5-dimethylpyrrol-1-yl)ethoxy]phenyl}-2-ethoxypropanoic acid and its pharmaceutically acceptable salts
(-) 3-{4-[2-(2,5-dimethylpyrrol-1-yl)ethoxy]phenyl}-2-ethoxypropanoic acid and its pharmaceutically acceptable salts
(±) 3-{4-[2-(2,5-diisopropyl-3-phenylpyrrol-1-yl)ethoxy]phenyl}-2-ethoxypropanoic acid and its pharmaceutically acceptable salts
(+) 3-{4-[2-(2,5-diisopropyl-3-phenylpyrrol-1-yl)ethoxy]phenyl}-2-ethoxypropanoic acid and its pharmaceutically acceptable salts
(-) 3-{4-[2-(2,5-diisopropyl-3-phenylpyrrol-1-yl)ethoxy]phenyl}-2-ethoxypropanoic acid and its pharmaceutically acceptable salts
(±) 3-(4-{2-[2-isopropyl-5-(4-methoxyphenyl)pyrrol-1-yl]ethoxy}phenyl)-2-ethoxypropanoic acid and its pharmaceutically acceptable salts
(+) 3-(4-{2-[2-isopropyl-5-(4-methoxyphenyl)pyrrol-1-yl]ethoxy}phenyl)-2-ethoxypropanoic acid and its pharmaceutically acceptable salts
(-) 3-(4-{2-[2-isopropyl-5-(4-methoxyphenyl)pyrrol-1-yl]ethoxy}phenyl)-2-ethoxypropanoic acid and its pharmaceutically acceptable salts
(±) 3-(4-{2-[2-(4-fluorophenyl)-5-isopropylpyrrol-1-yl]ethoxy}phenyl)-2-ethoxypropanoic acid and its pharmaceutically acceptable salts
(+) 3-(4-{2-[2-(4-fluorophenyl)-5-isopropylpyrrol-1-yl]ethoxy}phenyl)-2-ethoxypropanoic acid and its pharmaceutically acceptable salts
(-) 3-(4-{2-[2-(4-fluorophenyl)-5-isopropylpyrrol-1-yl]ethoxy}phenyl)-2-ethoxypropanoic acid and its pharmaceutically acceptable salts
(±) 3-(4-(2-[2-(4-fluorophenyl)-5-isopropyl-3-phenylpyrrol-1-yl]ethoxy)phenyl)-2-ethoxypropanoic acid and its pharmaceutically acceptable salts
(+) 3-(4-(2-[2-(4-fluorophenyl)-5-isopropyl-3-phenylpyrrol-1-yl]ethoxy)phenyl)-2-ethoxypropanoic acid and its pharmaceutically acceptable salts
(-) 3-(4-(2-[2-(4-fluorophenyl)-5-isopropyl-3-phenylpyrrol-1-yl]ethoxy)phenyl)-2-ethoxypropanoic acid and its pharmaceutically acceptable salts
(±) 3-(4-(2-[2-(4-fluorophenyl)-5-phenylpyrrol-1-yl]ethoxy)phenyl)-2-ethoxypropanoic acid and its pharmaceutically acceptable salts
(+) 3-(4-{2-[2-(4-fluorophenyl)-5-phenylpyrrol-1-yl]lethoxy}phenyl)-2-ethoxypropanoic acid and its pharmaceutically acceptable salts
(-) 3-(4-{2-[2-(4-fluorophenyl)-5-phenylpyrrol-1-yl]ethoxy}phenyl)-2-ethoxypropanoic acid and its pharmaceutically acceptable salts

(±) 3-[4-[2-(2-phenyl-3-carbethoxy-5-(4-fluorophenyl)pyrrol-1-yl)ethoxy]phenyl]- 2-ethoxypropanoic and its pharmaceutically acceptable salts

(+) 3-[4-[2-(2-phenyl-3-carbethoxy-5-(4-ffuorophenyl)pyrrol-1-yl)ethoxy]phenyl]- 2-ethoxypropanoic and its pharmaceutically acceptable salts

(-) 3-[4-[2-(2-phenyl-3-carbethoxy-5-(4-fluorophenyl)pyrrol-1-yl)ethoxy]phenyl]- 2-ethoxypropanoic and its pharmaceutically acceptable salts

(±) 3-(4-{2-[2-(4-fluorophenyl)-5-isopropyl-3-phenyl-4-phenylcarbamoylpyrrol-1-yl]ethoxy}phenyl)-2-ethoxypropanoic acid ethyl ester and its pharmaceutically acceptable salt thereof

(+) 3-(4-{2-[2-(4-fluorophenyl)-5-isopropyl-3-phenyl-4-phenylcarbamoylpyrrol-1-yl]ethoxy}phenyl)-2-ethoxypropanoic acid ethyl ester and its pharmaceutically acceptable salts

(-) 3-(4-{2-[2-(4-fluorophenyl)-5-isopropyl-3-phenyl-4-phenylcarbamoylpyrrol-1-yl]ethoxy}phenyl)-2-ethoxypropanoic acid ethyl ester and its pharmaceutically acceptable salts

(±) 3-(4-{3-[2-(4-fluorophenyl)-5-isopropyl-3-phenyl-4-phenylcarbamoylpyrrol-1-yl]propoxy}phenyl) -2-ethoxypropanoic acid ethyl ester and its pharmaceutically acceptable salts

(+) 3-(4-{3-[2-(4-fluorophenyl)-5-isopropyl-3-phenyl-4-phenylcarbamoylpyrrol-1-yl]propoxy}phenyl)-2-ethoxypropanoic acid ethyl ester and its pharmaceutically acceptable salts

(-) 3-(4-{3-[2-(4-fluorophenyl)-5-isopropyl-3-phenyl-4-phenylcarbamoylpyrrol-1-yl]propoxy}phenyl) -2-ethoxypropanoic acid ethyl ester and its pharmaceutically acceptable salts.

(±) 3-{4-[2-(2-isopropyl-5-methylpyrrol-1-yl)ethoxy]phenyl}-2-ethoxypropanoic acid and its pharmaceutically acceptable salts

(+) 3-{4-[2-(2-isopropyl-5-methylpyrrol-1-yl)ethoxy]phenyl}-2-ethoxypropanoic acid and its pharmaceutically acceptable salts

and (-) 3-{4-[2-(2-isopropyl-5-methylpyrrol-1-yl)ethoxy]phenyl}-2-ethoxypropanoic acid and its pharmaceutically acceptable salts

The present invention provides process for the preparation of novel compounds of this invention of the general formula (I), their tautomeric forms, their derivatives, their analogs, their stereoisomers, their polymorphs, their pharmaceutically acceptable salts and their pharmaceutically acceptable solvates wherein $R^1$, $R^2$, $R^3$, $R^4$, $R^5$, $R^6$, $R^7$, $R^8$, W, X, Y, Z, Ar and n are as defined previously can be prepared by any of the methods described below:

**Route 1:**

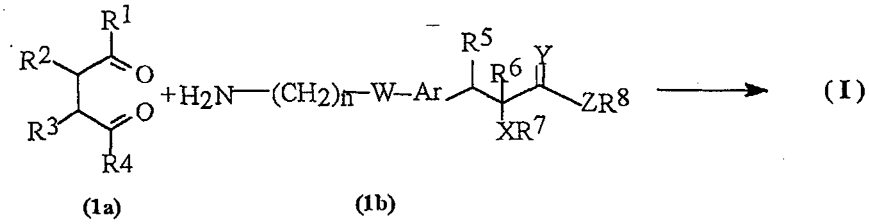

The reaction of a compound of general formula (1a), wherein all symbols are as defined earlier with a compound of formula (1b) which may be chiral or racemic, wherein all symbols are as defined earlier to yield a compound of general formula (I) wherein all symbols are as defined earlier using Paal-Knorr cyclization (Paal C. Ber., 1885, 18, 367; Knorr, L., Ber., 1885, 18, 299). The reaction may be carried out neat or in the presence of a solvent or a mixture thereof such as tetrahydrofuran, hexane, toluene, ethanol, heptane, petroleum ether, xylene, benzene, ethyl acetate, tert-butyl acetate, 1,2-dichloroethane, *iso*-propanol, dioxane, cyclohexane and the like. The reaction temperature may range from 0 °C to the reflux temperature of the solvent(s) used. The water produced may be removed by using a Dean Stark water separator or by water scavengers such as molecular sieves. The reaction may be carried out in the presence of an inert atmosphere such as $N_2$, He or Ar. The reaction may be carried out in the presence of an acid, such as acetic acid, propanoic acid, butyric acid, isobutyric acid, pivalic acid, *p*-toluenesulfonic acid, camphorsulfonic acid, benzenesulfonic acid, trifluoroacetic acid, chloroacetic acid, chloropropanoic acid, phenylacetic acid, phenylpropanoic acid, malonic acid, succinic acid, benzoic acid, halogenated benzoic acid. toluic acid. Mineral acids such as HCl or HBr may also be used. The reaction time may range from 5 minutes to 72 hours, preferably from 1 to 48 hours.

**Route 2 :**

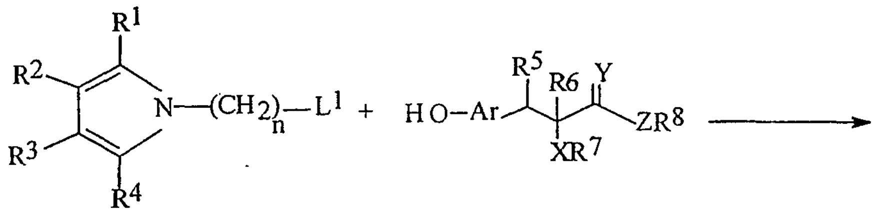
(I)

**(1c)** **(1d)**

The reaction of compound of formula (1c), wherein all the symbols are as defined earlier and $L^1$ represents a leaving group such as halogen atom, *p*-toluenesulfonate, methanesulfonate, trifluoromethanesulfonate with a compound of formula (1d) which may be chiral or racemic, wherein all the symbols are as defined earlier to produce a compound of general formula (I), wherein all the symbols are as defined earlier, may be carried out in the presence of solvents such as acetone, THF, DMSO, dioxane, DMF, DME or a mixture thereof. Bases such as alkali metal carbonates such as $K_2CO_3$, $Na_2CO_3$, alkali metal hydrides such as NaH, KH may be used. The reaction may be carried out at a temperature in the range 0 °C to 150 °C and the reaction time may range from 1 to 48 hours.

**Route 3 :**

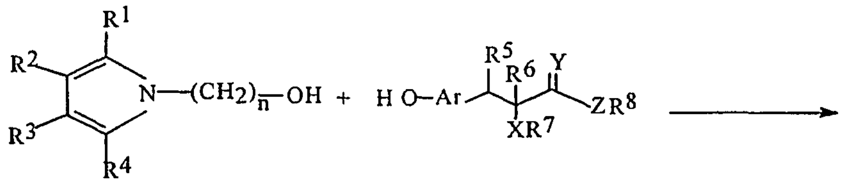
(I)

**(1e)** **(1d)**

The reaction of compound of general formula (1e) where all symbols are as defined earlier with a compound of general formula (1d) which may be chiral or racemic, defined earlier may be carried out using coupling agents such as DCC, EDC, triaryl phosphine/dialkyl azadicarboxylate such as $PPh_3$/DEAD or $PPh_3$/DIAD. Inert atmosphere may be maintained using $N_2$, Ar or He. Solvents such as THF, Dioxane, DME, toluene, $CH_2Cl_2$, $CHCl_3$ $CCl_4$, acetonitrile may be used. Compounds such as DMAP, HOBT may be used in the range of 0.05 to 2 equivalents. The reaction temperature in the range of 0 °C to reflux temperature of the solvent may be used, preferably, 20 °C to 80 °C. The duration of the reaction may range from 0.5 to 24 h, preferably 0.5 to 12 hours.

**Route 4:**

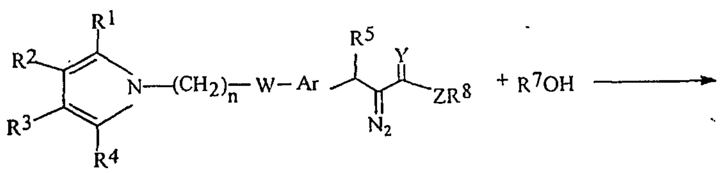
(I)

(1f) (1g)

The reaction of a compound of formula (1f) where all symbols are as defined earlier with an alcohol of formula (1g) where $R^7$ is as defined earlier except H, to produce a compound of formula (I) where all symbols are as defined earlier and X represents O atom, may be carried out in the presence of rhodium salts such as rhodium (II) acetate. Solvents such as benzene, toluene, ether, THF, dioxane may be used. $R^7OH$ may also be used as solvent to enhance the rate of the reaction. Inert atmosphere may be maintained using $N_2$, Ar or He. The reaction time may range from 0.25 to 48 hours, preferably 0.25 h to 8 h.

**Route 5:**

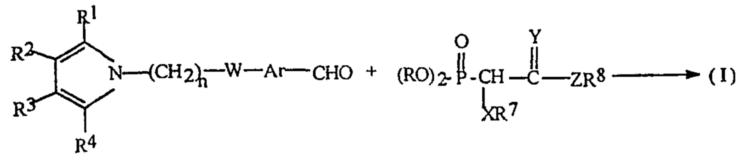

(1h) (1i)

The reaction of a compound of general formula (1h) wherein all the symbols are as defined earlier, with a compound of formula (1i), where all the symbols are as defined earlier and R represents ($C_1$-$C_8$) alkyl to afford a compound of formula (I) where $R^5$ and $R^6$ represent a bond and all other symbols are as defined earlier, may be carried out under Wittig Homer reaction conditions in the presence of a base such as alkali metal hydrides, like NaH or KI, alkali metal alkoxides such as NaOMe, NaOEt, $K^+$t-$BuO^-$ or mixture thereof, organolithiums like $CH_3Li$, BuLi, *sec*-BuLi, LDA. Aprotic solvents such as THF, dioxane, DMF, DMSO, DME or mixture thereof may be employed. HMPA favours the progression of the reaction but not essential. The reaction may be carried out at a temperature ranging from -80 °C to 50 °C, preferably, from 0 °C to 30 °C. The reaction proceeds more effectively under anhydrous conditions.

The compound of formula (I) where $R^5$ and $R^6$ represent a bond may be reduced to a compound of general formula (I) where $R^5$ and $R^6$ each represent hydrogen atom by reacting with hydrogen gas in the presence of a catalyst such as 5-10 % Pd/C, Rh/C, Pt/C Raney Ni or 5-100 % w/w of the above mixture thereof catalyst may be employed. The pressure of hydrogen gas may be one atmosphere to 80 psi. Suitable solvents are alcohols such as ethanol, methanol, ethyl acetate, acetic acid. Metal-solvent such as magnesium in alcohol or sodium amalgam in alcohol may also be used.

According to a feature of the present invention, there is provided an intermediate of formula (1h) wherein $R^1$, $R^2$, $R^3$, $R^4$, W, n and Ar are as defined earlier.

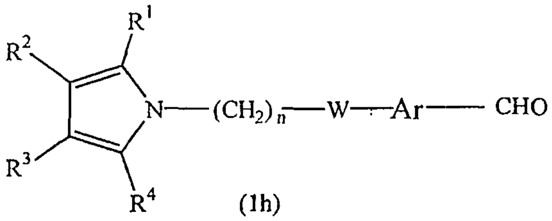

(1h)

According to another feature of the present invention, there is provided a process for the preparation of novel intermediate of the general formula (1h) as defined earlier which comprises reacting a compound of general formula (1c),

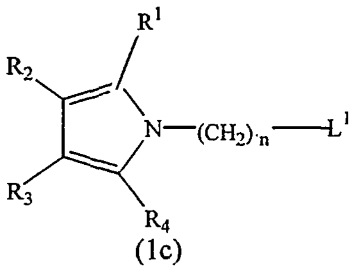

(1c)

HO— Ar—CHO

(1j)

wherein, $R^1$ - $R^4$, n are as defined earlier and $L^1$ is a halogen atom such as chlorine, bromine or iodine or a leaving group such as methanesulfonate, trifluoromethanesulfonate, p-toluenesulfonate with the compound of the formula (1j), where Ar is as defined earlier.

The reaction of the compound of formula (1c) with the compound of formula (1j) to produce a compound of formula (1h) may be carried out in the presence of solvents such as THF, DMF, DMSO, DME. Mixture of solvents may be used. The inert atmosphere may be maintained by using inert gases such as $N_2$, Ar or He. The reaction may be effected in the presence of a base such as $K_2CO_3$, $Na_2CO_3$, NaH or mixtures thereof. The reaction temperature may range from 20 °C to 150 °C, preferably at a temperature in the range from 30 °C to 100 °C. the duration of reaction of

the reaction may range from 1 to 24 hours, preferably from 2 to 6 hours.

Alternatively, the novel intermediate of the general formula (1h), can also be prepared by the reaction of compound of general formula (1e),

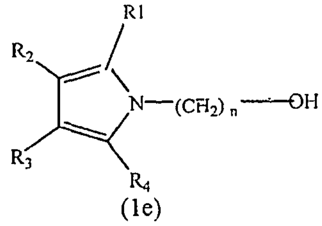

(1e)

$L^2$—Ar—CHO

(1k)

wherein $R^1$ - $R^4$, n are as defined earlier and with a compound of the formula (1k), where Ar is as defined earlier and $L^2$ is a halogen atom such as fluorine, chlorine, bromine or iodine. The reaction of the compound of formula (1e) with the compound of formula (1k) to produce a compound of formula (1h) may be carried out in the presence of solvents such as THF, DMF, DMSO, DME. Mixture of solvents may be used. The inert atmosphere may be maintained by using inert gases such as $N_2$, Ar or He. The reaction may be effected in the presence of a base such as $K_2CO_3$, $Na_2CO_3$, NaH or mixtures thereof. The reaction temperature may range from 20 °C to 150 °C, preferably at a temperature in the range from 30 °C to 100 °C. The duration of reaction of the reaction may range from 1 to 24 hours, preferably from 2 to 6 hours.

The novel intermediate of the formula (1h) defined above can also be obtained by the reaction of a compound (1e) as defined earlier with the compound of formula (1j) as defined earlier.

The reaction of compound of general formula (1e) with the compound of formula (1j) may be carried out using suitable coupling agents such as dicyclohexyl urea, triarylphosphine/dialkylazadicarboxylate such as $PPh_3$ /DEAD. The reaction may be carried out in the presence of solvents such as THF, DME, $CH_2Cl_2$, $CHCl_3$, toluene, acetonitrile, carbontetrachloride. The inert atmosphere may be maintained by using inert gases such as $N_2$, Ar or He. The reaction may be effected in the presence of DMAP, HOBT and they must be used in the range of 0.05 to 2 equivalents, preferably 0.25 to 1 equivalents. The reaction temperature may range from 0 °C to 100 °C, preferably at a temperature in the range from 20 °C to 80 °C. the duration of reaction of the reaction may range from 0.5 to 24 hours, preferably from 6 to 12 hours.

In another embodiment of this invention, there is provided a process for the preparation of a compound of the general formulae (1c) and (1e), which comprises reacting the compound of general formula (1a)

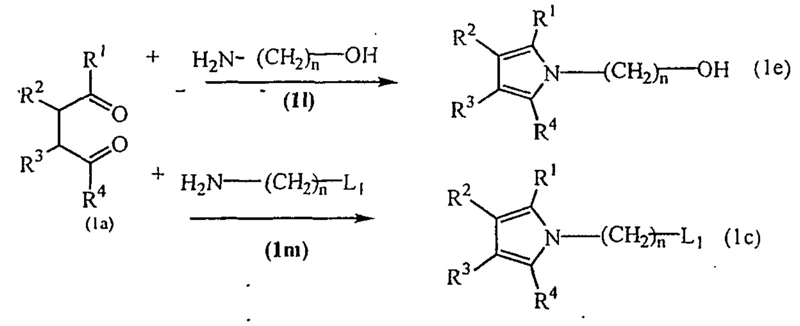
(1a) (1l) (1e) (1m) (1c)

wherein $R^1$ - $R^4$ are as defined earlier, with either substituted aminoalcohol (11), where n is as defined earlier or with substituted amine (1m), where n and $L^1$ as defined earlier, to yield the intermediate of the general formula (1c) or (1e). The reaction of compound of general formula (1a) with the compound of general formula either (11) or (1m) may be carried out neat or in presence of solvents or a mixture thereof such as tetrahydrofuran, hexane, toluene, ethanol, heptane, petroleum ether, xylene, benzene, ethyl acetate, tert-butyl acetate, 1,2-dichloroethane, iso-propanol, dioxane, cyclohexane. The reaction temperature may range from 0 °C to the reflux temperature of the solvent(s) used. The water produced may be removed by using a Dean Stark water separator or by water scavengers such as molecular sieves. The reaction may be carried out in the presence of an inert atmosphere such as $N_2$, He or Ar. The reaction may be carried out in the presence of an acid, such as acetic acid, propanoic acid, butyric acid, isobutyric acid, pivalic acid, p-toluenesulfonic acid, camphorsulfonic acid, benzenesulfonic acid, trifluoroacetic acid, chloroacetic acid, chloropropanoic acid, phenylacetic acid, phenylpropanoic acid, malonic acid, succinic acid, benzoic acid, halogenated benzoic acid, toluic acid.

The compounds of the present invention contain one or more chiral centers and therefore they also exist as stereoisomers. The stereoisomers of the compounds of the present invention may be prepared by one or more ways

presented below:

i. One or more of the reagents may be used in their single isomeric form. For example, compound (1b) or (1d) may be pure stereoisomers.

ii. Optically pure catalysts or chiral ligands along with metal catalysts may be employed in the reduction process. The metal catalyst may be Rhodium, Ruthenium, Indium and the like. The chiral ligands may preferably be chiral phosphines. (Principles of Asymmetric synthesis J E Baldwin Ed. Tetrahedron series, Volume 14, Page no. 311-316)

iii. Mixture of stereoisomers may be resolved by conventional methods such as microbial resolution, resolving the diastereomeric salts formed with chiral acids or chiral bases. Chiral acids may be tartaric acid, mandelic acid, lactic acid, camphorsulfonic acid, amino acids. Chiral bases may be cinchona alkaloids, brucine or a basic amino acid such as lysine, arginine.

iv. Resolution of the mixture of stereoisomers may also be effected by chemical methods by derivation of the compound with a chiral compound such as chiral amines, chiral acids, chiral amino alcohols, amino acids into a 1: 1 mixture of diastereomers and the diastereomers may be separated by conventional methods of fractional crystallization, chromatography and the like foilowed by cleaving the derivative (Jaques et al. "Enantiomers, Racemates and Resolution", Wiley Interscience, 1981).

The pharmaceutically acceptable salts forming a part of this invention may be prepared by treating the compound of formula (I) with 1-6 equivalents of a base such as sodium hydride, sodium methoxide, sodium ethoxide, sodium hydroxide, potassium tert-butoxide, calcium hydroxide, calcium acetate, calcium chloride, magnesium hydroxide, magnesium chloride. Solvents such as water, acetone, ether, THF, methanol, ethanol, t-butanol, dioxane, isopropanol, isopropyl ether or mixtures thereof may be used. Organic bases such as lysine, arginine, methyl benzylamine, ethanolamine, diethanolamine, tromethamine, choline, guanidine and their derivatives may be used. Acid addition salts, wherever applicable may be prepared by treatment with acids such as tartaric acid, mandelic acid, fumaric acid, maleic acid, lactic acid, salicylic acid, citric acid, ascorbic acid, benzene sulfonic acid, p-toluene sulfonic acid, hydroxynaphthoic acid, methane sulfonic acid, malic acetic acid, benzoic acid, succinic acid, palmitic acid, hydrochloric acid, hydrobromic acid, sulfuric acid, nitric acid in solvents such as water, alcohols, ethers, ethyl acetate, dioxane, DMF or a lower alkyl ketone such as acetone, or mixtures thereof.

Different polymorphs may by prepared by crystallization of compound of formula (I) under different conditions such as different solvents or solvent mixtures in varying proportions for recrystallization, various ways of crystallization such as slow cooling, fast cooling or a very fast cooling or a gradual cooling during crystallization. Different polymorphs may also be obtained by heating the compound, melting the compound and solidification by gradual or fast cooling, heating or melting under vacuum or under inert atmosphere, and cooling under either vacuum or inert atmosphere. The various polymorphs may be identified by differential scanning calorimeter, powder X-ray diffraction, IR spectroscopy or solid probe NMR spectroscopy.

Another aspect of the present invention comprises a pharmaceutical composition, containing at least one of the compounds of the general formula (I), their derivatives, their analogs, their tautomeric forms, their stereoisomers, their polymorphs, their pharmaceutically acceptable salts, their pharmaceutically acceptable solvates thereof as an active ingredient, together with pharmaceutically employed carriers diluents.

Pharmaceutical compositions containing a compound of the present invention may be prepared by conventional techniques, e.g. as described in Remington: the Science and Practice of Pharmacy, 19th Ed., 1995. The compositions may be in the conventional forms, such as capsules, tablets, powders, solutions, suspensions, syrups, aerosols or topical applications. They may contain suitable solid or liquid carriers or in suitable sterile media to form injectable solutions or suspensions. The compositions may contain 0.5 to 20 %, preferably 0.5 to 10 % by weight of the active compound, the remaining being pharmaceutically acceptable carriers, excipients, diluents, solvents and the like.

Typical compositions containing a compound of formula (I) or a pharmaceutically acceptable acid addition salt thereof, associated with a pharmaceutically acceptable excipients which may be a carrier or a diluent or be diluted by a carrier, or enclosed within a carrier which can be in the form of a capsule, sachet, paper or other container. When the carrier serves as a diluent, it may be solid, semi-solid, or liquid material, which acts as a vehicle, excipients or medium for the active compound. The active compound can be absorbed on a granular solid container for example in a sachet. Some of suitable carriers are water, salt solutions, alcohols, polyethylene glycols, polyhydroxyethoxylated castor oil, peanut oil, olive oil, gelatin, lactose, terra alba, sucrose, cyclodextrin, amylose, magnesium sterate, talc, gelatin, agar, pectin, acacia, stearic acid or lower alkyl ethers of cellulose, silicic acid, fatty acids, fatty acid amines, fatty acids monoglycerides and diglycerides, pentaerythritol fatty acids esters, polyoxyethylene, hydroxymethylcellulose and polyvinylpyrrolidone. Similarly, the carrier or diluent may include any sustained release material known in the art, such as glyceryl

monostearate or glyceryl distearate, alone or mixed with a wax. The formulations may also include wetting agents, emulsifying and suspending agents, preserving agents, sweetening agents or flavoring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, buffers and/or coloring substances, which do not deleteriously react with the active compounds.

The route of administration may be any route, which effectively transports the active drug to the appropriate or desired site of action effectively, such as oral, nasal, transdermal, pulmonary or parental e.g. rectal, depot, subcutaneous, intravenous, intraurethral, intramuscular, intranasal, ophthalmic solution or an ointment, preferably through oral route.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of formula (I) dissolved or suspended in a liquid carrier, in particular an aqueous carrier, for aerosol application. The carrier may contain additives such as solubilizing agent, e.g. propylene glycol, surfactants, absorption enhancers such as lecithin (phosphatidylcholine) or cyclodextrin, or preservatives such as parabens.

For parental application, particularly suitable are injectable solutions or suspensions, preferably aqueous solutions with the active compound dissolved in polyhydroxylated castor oil.

Tablet, dragees or capsules having talc and/or a carbohydrate carrier or binder or the like are particularly suitable for oral application. Preferably, carriers for tablets, dragees or capsules include lactose, corn starch and/or potato starch. A syrup or elixir can be used in cases where a sweetened vehicle can be employed.

A typical tablet which may be prepared by conventional tabletting techniques may contain:

| | | |
|---|---|---|
| Core: | | |
| | Active compound (as free compound or salt thereof) | 5.0 mg |
| | Colloidal silicon dioxide (Aerosil) | 1.5 mg |
| | Cellulose, misrocrytalline (Avicel) | 70.0 mg |
| | Modified cellulose gum (Ac-Di-Sol) | 7.5 mg |
| | Magnesium sterate | ad. |
| Coating: | | |
| | HPMC approx. | 9.0 mg |
| | *Mywacett 9-40 T approx | 0.9 mg |
| | *Acylated monoglyceride used as plasticizer for film coating. | |

The compounds of general formula (I) or the compositions thereof are useful for the treatment and/or prophylaxis of disease caused by metabolic disorders such as hyperlipidemia, insulin resistance, Leptin resistance, hyperglycemia, obesity, or inflammation.

These compounds are useful for the treatment of hypercholesteremia, familial hypercholesteremia, hypertriglyceridemia, type 2 diabetes, dyslipidemia, disorders related to syndrome X such as hypertension, obesity, insulin resistance, coronary heart disease, atherosclerosis, xanthoma, stroke, peripheral vascular diseases and related disorders, diabetic complications, certain renal diseases such as glomerulonephritis, glomerulosclerosis, nephrotic syndrome, hypertensive nephrosclerosis, retinopathy, nephropathy, psoriasis, polycystic ovarian syndrome, osteoporosis, inflammatory bowel diseases, myotonic dystrophy, arteriosclerosis, Xanthoma, pancreatitis and for the treatment of cancer.

The compounds of the invention may be administered to a mammal, especially, a human in need of such treatment, prevention, elimination, alleviation or amelioration of diseases mentioned above.

The compounds of the present invention are effective over a wide dosage range, however, the exact dosage, mode of administration and form of composition depends upon the subject to be treated and is determined by the physician or veterinarian responsible for treating the subject. Generally, dosages from about 0.025 to about 200 mg preferably from about 0.1 to about 100 mg, per day may be used. Generally, the unit dosage form comprises about 0.01 to 100 mg of the compound of formula (I), as an active ingredient together with a pharmaceutically acceptable carrier. Usually suitable dosage forms for nasal, oral, transdermal or pulmonary administration comprises from about 0.001 mg to about 100 mg, preferably from 0.01 mg to about 50 mg of the active ingredient mixed with a pharmaceutically acceptable carrier or diluent.

In another aspect of the present disclosure, method of treatment and/or prevention of the diseases mentioned above are provided.

In a further aspect of the present invention, use of one or more compounds of the general formula (I) or pharmaceutically acceptable salts, for the preparation of a medicament thereof for the treatment and/or prevention of diseases mentioned in this document is provided.

In still further aspect of the present invention use of the compounds of the present invention together with statins, glitazones, sulfonylureas, fibric acid derivatives, α-glycosidase inhibitors or antioxidants is provided.

The invention is explained in detail in the examples given below which are provided by the way of illustration.

**PREPARATION 1:**

**Preparation of 1-(2-hydroxyethyl)-2,5-dimethyl-1*H*-pyrrole (Compound No. 2):**

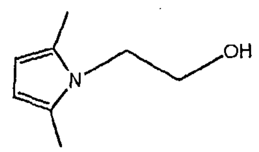

(Compound No. 2)

A mixture of hexan-2,5-dione (5 g), ethanolamine (26.7 g), pivalic acid (23.26 g) and the solvent mixture containing n-heptane: tetrahydrofuran: toluene (4:1:1, 5 mL) was refluxed with stirring at 110 - 120 °C. Water formed during the reaction was removed azeotropically during 3 - 4 h. The mixture was cooled and the solvent was removed. The residue obtained was dissolved in dichloromethane (30 mL), washed with saturated sodium bicarbonate solution (30 mL), water (30 mL), and then with brine (30 mL), dried ($Na_2SO_4$) and the solvent was evaporated. The crude compound obtained as an oily mass, was purified by column chromatography (silica gel 100-200), using ethyl acetate: hexane (2: 8) as an eluent to obtain the title compound.

In like manner to that described in Preparation 1, following compounds of the formula (1e) (Given in Table 1) were prepared from the appropriately substituted diketones. The latter can be synthesized by using various routes found in literature.

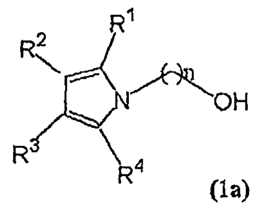

(1a)

**Table 1:**

| Comp. No | Substituents on the pyrrole ring in (1e) | | | | n = | Mol. Wt. (mp°C) | Yield (%w/w) | $^1$H NMR (300MHz, δ, $CDCl_3$) |
|---|---|---|---|---|---|---|---|---|
| | $R^1$ | $R^2$ | $R^3$ | $R^4$ | | | | |
| 1. | H | H | H | H | 2 | 111 | 98 | - |
| 2. | $CH_3$ | H | H | $CH_3$ | 2 | 139 | 65 | 2.21 (6H, s); 3.70-3.72 (2H, m); 3.89(2H, t, J = 5.8 Hz); 5.76 (2H, s). |

(continued)

| Comp. No | Substituents on the pyrrole ring in (1e) | | | | n = | Mol. Wt. (mp°C) | Yield (%w/w) | $^1$H NMR (300 MHz, δ, $CDCl_3$) |
|---|---|---|---|---|---|---|---|---|
| | $R^1$ | $R^2$ | $R^3$ | $R^4$ | | | | |
| 3. | i-Pr | 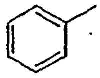 | H | i-Pr | 2 | 271 | 42 | 1.25(12H, 4d, J=6.5 Hz); 2.97 (1H, sept, J = 6.7 Hz); 3.24 (1H, sep, J = 6.7 Hz); 3.85 (2H, m); 4.1(2H, t, J = 7 Hz); 5.87(1H, s); 7.19-7.32 (5H, m) |
| 4. | i-Pr | H | H | 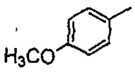 | 2 | 259 | 84 | 1.27 (6H, d, J = 6.5 Hz); 2.99-3.04 (1H, m); 3.53 (2H, t, J = 6.15 Hz); 3.82 (3H, s); 4.09 (2H, t, J = 6.2 Hz); 5.96 (1H, d, J = 3.5 Hz); 6.67 (1H, d, J = 3.48 Hz); 6.91 (2H, d, J = 8.9 Hz); 7.29 (2H, d, J = 8.6 Hz) |

(continued)

| Comp. No | Substituents on the pyrrole ring in (1e) | | | | n = | Mol. Wt. (mp°C) | Yield (%w/w) | $^1$H NMR (300 MHz, δ, $CDCl_3$) |
|---|---|---|---|---|---|---|---|---|
| | R1 | R2 | R3 | R4 | | | | |
| 5. | i-Pr | H | H | 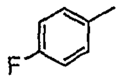 | 2 | 247 | - | 1.27 (6H, d, J = 6.0 Hz); 2.97-3.06 (1H, m); 3.53 (2H, t, J = 6.0 Hz); 4.08 (2H, t, J = 6.0 Hz); 5.99 (1H, d, J = 3.60 Hz); 6.10 (1H, d, J = 3.3 Hz); 7.05-7.1 (2H, t, J = 8.8 Hz); 7.34-7.37 (2H, m) |
| 6. | i-Pr | H | 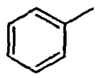 | 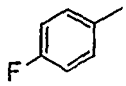 | 2 | 323.2 (109°C) | 55 | 1.34 (6H, d, J = 7 Hz); 3.09 (1H, sep, J = 7 Hz); 3.57 (2H, t, J = 4.5 Hz); 4.02 (2H, t, J = 4.5 Hz); 7.03-7.30 (9H, m) |
| 7. | i-Pr | 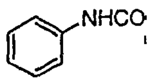 | 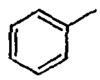 | 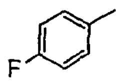 2 | 2 | 442 (175-178°C) | 52 | 1.47 (6H, d, J = 7.2 Hz); 3.5-3.6 (1H, m); 3.59 (2H, t, J = 6.2 Hz); 3.99 (2H, t, J = 6.6 Hz); 6.79 (1H, s); 6.91-7.0 (3H, m) 7.08-7.19 (10H, m) |
| 8. | i-Pr | 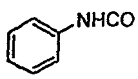 | 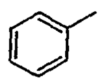 | 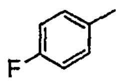 | 3 | 456 (58-62°C) | 50 | --- |

(continued)

| Comp. No | Substituents on the pyrrole ring in (1e) | | | | n = | Mol. Wt. (mp°C) | Yield (%w/w) | $^1H$ NMR (300 MHz, δ, $CDCl_3$) |
|---|---|---|---|---|---|---|---|---|
| | $R^1$ | $R^2$ | $R^3$ | $R^4$ | | | | |
| 9. | 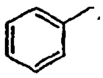 | -H | H | 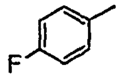 | 2 | 281 | 79 | 1.55 (1H, s); 3.3 (2H, dd, J =6.0 Hz); 4.2 (2H, t, J = 6.0 Hz); 6.25 (2H, dd, J =3.6 Hz); 7.1(2H, t, J =7.0 Hz); 7.4 (1H, m, J = 9.0 Hz); 7.42-7.47 (6H, m) |
| 10. | 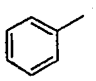 | -COOEt | H | 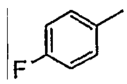 | 2 | 353 | 55 | 1.10 (3H, t, J = 7.0 Hz); 1.60 (1H, s, OH); 3.35 (2H, t, J = 6.0 Hz); 4.00 (2H, t, J = 6.0 Hz); 4.10 (2H, t, J = Hz); 6.69 (1H, s); 7.10 (2H, t, J = 9.9 Hz); 7.39-7.46 (7H, m) |
| 11. | i-Pr | H | H | $CH_3$ | 2 | 167 | 68 | 1.2 (6H, d, J = 8 Hz); 2.2 (3H, s); 2.94 (1H, septet); 3.77 (2H, t, J = 6.9 Hz); 3.97 (2H, t, J = 6.9 Hz) |

**PREPARATION 2 :**

Compound 8 described in the table 1, can also be prepared by alternative route using the corresponding aldehyde in better yields. The process is given below:

A mixture containing corresponding aldehyde (2g) and sodium borohydride (0.167 g) was dissolved in absolute alcohol (20 mL). It was stirred at 0-5 °C for about 2 h. The solid product obtained, was diluted with ice-cold water (40 mL), and stirred for 15 min, filtered and washed with water (2 x 10 mL), dried in vacuum desiccator over phosphorous pentoxide (2 g, 100 %).

**PREPARATION 3:**

**Preparation of Methyl 2-(2,5-dimethyl-1*H*-pyrrol-1-yl)ethyl sulfonate (Compound No. 13):**

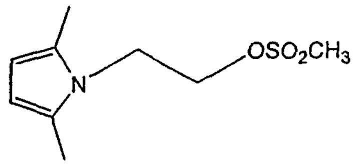

To a solution of compound 2 obtained in preparation 1 (3.0 g), triethylamine (11mL) was added methane sulfonyl chloride (5 g) at 0 °C and was stirred at 0 °C for 1h under nitrogen atmosphere. The mixture was warmed to temperature of about 20 to 25 °C and was stirred at that temperature for about 2 h (TLC). After the completion of reaction, water (30 mL) was added and the organic layer was separated. The mixture was washed with saturated sodium bicarbonate solution (20 mL), water (20 mL), and then with brine (20 mL), and dried over $Na_2SO_4$. The organic layer was evaporated under reduced pressure. The crude substance was used in the next step without purification. In like manner to that described in Preparation 3 following compounds of the formula (1c) (given in Table 2) were prepared from the appropriately substituted pyrrole derivatives (1a) described in Table 1:

Table 2 :

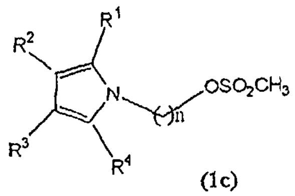

(1c)

| Comp . No. | Substituents on the pyrrole ring in (1c) | | | | n = | Mol. Wt. (mp°C) | Yield (%w/w) | $^1H$ NMR (300 MHz, δ, $CDCl_3$) |
|---|---|---|---|---|---|---|---|---|
| | $R^1$ | $R^2$ | $R^3$ | $R^4$ | | | | |
| 12. | H | H | H | H | 2 | 189 | 26 | 2.7 (3H, s); 4.43 (2H, t, J = 5.2 Hz); 6.17 (2H, t, J = 2.1Hz); 6.7 (2H, t, J = 2.1 Hz); |

(continued)

| Comp . No. | Substituents on the pyrrole ring in (1c) | | | | n = | Mol. Wt. (mp°C) | Yield (%w/w) | $^1$H NMR (300 MHz, $\delta$, $CDCl_3$) |
|---|---|---|---|---|---|---|---|---|
| | $R^1$ | $R^2$ | $R^3$ | $R^4$ | | | | |
| 13. | $CH_3$ | H | H | $CH_3$ | 2 | 217 | 64 | 2.23 (6H, s); 2.68 (3H, s); 4,08 (2H, t, J = 5.8 Hz); 4.34 (2H, t, J = 5.8 Hz); 5.78 (2H, s) |
| 14. | i-Pr | 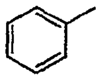 | H | i-Pr | 2 | 349 | 97 | --- |
| 15. | i-Pr | H | H | 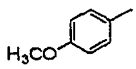 | 2 | 337 | 99 | --- |
| 16. | i-Pr | H | H | 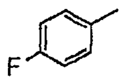 | 2 | 325 | 72 | 1.29 (6H, d, J = 6.0 Hz); 2.69 (3H, s); 2.92 - 2.99 (1H, m); 4.05 (2H, t, J = 6.0 Hz) 4,27 (2H, t, J = 6.0 Hz); 6.00 (1H, d, J = 3.4 Hz); 6.1 (1H, d, J = 3.4 Hz); 7.07 - 7.1 (2H, t, J = 6.0 Hz); 7.30 - 7.35 (2H, m) |

(continued)

| Comp . No. | Substituents on the pyrrole ring in (1c) | | | | n = | Mol. Wt. (mp°C) | Yield (%w/w) | $^1$H NMR (300 MHz, δ, $CDCl_3$) |
|---|---|---|---|---|---|---|---|---|
| | R1 | R2 | R3 | R4 | | | | |
| 17. | i-Pr | H | 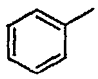 | 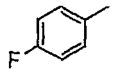 | 2 | 369 | 61 | 1.35 (6H, d, J = 7 Hz); 2.76 (3H, s); 3.0 - 3.05 (1H, m); 4.05 (2H, t, J = 6.2 Hz); 4.15 (2H, t, J = 6 Hz); 6.22 (1H, s); 7.07 - 7.30 (9H, m) |
| 18. | i-Pr | 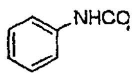 | 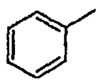 | 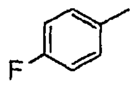 | 2 | 520 (160 - 162 °C) | 85 | --- |
| 19. | i-Pr | 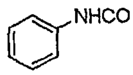 | 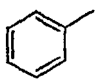 | 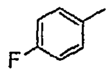 | 3 | 534 | 100 | --- |
| 20. | 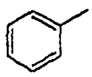 | H | H | 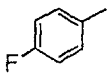 | 2 | 359 | 98 | --- |
| 21. | 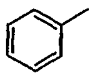 | -COOEt | H | 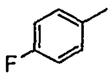 | 2 | 431 | 98.3 | --- |

(continued)

| Comp . No. | Substituents on the pyrrole ring in (1c) | | | | n = | Mol. Wt. (mp°C) | Yield (%w/w) | $^1$H NMR (300 MHz, δ, $CDCl_3$) |
|---|---|---|---|---|---|---|---|---|
| | $R^1$ | $R^2$ | $R^3$ | $R^4$ | | | | |
| 22. | i-Pr | H | H | $CH_3$ | 2 | 245 | 97.1 | 1.28 (6h, d, J = 7.7 Hz); 2.25 (3H, s); 2.83 - 2.92 (1H, m); 4.14 (2H, t, J = 6.9 Hz); 4.34 (2H, t, J = 6.9 Hz); 5.83 (2H, s). |

**EXAMPLE 2 :**

**Preparation of Ethyl 3-{4-[2-(2,5-dimethylpyrrol-1-yl)ethoxy]phenyl}-2-ethoxypropanoate**

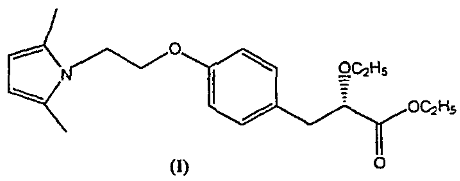

(I)

A mixture ethyl 3-(4-hydroxyphenyl)-2-ethoxypropanoate (1.12 g), and potassium carbonate (2.37 g) in dimethyl formamide (20 mL) was stirred at 70 °C - 80 °C for 10 min, after which mesylate (comp. No.13) (2.3 g) in dimethyl formamide (10 mL) was added. The reaction mixture was stirred at 70 °C to 80 °C for 5 h and allowed to stand overnight at 25 °C - 30 °C (ca.16 h). The reaction mixture was diluted with water (40 mL). The product was extracted with ethyl acetate (2 x 100 mL). The organic layer was washed with saturated sodium bicarbonate solution (65 mL), water (2 x 80 mL), brine (80 mL) and was dried over sodium sulfate. Ethyl acetate was evaporated under reduced pressure to obtain an oily product.

The crude (3 g) product was chromatographed over silica gel using chloroform : ethyl acetate (9 : 1) as an eluent to afford the pure titled compound (2.6 g, 89 %).

In like manner to that described in above example, the following compounds of the formula (I) (given in the Table 3) were prepared from appropriately substituted pyrrole derivatives as described in Table 2:

Table 3 :

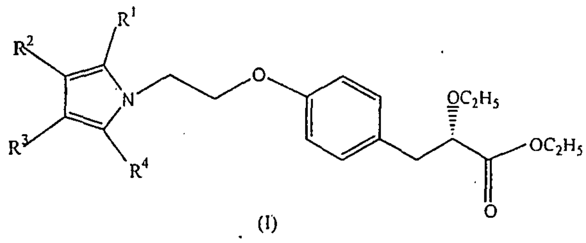

(I)

| Ex. No. | Substituents on the pyrrole ring in (I) | | | | n = | Mol. Wt. (mp°C) | Yield (%w/ w ) | $^1$H NMR (300 MHz, δ, $CDCl_3$) |
|---|---|---|---|---|---|---|---|---|
| | $R^1$ | $R^2$ | $R^3$ | $R^4$ | | | | |
| 1. | H | H | H | H | 2 | 331 | 37 | 1.15 (3H, t, J = 6.9 Hz); 1.22 (3H, t, J = 6.9 Hz); 2.94 (2H, dd); 3.33-3.38 (1H, m); 3.54 - 3.65 (1H, m); 3.95 (1H, dd); 4.12 - 4.26 (6H, m); 6.16 (2H, t, J = 2.1 Hz); 6.7 (2H, t, J = 2.1Hz); 6.8 (2H, d, J = 8.5Hz); 7.15 (2H, d, J = 8.5Hz). |
| 2 | $CH_3$ | H | H | $CH_3$ | 2 | 359 | 57 | 1.15 (3H, t, J = 6.9 Hz); 1.25 (3H, t, J = 6.9 Hz); 2.27 (6H, s); 2.91-2.94 (2H, m); 3.32 - 3.60 (2H, m); 3.97-4.2 (7H, m); 5.78 (2H, s); 6.78 (2H, d, J = 8.5Hz); 7.15 (2H, d, J = 8.5Hz). |

(continued)

| Ex. No. | Substituents on the pyrrole ring in (I) | | | | n = | Mol. Wt. (mp°C) | Yield (%w/ w ) | $^1$H NMR (300 MHz, δ, $CDCl_3$) |
|---|---|---|---|---|---|---|---|---|
| | $R^1$ | $R^2$ | $R^3$ | $R^4$ | | | | |
| 3 | i-Pr | 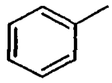 | H | i-Pr | 2 | 491 | 35 | 1.16 (3H, t, J = 6.9 Hz); 1.2-1.3 (15H, m); 2.94 - 2.96 (3H, m); 3.31 - 3.34 (2H, m); 3.96 (2H, t, J = 6.9 Hz); 4.1 - 4.2 (4H, m); 4.3 (2H, t, J = 6.9); 5.89 (1H, s); 6.8 (2H, d, J = 8.5Hz).; 7.15 (2H, d, J = 8.5Hz). 7.2 - 7.33 (5H, m) |

(continued)

| Ex. No. | Substituents on the pyrrole ring in (I) | | | | n = | Mol. Wt. (mp°C) | Yield (%w/ w ) | $^1$H NMR (300 MHz, δ, $CDCl_3$) |
|---|---|---|---|---|---|---|---|---|
| | $R^1$ | $R^2$ | $R^3$ | $R^4$ | | | | |
| 4 | i-Pr | H | H | 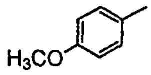 | 2 | 479 | 33 | 1.1 (3H, t, J = 7 Hz); 1.2 (3H, t, J = 7 Hz); 1.31 (6H, d, J= 6 Hz); 3.0 - 3.1 (1H, m); 2.90 (2H, dd); 3.33 (2H, m); 3.8 (3H, s); 3.85 (2H, t,); 3.92 (1H, t); 4.12 - 4.16 (2H, q, J = 7.14 Hz); 4.28 (2H, t, J = 6.8 Hz); 5.98(1H, d, J = 3.4 Hz); 6.07 (1H, d, J = 3.5 Hz); 6.56 (2H, d, J = 8.6 Hz); 6.93 (2H, d, J = 8.7 Hz) 7.32 (2H, d, J = 8.5 Hz); 7.05 (2H, d, J = 8.5 Hz); |

(continued)

| Ex. No. | Substituents on the pyrrole ring in (I) | | | | n = | Mol. Wt. (mp°C) | Yield (%w/ w ) | $^1$H NMR (300 MHz, $\delta$, $CDCl_3$) |
|---|---|---|---|---|---|---|---|---|
| | $R^1$ | $R^2$ | $R^3$ | $R^4$ | | | | |
| 5 | i-Pr | H | H | 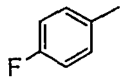 | 2 | 467 | 51 | 1.15 (3H, t, J=6.9Hz); 1.22 (3H, t, J=7.1 Hz); 1.31 (6H, d, J=6 Hz); 2.90 (2H, dd); 3.33-3.35 (1H, m); 3.84 (2H, t, J=6.6 Hz); 3.33 - 3.58 (2H,m); 3.91 - 3.95 (1H, dd); 4.12-4.19 (2H, q, J= 7.0 Hz); 4.29 (2H, t, J=6.6 Hz); 6.55 (2H, d, J=8.6 Hz); 6.10 (1H, d, J=3.5 Hz); 5.98 (1H, d, J=3.4 Hz); 7.0 - 7.1 (4H, m); 7.3 - 7.38 (2H, m) |

(continued)

| Ex. No. | Substituents on the pyrrole ring in (I) | | | | n = | Mol. Wt. (mp°C) | Yield (%w/ w ) | $^1$H NMR (300 MHz, $\delta$, $CDCl_3$) |
|---|---|---|---|---|---|---|---|---|
| | $R^1$ | $R^2$ | $R^3$ | $R^4$ | | | | |
| 6 | i-Pr | H | 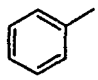 | 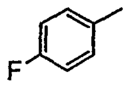 | 2 | 543 | 48 | 1.1 (3H, t, J = 6.99 Hz); 1.2 (3H, t, J = 7.1 Hz); 1.36 (6H, d, J = 7 Hz); 2.9 (2H, d, J= 6.29 Hz); 3.0-3.1 (1H, m); 3.3 - 3.58 (2H, m); 3.8 (2H, t, J = 6.8 Hz); 3.9 (2H, t, J = 7 Hz); 4.1 - 4.2 (4H, m); 6.2 (1H,s); 6.5-7.3 (13H, m). |
| 7 | i-Pr | 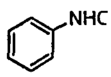 | 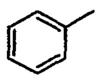 | 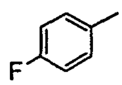 | 2 | 662 | 44 | 1.08 (3H, t, J = 7.0 Hz); 1.16 (3H, t, J = 7.0 Hz); 1.49 (6H d, J = 7 Hz); 2.85 (2H, dd) 3.26 (1H, m); 3.5 (2H, m) , 3.87 (2H, t); 3.9 (1H, t); 4.09 (2H, q); 4.19 (2H, t): 6.53 (2H, d, J = 8.5 Hz) 6.79 (1H, s); 6.90-7.18 (16H, m) |

(continued)

| Ex. No. | Substituents on the pyrrole ring in (I) | | | | n = | Mol. Wt. (mp°C) | Yield (%w/ w ) | $^1H$ NMR (300 MHz, δ, $CDCl_3$) |
|---|---|---|---|---|---|---|---|---|
| | $R^1$ | $R^2$ | $R^3$ | $R^4$ | | | | |
| 8 | i-Pr | 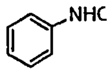 | 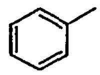 | 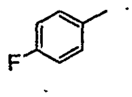 | 3 | 676 | 89 | 1.14(2H, t,, J = 6.9 Hz) 1.22 (3H, t, J = 7 Hz); 1.53 (6H, d, J = 7 Hz); 1.97 (2H, m); 2.91 (2H, dd) 3.32 (1H, m); 3.56 (2H, m); 3.76 (2H, t); 3.93 (1H, t); 4.07 (2H, t); 4.15 (2H, q, J = 7 Hz); 6.62 - 6.65 (2H, d); 6.84 (1H, s); 6.9 - 6.98 (3H, m) ; 7.03 - 7.05 (2H, d); 7.09 - 7.18 (10H, m). |

(continued)

| Ex. No. | Substituents on the pyrrole ring in (I) | | | | n = | Mol. Wt. | Yield | $^1$H NMR |
|---|---|---|---|---|---|---|---|---|
| | $R^1$ | $R^2$ | $R^3$ | $R^4$ | | (mp°C) | (%w/ w ) | (300 MHz, δ, $CDCl_3$) |
| 9 | 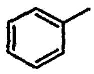 | H | H | 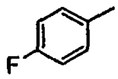 | 2 | 501 | 15 | 1.12(3H, t, J = 7.0 Hz); 1.21 (3H, t, J = 7.0 Hz); 2.88 (2H, d, J = 6.0 Hz); 3.3 (1H, m); 3.6 (1H, m): 3.61 (2H, t); 3.9 (1H, m); 4.1 (2H, t, J = 7.9 Hz); 4.37 (2H, t, J = 6.0 Hz); 6.26 (2H, dd, J =3.3 Hz); 6.9 (2H, d, J = 9.0 Hz): 7.1 (2H, m); 7.41 - 7.49 (9H, m). |
| 10 | 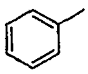 | -COOEt | H | 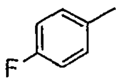 | 2 | 573 | 13.5 | 1.1 - 1.25 (9H, m); 2.8 (2H, d, J = 6.3 Hz); 3.3 (1H, m); 3.6 (1H, m); 3.61 (2H, m); 3.9 (1H, t); 4.1 - 4.21 (6H, m); 6.3 (1H, s); 6.9 (2H, d, J = 9.0 Hz); 7.1 (2H, m); 7.42 - 7.47 (9H, m) |

(continued)

| Ex. No. | Substituents on the pyrrole ring in (I) | | | | n = | Mol. Wt. (mp°C) | Yield (%w/ w ) | $^1$H NMR (300 MHz, δ, $CDCl_3$) |
|---|---|---|---|---|---|---|---|---|
| | $R^1$ | $R^2$ | $R^3$ | $R^4$ | | | | |
| 11 | i-Pr | H | H | $CH_3$ | 2 | 387 | 32.4 | 1.15 (3H, t, J = 6.9 Hz); 1.2 (3H, t, J = 6.9 Hz); 1.25 (6H, d, J = 6.7 Hz); 2.27 (3H,s); 2.9 - 3.0 (3H, m); 3.3 - 3.63 (2H, m); 3.96 (1H, dd, ); 4.06 (2H, t, J = 6.9 Hz); 4.14 - 4.24 (4H, m); 5.83 (2H, s); 6.73 (2H, d, J = 8.5 Hz); 7.15 (2H, d, J = 8.5 Hz). |

**EXAMPLE 13 :**

**Preparation of 3-{4-[2-(2,5-Dimethylpyrrol-1-yl)ethoxy]phenyl}-2-ethoxypropanoic acid**

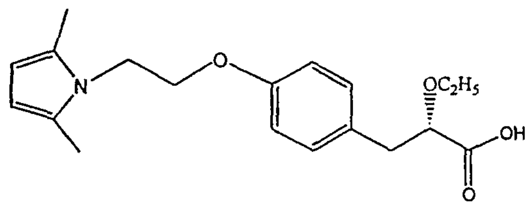

Example no. 13

A mixture of substituted ester (prepared in example 2) (1.38 g), sodium hydroxide (3.0 %, 15 mL) in methanol (20 mL) was stirred at 20 °C to 25 °C for 10 h. Methanol was evaporated under reduced pressure. The residue was diluted with water (20 mL) and it was acidified with dilute hydrochloric acid. The product was extracted with ethyl acetate (3 x 20 mL). The organic layer was washed with saturated sodium bicarbonate solution (65 mL), water (2 x 80 mL), brine (80 mL) and it was dried over sodium sulfate to obtain an oily product (1.2 g, 94 %).The crude product (3 g) was separated by column chromatography using silica gel using hexane : ethyl acetate (9 : 1) as an eluent to afford the pure titled compound (0.75 g, 59 %).

In like manner to that described in Example 13 the following compounds of the formula (I) (given in Table 4) were similarly prepared from the appropriately substituted pyrrole derivatives:

**Table 4 :**

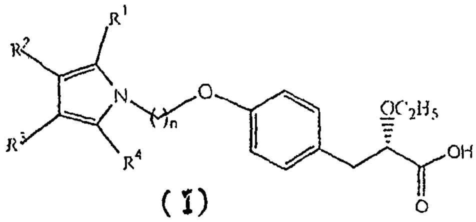

(I)

| Ex. No. | Substituents on the pyrrole ring in (I) | | | | n= | Mol. Wt. (mp°C) | Yield (%w/w) | $^1$H NMR (300 MHz, δ, $CDCl_3$) |
|---|---|---|---|---|---|---|---|---|
| | $R^1$ | $R^2$ | $R^3$ | $R^4$ | | | | |
| 12 | H | H | H | H | 2 | 303 | 79 | 1.16 (3H, t, J 6.9Hz); 2.97(1H, dd); 3.0 (1h, dd); 3.36 - 3.6 (2H, m); 4.01 (1H, dd); 4.17 - 4.28 (4H, m) 6.16 (2H, t, J = 2.1Hz); 6.75 - 6.80 (4H, m); 6.7 (2H, t, J = 2.1Hz); 6.8 (2H, d, J = 8.5Hz); 7.15 (2H, d, J = 8.5Hz). |
| 13 | $CH_3$ | H | H | $CH_3$ | 2 | 331.2 (102) | 59 | 7.18(3H, t, J = 7 Hz); 2.28 (6H, s); 2.93 - 3.08 (2H, m); 3.45 - 3.59 (2H, m); 4.03 - 4.18 (5H, m); 5.79 (1H, s); 6.78(2H, d, J = 8.5Hz); 7.15 (2H, d, J = 8.5Hz). |

(continued)

| Ex. No. | Substituents on the pyrrole ring in (I) | | | | n= | Mol. Wt. (mp°C) | Yield (%w/w) | $^1H$ NMR (300 MHz, $\delta$, $CDCl_3$) |
|---|---|---|---|---|---|---|---|---|
| | $R^1$ | $R^2$ | $R^3$ | $R^4$ | | | | |
| 14 | i-Pr | 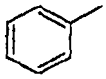 | H | i-Pr | 2 | 463 | 48 | 1.12(3H, t, J = 6.9 Hz); 1.2-1.3 (12H, m); 2.96 - 3.76 (7H, m); 4.03 - 4.05 (2H,m); 4.30 (2H, t, J = 6.9 Hz); 5,89 (1H, s); 6.80 (2H, d, J = 8.5Hz); 7.15 (2H, d, J = 8.5Hz); 7.2 - 7.33 (5H, m). |

(continued)

| Ex. No. | Substituents on the pyrrole ring in (I) | | | | n= | Mol. Wt. (mp°C) | Yield (%w/w) | $^1H$ NMR (300 MHz, δ, $CDCl_3$) |
|---|---|---|---|---|---|---|---|---|
| | $R^1$ | $R^2$ | $R^3$ | $R^4$ | | | | |
| 15 | i-Pr | H | H | 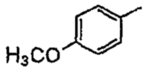 | 2 | 451 | 84 | 1.2(3H, t, J = 7 Hz); 1.29(6H, d, J = 6 Hz); 2.90 (2H, dd); 3.04 - 3.06 (1H, m); 3.33 - 3.59 (2H, m); 3.8 (3H, s); 4.0 (1H, t); 3.84 (2H, t, J = 6 Hz); 4.28 (2H, t, J = 6.7 Hz); 5.98 (1H, d, J = 3.4 Hz); 6.56(2H, d, J = 8.6 Hz); 6.08 (1H, d, J = 3.5 Hz); 6.93 (2H, d, J = 8.7Hz); 7.03 (2H, t, J = 8.5Hz); 7.32 (2H, d, J = 8,5Hz). |

(continued)

| Ex. No. | Substituents on the pyrrole ring in (I) | | | | n= | Mol. Wt. (mp°C) | Yield (%w/w) | $^1$H NMR (300 MHz, δ, $CDCl_3$) |
|---|---|---|---|---|---|---|---|---|
| | $R^1$ | $R^2$ | $R^3$ | $R^4$ | | | | |
| 16 | i-Pr | H | H | 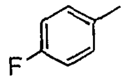 | 2 | 439 | 36 | 1.17 (3H, t, J = 6.9 Hz); 1.3 (6H, d, J = 6.9 Hz); 2.93 (2H, dd); 3.03 - 3.1 (1H, m); 3.33-3.58 (2H, m); 3.84 (2H, t, J = 6.5 Hz); 4.0 (1H,m); 4.29 (2H, t, J = 6.6 Hz); 6.56 (2H, d, J = 8.6 Hz): 6.10 (1H, d, J=3.5 Hz); 6.00 (1H, d, J = 3.5 Hz); 7.0-7.1 (4H, m); 7.3-7.38 (2H, m), |
| 17 | i-Pr | H | 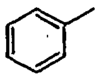 | 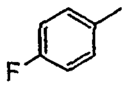 | 2 | 515 (127-128) | 53 | 1.19 (3H, t, J= 6.9 Hz); 1.36 (6H, d, J=7 Hz); 2.95 (2H, dd, J= 7.1 Hz): 3.0-3.1 (1H,m); 3.45 - 3.57 (2H, m); 3.83 (2H, t, J= 6.5 Hz); 4.0-4.04 (1H,m); 4.2 (2H, t, J=6.7 Hz); 6.2 (1H, s); 6.5-7.28 (13H,m). |

(continued)

| Ex. No. | Substituents on the pyrrole ring in (I) | | | | n= | Mol. Wt. (mp°C) | Yield (%w/w) | $^1$H NMR (300 MHz, δ, $CDCl_3$) |
|---|---|---|---|---|---|---|---|---|
| | $R^1$ | $R^2$ | $R^3$ | $R^4$ | | | | |
| 18 | i-Pr | 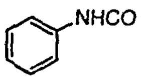 | 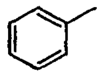 | 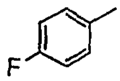 | 2 | 634 (112-114) | 61 | 0.91 (3H, t, J = 6.7 Hz): 1.45 (6H, d, J= 6.8 Hz); 2.91 (2H, dd); 3.13 (1H, m); 3.32 - 3.49 (2H, m); 3.80 (3H, m) 4.15 (2H, t, J= 6.5 Hz); 6.46 (2H, d); 6.78 (1H, 5); 6.86-7.18 (16H, m). |
| 19 | i-Pr | 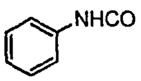 | 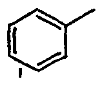 | 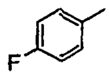 | 3 | 648 (114-116) | 24 | 1.1 (3H, t, J=7Hz); 1.47 (6H, d, J=7Hz); 1.91 (2H, m); 3.01 (2H, dd); 3.41 (1H, m); 3.98 (1H, t); 3.71 (2H. t, J= 6 Hz); 4.02 (2H, t, J= 7.2 Hz): 6.59 (1H. t); 6.78 (1H, s); 6.9 (2H, m); 7.1 (10H. m). |

(continued)

| Ex. No. | Substituents on the pyrrole ring in (I) | | | | n= | Mol. Wt. (mp°C) | Yield (%w/w) | $^1$H NMR (300 MHz, $\delta$, $CDCl_3$) |
|---|---|---|---|---|---|---|---|---|
| | $R^1$ | $R^2$ | $R^3$ | $R^4$ | | | | |
| 20 | 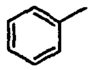 | -H | H | 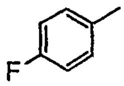 2 | | 473 | 60.3 | 0.9 (3H, t); 2.6 (1H, t); 2.9 (2H, d); 3.2 (1H, m); 3.5 (2H, t); 3.6 (1H, m); 6.21 (2H, dd, J= 3 Hz); 6.9 (2H, d); 7.0 (2H, t, J = 9.0 Hz): 731-7.6 (9H,m). |
| 21 | 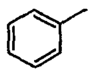 | -COOEt | H | 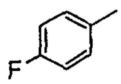 | 2 | 545 | 83 | 0.9 (3H, t); 2.6 (1H, t); 2.9 (2H, d); 3.2(1H, m); 3.5 (2H, t); 3.6 (1H, m); 6.7 (1H, s); 6.9 (2H, d); 7.1 (2H, t); 7.29-7.6 (9H, m). |
| 22 | i-Pr | H | H | $CH_3$ | 2 | 359 | 20 | 1.17(3H, t, J=6.9Hz); 1.26 (6H, d, J = 6.7 Hz); 2.27 (3H,s); 2.9-3.0 (1H, m); 3.07 (1H, dd); 3.42-3.58 (2H, m); 4.02- 4.08 (3H, m); 4.2 (2H, t, J = 6.3 Hz); 5.83 (2H, s); 6.7 (2H, d, J = 8 Hz); 7.15(2H, d,J=8Hz). |

**EXAMPLES 23- 33** are the corresponding sodium salts of the acids in the example 12 - 22 prepared according to the following method.

**EXAMPLE 26 :**

**Preparation of sodium salt of 3-{4-[2-(2,5-Dimethylpyrrol-1-yl)ethoxy]phenyl}-2-ethoxypropanoic acid**

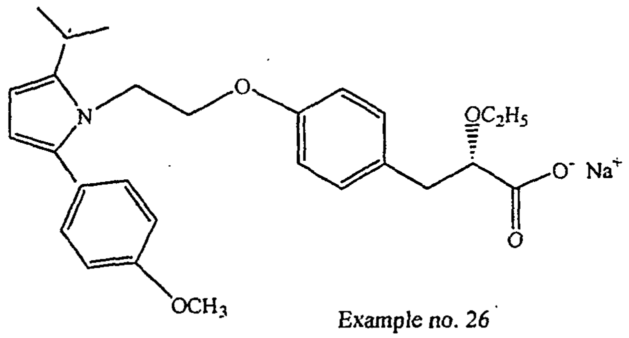

Example no. 26

To acid compound (prepared in example 15 above) (0.64 g) taken in 20 mL methanol, sodium hydroxide (0.056 g) was added and stirred for 3 hour at 20 °C-25 °C. Afterwards, methanol was distilled at reduced pressure, to obtain the titled compound (0.5 g).

**EXAMPLES 34- 44** are the corresponding calcium salts of the acids in the example 12 - 22 prepared according to the following method.

**EXAMPLE 37 :**

**Preparation of calcium salt of 3-{4-[2-(2,5-Dimethylpyrrol-1-yl)ethoxy]phenyl}-2-ethoxypropanoic acid**

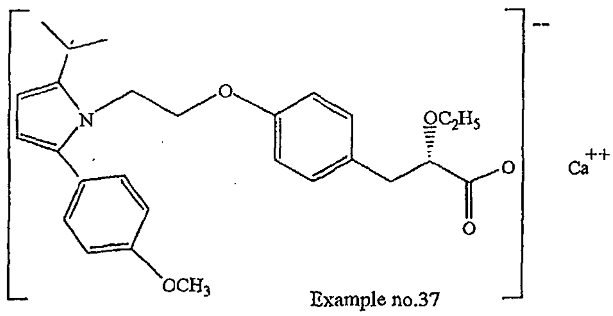

Example no.37

The sodium salt (obtained in example 26)(0.5 g) was dissolved in methanol (20 mL) and treated with calcium acetate (0.195 g) at 20 °C - 25 °C. Further, 50 mL of water was added when the calcium salt of the acid precipitates out. The precipitate was filtered, washed with water and then with di-isopropyl ether (2 x 20 mL) to afford the title compound.

Similarly, other pharmaceutically acceptable salts can be prepared in a similar way as described above using the appropriate acids/bases or according to the methods known in literature. '

The compounds of the present invention lowered random blood sugar level, triglyceride, total cholesterol, LDL, VLDL and increased HDL. This was demonstrated by in vivo animal experiments.

Demonstration of *in vivo* efficacy of compounds:

**1. Plasma triglyceride and total cholesterol lowering activity in Swiss albino mice :**

Male Swiss albino mice (SAM) were obtained from NTN, Hyderabad, India and housed in Zydus animal house. All these animals were maintained under 12 hour light and dark cycle at 25±1 °C. Animals were given standard laboratory

chow (NIN, Hyderabad, India) and water ad libitum. SAM of 20-25 g body weight range were used.

The test compounds were administered orally to Swiss albino mice at 0.3 to 50 mg / kg/ day dose for 6 days. Control mice were treated with vehicle (0.25% of Carboxymethylcellulose; dose 10 ml/kg).

The blood samples were collected in fed state 1hour after drug administration on 0 and 6 day of the treatment. The blood was collected from the retro-orbital sinus through non-heparinised capillary and the serum was analyzed for triglyceride and total cholesterol (Wieland, O. Methods of Enzymatic analysis. Bergermeyer, H., O., Ed., 1963. 211-214; Trinder, P. Ann. Clin. Biochem. 1969. 6: 24-27). Measurement of plasma triglyceride and total cholesterol done using commercial kits (Zydus-Cadila, Pathline, Ahmedabad, India). Formula for calculation
Percentage reduction in triglycerides/total cholesterol were calculated according to the formula :

$$\text{Percentage reduction (\%)} = 1 - \frac{\left[ TT/TO \right]}{TC/OC} \, TT \times 100$$

OC = Zero day control group value OT = Zero day treated group value
TC = Test day control group TT = Test day treated group

**2. Cholesterol lowering activity in hypercholesterolemic rat models**

Male Sprague Dawley rats stock bred in Zydus animal house were maintained under 12 hour light and dark cycle at 25±1 °C. Rats of 180-200 g body weight range were used for the experiment. Animals were made hypercholesterolemic by feeding 2% cholesterol and 1% sodium cholate mixed with standard laboratory chow (NIN, Hyderabad, India) and water ad libitum for 15 days. Throughout the experiment, the animals were maintained on the same diet (Petit, D., Bonnefis, M. T., Rey, C and Infante, R. Effects of ciprofibrate on liver lipids and lipoprotein synthesis in normal and hyperlipidemic rats. Atherosclerosis. 1988. 74: 215-225).

The test compounds were administered orally at a dose 0.1 to 50 mg/ kg/ day for 6 days. Control group was treated with vehicle alone (0.25% of Carboxymethylcellulose; dose 10 ml/kg).

The blood samples were collected in fed state 1hour after drug administration on 0 and 6 day of the treatment. The blood was collected from the retro-orbital sinus through non-heparinised capillary and the serum samples were analyzed for triglyceride and total cholesterol and HDL using commercial kits (Zydus-Cadila, Pathline, Ahmedabad, India). LDL and VLDL cholesterol were calculated from the data obtained for total cholesterol, HDL and triglyceride. The reductions of various parameters examined are calculated according to the formula.

LDL and VLDL cholesterol levels were calculated according to the formula :

$$\text{LDL cholesterol in mg/dl} = \text{Total cholesterol} - \text{HDL cholesterol} - \text{triglyceride}$$

$$\text{VLDL cholesterol in mg/dl} = \text{Total cholesterol} - \text{HDL cholesterol} - \text{LDL cholesterol}$$

## Claims

**1.** A compound represented by the formula (I):

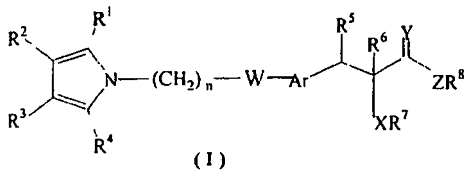

(I)

its tautomeric forms, its stereoisomers, its pharmaceutically acceptable salts and its pharmaceutically acceptable solvates, wherein one or more groups $R^1$, $R^2$ $R^3$, $R^4$ may be same or different and represent hydrogen, halogen, perhaloalkyl, amino, nitro, cyano, formyl, amidino, guanidino, substituted or unsubstituted groups selected from

- ($C_1$-$C_{12}$) alkyl groups;
- linear or branched ($C_2$-$C_{12}$)alkenyl groups;
- ($C_3$-$C_7$)cycloalkyl groups;
- ($C_3$-$C_7$)cycloalkenyl groups;
- bicycloalkyl selected from bicyclooctane, bicyclononane and bicyclodecane; bicycloalkenyl selected from bicyclooctene, bicyclononene and bicyclodecene;
- ($C_1$-$C_{12}$) alkoxy groups;
- cyclo ($C_3$-$C_7$)alkoxy groups;
- aryl groups selected from phenyl, naphthyl, benzo-[1,3]dioxole or biphenyl groups;
- aryloxy groups selected from phenoxy, naphthyloxy, biphenyloxy groups;
- aralkyl groups wherein aryl and alkyl groups are as defined earlier;
- aralkoxy groups wherein aralkyl group as defined earlier;
- heteroaryl groups selected from pyridyl, thienyl, furyl, pyrrolyl, oxazolyl, thiazolyl, imidazolyl, oxadiazolyl, tetrazolyl, benzopyranyl, benzofuranyl groups;
- heteroaralkyl groups selected from furanmethyl, pyridinemethyl, oxazolemethyl, oxazolethyl groups;
- heterocyclyl groups selected from aziridinyl, pyrrolidinyl, piperidinyl.
- heterocyclylalkyl group selected from pyrrolidinealkyl, piperidinealkyl, morpholinealkyl, thiomorpholinealkyl, oxazolinealkyl.
- heterocycloalkoxy groups wherein heterocyclyl and and alkoxy group as defined earlier;
- heteroaryloxy groups wherein the heteroaryl group as defined earlier;
- heteroaralkoxy groups, wherein the heteroaralkyl group as defined earlier;
- acyl groups selected from acetyl, propionyl or benzoyl groups;
- acyloxy groups selected from MeCOO, EtCOO, PhCOO groups;
- acylamino groups selected from $CH_3CONH$, $C_2HsCONH$, $C_3H_7CONH$, $C_6H_5CONH$ groups;
- ($C_1$-$C_6$)monoalkylamino groups;
- ($C_1$-$C_6$) dialkylamino groups;
- arylamino wherein the aryl group is as defined earlier ;
- aralkylamino groups wherein the aryl group is as defined earlier;
- alkoxycarbonyl, wherein the alkoxy group is as defined earlier;
- aryloxycarbonyl, wherein the aryloxy group is as defined earlier
- aralkoxycarbonyl, wherein the aralkoxy group is as defined earlier;
- heterocycloalkoxycarbonyl, wherein the heterocycloalkoxy group is -as defined earlier;
- heteroaryloxycarbonyl, wherein the heteroaryloxy group is as defined earlier;
- heteroaralkoxycarbonyl wherein the heteroaralkoxy group is as defined earlier;
- hydroxyalkyl wherein the alkyl group is as defined earlier;
- aminoalkyl wherein the alkyl group is as defined earlier;
- mono or disubstituted alkylaminoalkyl where alkyl group is as defined earlier and substituted group is selected from alkyl, cycloalkyl, alkoxy, cycloalkox,y, aryl, aralkyl, aralkoxyalkyl, heterocyclyl, heteroaryl, heteroaralkyl, acyl, acyloxy, hydroxyalkyl, amino, acylamino, arylamino, aminoalkyl, aryloxy, aralkoxy, alkylamino, alkoxyalkyl, alkylthio or thioalkyl groups;
- alkoxyalkyl wherein the alkyl group is as defined earlier;
- aryloxyalkyl wherein the alkyl group is as defined earlier;
- aralkoxyalkyl wherein the alkyl group is as defined earlier;
- alkylthio or thioalkyl groups wherein the alkyl group is as defined earlier;
- alkoxycarbonylamino wherein the alkoxycarbonyl group is as defined earlier;
- aryloxycarbonylamino wherein the aryloxycarbonyl group is as defined earlier;
- aralkyloxycarbonylamino wherein the aralkyloxycarbonyl group is -as defined earlier;
- aminocarbonylamino wherein the aminocarbonyl group is as defined earlier;
- hydrazino, hydroxylamino; carboxylic acid and its derivatives selected from ($C_1$-$C_3$)esters, wherein the substituents on $R^1$, $R^2$, $R^3$ and $R^4$ are selected from alkyl, cycloalkyl, alkoxy, cycloalkoxy, aryl, aralkyl, aralkoxyalkyl, heterocyclyl, heteroaryl, heteroaralkyl, acyl, acyloxy, hydroxyalkyl, amino, acylamino, arylamino, aminoalkyl, aryloxy, aralkoxy, alkylamino, alkoxyalkyl,
alkylthio, thioalkyl groups, wherein each of the terms are as defined earlier ;

n is an integer ranging from 1 to 8; W represents O, S or $NR^9$ where $R^9$ represents alkyl or aryl as defined earlier; Ar represents a substituted or unsubstituted divalent single or fused aromatic, heteroaromatic or heterocyclic group, each of these terms are selected from those as defined earlier, the substituents on 'Ar' are selected from linear or branched alkyl, alkoxy, halogen, haloalkyl, haloalkoxy, acyl, amino, acylamino, thio or carboxylic or sulfonic acids; $R^5$ and $R^6$ represent both hydrogen or together represent a bond; $R^5$ and $R^6$ may also represent a hydroxy, alkyl, alkoxy, halogen, acyl, aralkyl groups, each of these terms are selected from those as defined earlier; X represents O or S; $R^7$ represents hydrogen, perfluoroalkyl, or groups selected from alkyl, cycloalkyl, aryl, aralkyl, heteroaryl, heteroaralkyl, alkoxyalkyl, aryloxyalkyl, alkoxycarbonyl, aryloxycarbonyl, cycloalkyloxycarbonyl, alkylaminocarbonyl, arylaminocarbonyl, acyl groups, each of these terms are as defined earlier; Y represents O or S; Z represents oxygen or $NR^{10}$, where $R^{10}$ represents hydrogen or groups selected from alkyl, aryl, aralkyl, hydroxyalkyl, aminoalkyl, heteroaryl, heteroaralkyl groups, each of these terms are selected from those are as defined earlier; $R^8$ represents hydrogen, groups selected from alkyl, aryl, aralkyl, heteroaryl, heteroaralkyl, hydroxyalkyl, alkoxyalkyl, alkylaminoalkyl groups, each of these terms are as defined earlier;
$R^{10}$ and $R^8$ together may form a 5 or 6 membered substituted or unsubstituted cyclic ring structure containing carbon atoms or containing one or more heteroatoms selected from O, N and S, the cyclic structures are those as claimed earlier, wherein on the cyclic structure formed by $R^8$ and $R^{10}$ taken together substituents are selected from halogen, hydroxy, alkyl, oxo, aralkyl.

**2.** A compound according to claims 1, wherein Ar represents substituted or unsubstituted divalent phenylene, naphthylene groups and substituted group are selected from linear or branched alkyl, alkoxy, halogen, haloalkyl, haloalkoxy, acyl, amino, acylamino, thio or carboxylic or sulfonic acids.

**3.** A compound according to claim 1, which is selected from:

(±) Ethyl 3-{4-[2-(pyrrol-1-yl)ethoxy]phenyl}-2-ethoxypropanoate
(+) Ethyl 3-{4-[2-(pyrrol-1-yl)ethoxy]phenyl}-2-ethoxypropanoate
(-) Ethyl 3-{4-[2-(pyrrol-1-yl)ethoxy]phenyl}-2-ethoxypropanoate
(±) Ethyl 3-{4-[2-(2,5-dimethylpyrrol-1-yl)ethoxy]phenyl}-2-ethoxypropanoate
(+) Ethyl 3-{4-[2-(2,5-dimethylpyrrol-1-yl)ethoxy]phenyl}-2-ethoxypropanoate
(-) Ethyl 3-{4-[2-(2,5-dimethylpyrrol-1-yl)ethoxy]phenyl}-2-ethoxypropanoate
(±) Ethyl 3-{4-[2-(2,5-diisopropyl-3-phenylpyrrol-1-yl)ethoxy]phenyl}-2-ethoxypropanoate
(+) Ethyl 3-{4-[2-(2,5-diisopropyl-3-phenylpyrrol-1-yl)ethoxy]phenyl}-2-ethoxypropanoate
(-) Ethyl 3-{4-[2-(2,5-diisopropyl-3-phenylpyrrol-1-yl)ethoxy]phenyl}-2-ethoxypropanoate
(±) Ethyl 3-(4-{2-[2-isopropyl-5-(4-methoxyphenyl)pyrrol-1-yl]ethoxy}phenyl)-2-ethoxypropanoate
(+) Ethyl 3-(4-{2-[2-isopropyl-5-(4-methoxyphenyl)pyrrol-1-yl]ethoxy}phenyl)-2-ethoxypropanoate
(-) Ethyl 3-(4-{2-[2-isopropyl-5-(4-methoxyphenyl)pyrrol-1-yl]ethoxy}phenyl)-2-ethoxypropanoate
(±) Ethyl 3-(4-{2-[2-(4-fluorophenyl)-5-isopropylpyrrol-1-yl]ethoxy}phenyl)-2-ethoxypropanoate
(+) Ethyl 3-(4-{2-[2-(4-fluorophenyl)-5-isopropylpyrrol-1-yl]ethoxy}phenyl)-2-ethoxypropanoate
(-) Ethyl 3-(4-{2-[2-(4-fluorophenyl)-5-isopropylpyrrol-1-yl]ethoxy}phenyl)-2-ethoxypropanoate
(±) Ethyl 3-(4-{2-[2-(4-fluorophenyl)-5-isopropyl-3-phenylpyrrol-1-yl]ethoxy}phenyl)-2-ethoxypropanoate
(+) Ethyl 3-(4-{2-[2-(4-fluorophenyl)-5-isopropyl-3-phenylpyrrol-1-yl]ethoxy}phenyl)-2-ethoxypropanoate
(-) Ethyl 3-(4-{2-[2-(4-fluorophenyl)-5-isopropyl-3-phenylpyrrol-l-yl]ethoxy}phenyl)-2-ethoxypropanoate
(±) Ethyl 3-(4-{2-[2-(4-fluorophenyl)-5-phenylpyrrol-1-yl]ethoxy}phenyl)-2-ethoxypropanoate
(+) Ethyl 3-(4-{2-[2-(4-fluorophenyl)-5-phenylpyrrol-1-yl]ethoxy}phenyl)-2-ethoxypropanoate
(-) Ethyl 3-(4-{2-[2-(4-fluorophenyl)-5-phenylpyrrol-1-yl]ethoxy}phenyl)-2-ethoxyropanoate
(±) Ethyl 3-[4-[2-[2-(2-phenyl-3-carboxy-5-(4-fluorophenyl)pyrrol-1-yl)ethoxy]phenyl-2-ethoxypropanoate
(+) Ethyl 3-[4-[2-[2-(2-phenyl-3-carboxy-5-(4-fluorophenyl)pyrrol-1-yl)ethoxylphenyl-2-ethoxypropanoate
(-) Ethyl 3-[4-[2-[2-(2-phenyl-3-carboxy-5-(4-fluorophenyl)pyrrol-1-yl)ethoxy]phenyl-2-ethoxypropanoate
(±) Ethyl 3-(4-{2-[2-(4-fluorophenyl)-5-isopropyl-3-phenyl-4-phenylcarbamoylpyrrol-1-yl]ethoxy}phenyl)-2-ethoxypropanoate
(+) Ethyl 3-(4-{2-[2-(4-fluorophenyl)-5-isopropyl-3-phenyl-4-phenylcarbamoylpyrrol-1-yl]ethoxy}phenyl)-2-ethoxypropanoate
(-) Ethyl 3-(4-{2-[2-(4-fluorophenyl)-5-isopropyl-3-phenyl-4-phenylcarbamoylpyrrol-1-yl]ethoxy}phenyl)-2-ethoxypropanoate
(±) Ethyl 3-(4-{2-[2-(4-fluorophenyl)-5-isopropyl-3-phenyl-4-phenylcarbamoylpyrrol-1-yl]propoxy}phenyl)-2-ethoxypropanoate
(+) Ethyl 3-(4-{3-[2-(4-fluorophenyl)-5-isopropyl-3-phenyl-4-phenylcarbamoylpyrrol-1-yl]propoxy}phenyl)-2-ethoxypropanoate

(-) Ethyl 3-(4-{3-[2-(4-fluorophenyl)-5-isopropyl-3-phenyl-4-phenylcarbamoylpyrrol-1-yl]propoxy}phenyl)-2-ethoxypropanoate
(±) Ethyl 3-(4-{2-[2-isopropyl-5-methylpyrrol-1-yl)ethoxy]phenyl}-2-ethoxypropanoate
(+) Ethyl 3-{4-[2-(2-isopropyl-5-methylpyrrol-1-yl)ethoxy]phenyl}-2-ethoxypropanate
(-) Ethyl 3-{4-[2-[2-isopropyl-5-methylpyrrol-1-yl)ethoxy]phenyl}-2-ethoxypxopanoate
(±) 3-{4-[2-(pyrrol-1-yl)ethoxy]phenyl}-2-ethoxypropanoic acid and its pharmaceutically acceptable salts
(+) 3-{4-[2-(pyrrol-1-yl)ethoxy]phenyl}-2-ethoxypropanoic acid and its pharmaceutically acceptable salts
(-) 3-{4-[2-(pyrrol-1-yl)ethoxy]phenyl}-2-ethoxypropanoic acid and its pharmaceutically acceptable salts
(±) 3-{4-[2-(2,5-dimethylpyrrol-1-yl)ethoxylphenyl}-2-ethoxypropanoic acid and its pharmaceutically acceptable salts
(+) 3-{4-[2-(2,5-dimethylpyrrol-1-yl)ethoxy]phenyl}-2-ethoxypropanoic acid and its pharmaceutically acceptable salts
(-) 3-{4-[2-(2,5-dimethylpyrrol-1-yl)ethoxy]phenyl}-2-ethoxypropanoic acid and its pharmaceutically acceptable salts
(±) 3-{4-[2-(2,5-diisopropyl-3-phenylpyrrol-1-yl)ethoxy]phenyl}-2-ethoxypropanoic acid and its pharmaceutically acceptable salts
(+) 3-{4-[2-(2,5-diisopropyl-3-phenylpyrrol-1-yl)ethoxy]phenyl}-2-ethoxypropanoic acid and its pharmaceutically acceptable salts
(-) 3-{4-[2-(2,5-diisopropyl-3-phenylpyrrol-1-yl)ethoxy]phenyl}-2-ethoxypropanoic acid and its pharmaceutically acceptable salts
(±) 3-(4-{2-[2-isopropyl-5-(4-methoxyphenyl)pyrrol-1-yl]ethoxy}phenyl)-2-ethoxypropanoic acid and its pharmaceutically acceptable salts
(+) 3-(4-{2-[2-isopropyl-5-(4-methoxyphenyl)pyrrol-1-yl]ethoxy}phenyl)-2-ethoxypropanoic acid and its pharmaceutically acceptable salts
(-) 3-(4-{2-[2-isopropyl-5-(4-methoxyphenyl)pyrrol-1-yl]ethoxy}phenyl)-2-ethoxypropanoic acid and its pharmaceutically acceptable salts
(±) 3-(4-{2-[2-(4-fluorophenyl)-5-isopropylpyrrol-1-yl]ethoxy}phenyl)-2-ethoxypropanoic acid and its pharmaceutically acceptable salts
(+) 3-(4-{2-[2-(4-fluorophenyl)-5-isopropylpyrrol-1-yl]ethoxy}phenyl)-2-ethoxypropanoic acid and its pharmaceutically acceptable salts
(-) 3-(4-{2-[2-(4-fluorophenyl)-5-isopropylpyrrol-1-yl]ethoxy}phenyl)-2-ethoxypropanoic acid and its pharmaceutically acceptable salts
(±) 3-(4-(2-[2-(4-fluorophenyl)-5-isopropyl-3-phenylpyrrol-1-yl]ethoxy)phenyl)-2-ethoxypropanoic acid and its pharmaceutically acceptable salts
(+) 3-(4-(2-[2-(4-fluorophenyl)-5-isopropyl-3-phenylpyrrol-l-yl]ethoxy)phenyl)-2-ethoxypropanoic acid and its pharmaceutically acceptable salts
(-) 3-(4-(2-[2-(4-fluorophonyl)-5-isopropyl-3-phenylpyrrol-1-yl]ethoxy)phenyl)-2-ethoxypropanoic acid and its pharmaceutically acceptable salts
(±) 3-(4-(2-[2-(4-fluorophenyl)-5-phenylpyrrol-1-yl]ethoxy)phenyl)-2-ethoxypropanoic acid and its pharmaceutically acceptable salts
(+) 3-(4-{2-[2-(4-fluorophenyl)-5-phenylpyrrol-1-yl]ethoxy}phenyl)-2-ethoxypropanoic acid and its pharmaceutically acceptable salts
(-) 3-(4-{2-[2-(4-fluorophenyl)-5-phenylpyrrol-1-yl]ethoxy}phenyl)-2-ethoxypropanoic acid and its pharmaceutically acceptable salts
(±) 3-[4-[2-(2-phenyl-3-carbethoxy-5-(4-fluorophenyl)pyrrol-1-yl)ethoxy]phenyl]-2-ethoxypropanoic acid and its pharmaceutically acceptable salts
(+) 3-[4-[2-(2-phenyl-3-carbethoxy-5-(4-fluorophenyl)pyrrol-1-yl)ethoxy]phenyl]-2-ethoxypropanoic acid and its pharmaceutically acceptable salts
(-) 3-[4-[2-(2-phenyl-3-carbethoxy-5-(4-fluorophenyl)pyrrol-1-yl)ethoxy]phenyl]-2-ethoxypropanoic acid and its pharmaceutically acceptable salts
(±) 3-(4-{2-[2-(4-fluorophenyl)-5-isopropyl-3-phenyl-4-phenylcarbamoylpyrrol-1-yl]ethoxy}phenyl)-2-ethoxypropanoic acid and its pharmaceutically acceptable salts
(+) 3-(4-{2-[2-(4-fluorophenyl)-5-isopropyl-3-phenyl-4-phenylcarbamoylpyrrol-1-yl]ethoxy}phenyl)-2-ethoxypropanoic acid and its pharmaceutically acceptable salts
(-) 3-(4-{2-[2-(4-fluorophenyl)-5-isopropyl-3-phenyl-4-phenylcarbamoylpyrrol-1-yl]ethoxy}phenyl)-2-ethoxypropanoic acid and its pharmaceutically acceptable salts
(±) 3-(4-{3-[2-(4-fluorophenyl)-5-isopropyl-3-phenyl-4-phenylcarbamoylpyrrol-1-yl]propoxy}phenyl)-2-ethoxypropanoic acid and its pharmaceutically acceptable salts

(+) 3-(4-{3-[2-(4-fluorophenyl)-5-isopropyl-3-phenyl-4-phenylcarbamoylpyrrol-1-yl]propoxy}phenyl)-2-ethoxypropanoic acid and its pharmaceutically acceptable salts
(-) 3-(4-{3-[2-(4-fluorophenyl)-5-isopropyl-3-phenyl-4-phenylcarbamoylpyrrol-1-yl]propoxy}phenyl)-2-ethoxypropanoic acid and its pharmaceutically acceptable salts
(±) Ethyl 3-{4-[2-(2-isopropyl-5-methylpyrrol-1-yl)ethoxy]phenyl}-2-ethoxypropanoic acid and its pharmaceutically acceptable salts
(+) Ethyl 3-{4-[2-(2-isopropyl-5-methylpyrrol-1-yl)ethoxy]phenyl}-2-ethoxypropanoic acid and its pharmaceutically acceptable salts
(-) Ethyl 3-{4-[2-(2-isopropyl-5-methylpyrrol-1-yl)ethoxy]phenyl}-2-ethoxypropanoic acid and its pharmaceutically acceptable salts.

**4.** A process for the preparation of a compound of formula (I) according to claim 1 selected from;

a. reacting a compound of formula (1a),

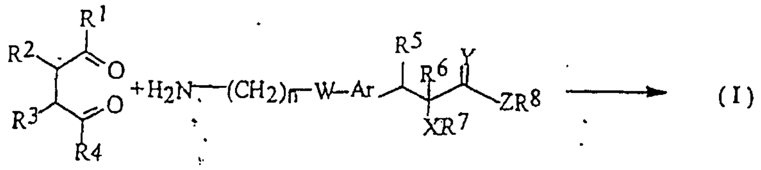

(1a) (1b)

where all the symbols are as defined earlier, with a compound of formula (1b) which may be racemic or chiral, where all symbols are as defined earlier to yield a compound of formula (I) where all symbols are as defined earlier;
b. reacting a compound of formula (1c),

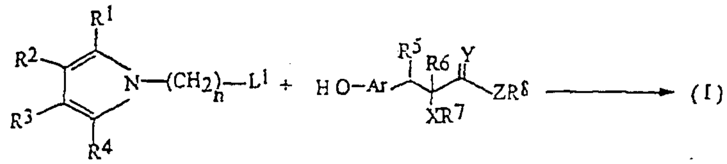

(1c) (1d)

where all the symbols are as defined earlier and $L^1$ represents a leaving group, with a compound of formula (1d) which may be racemic or chiral, wherein all symbols are as defined earlier;
c. reacting the compound of formula (1e),

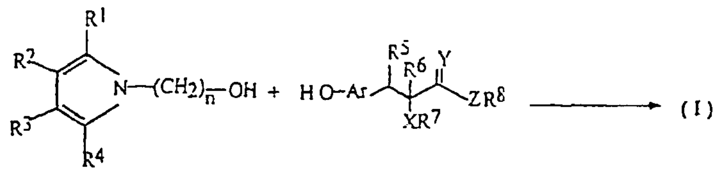

(1e) (1d)

where all symbols are as defined earlier with a compound of general formula (1d) which may be racemic or chiral, defined earlier;
d. reacting a compound of formula (1f)

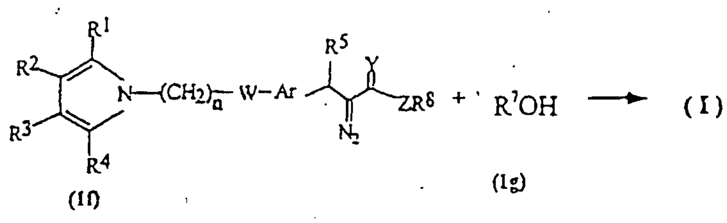

(1f) (1g)

where all the symbols are as defined earlier with an alcohol of formula (1g) wherein $R^7$ is as defined earlier except H to produce a compound of formula (I), to produce a compound of general formula (I) wherein all symbols are as defined earlier and X represents 'O' atoms;

e. reacting a compound of general formula (1h),

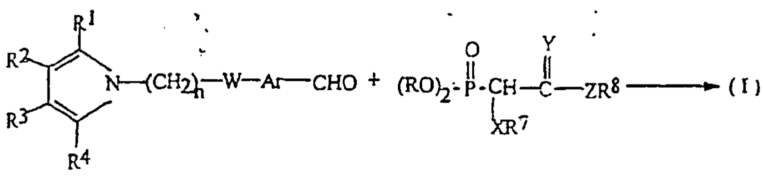

(1h) (1i)

where all the symbols are as defined earlier, with a compound of formula (1i) which may be chiral or racemic, where all the symbols are as defined earlier and R represents ($C_1$-$C_8$) alkyl to afford a compound of formula (I) wherein all symbols are as defined earlier and $R^5$ and $R^6$ together form a bond.

**5.** A process according to the claim 4, comprising of carrying out one or more of the following optional steps:

i. Converting a compound of formula (I) into a further compound of formula (I);
ii. Removing any protecting group;
iii. Resolving the racemic mixture into pure enantiomers by the known methods and
iv. Preparing a pharmaceutically acceptable salt of a compound of formula (I) and/or a pharmaceutically acceptable solvate thereof.

**6.** A compound of the general formula (I), its tautomeric forms, its stereoisomers, its polymorphs, its pharmaceutically acceptable salts and its pharmaceutically acceptable solvates for preparing a medicament.

**7.** A pharmaceutical composition which comprises a compound according to general formula (I), their tautomeric forms, their stereoisomers, their polymorphs, their pharmaceutically acceptable salts, their pharmaceutically acceptable solvates and pharmaceutically acceptable compositions containing them.

**8.** A pharmaceutical composition which comprises a compound according to general formula (I), according to claim 7, in the form of a tablet, capsule, powder, syrup, solution or suspension.

**9.** Use of a compound of formula (I), as defined in claim 1 or claim 2 or a pharmaceutical composition as claimed in claim 7 and 8, for the manufacture of a medicament for the treatment and/or prophylaxis of hyperglycemia, hyperlipidemia, hypertension, cardiovascular disease and certain eating disorders.

**10.** An intermediate defined by the formula (1h),

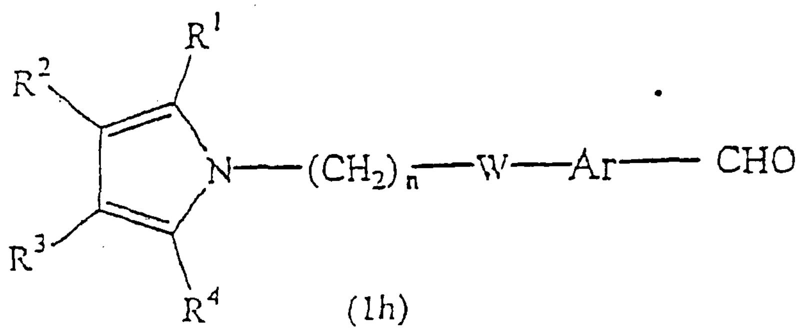

(1h)

wherein one or more groups $R^1$, $R^2$, $R^3$, $R^4$ , W, Ar and n are as defined in Claim 1 optionally containing one or more double bonds and optionally containing one or more heteroatoms selected from O, N, or S.

**11.** A process provided for the preparation of intermediate of the general formula (1h), which comprises

a. reacting the compound of the general formula (1c) where $R^1$, $R^2$, $R^3$, $R^4$ and n are as defined in claim 1,

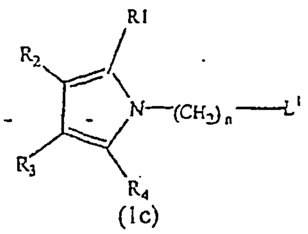

(1c)

$OH-Ar-CHO$

(1j)

with the compound of general formula (1j), where Ar is as defined in claims 1 and 2,
b. reacting the compound of the formula (1e) where $R^1$, $R^2$, $R^3$, $R^4$ and n are as defined in Claim 1,

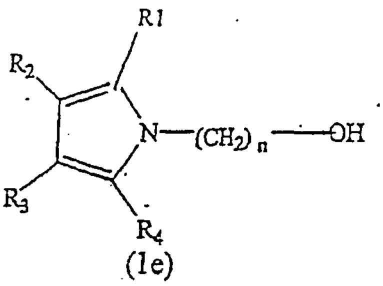

(1e)

$L^2-Ar-CHO$

(1k)

with compound of the formula (1k), where $L^2$ is fluorine, chlorine, bromine or iodine and Ar is as defined in the claims 1 and 2.

**Patentansprüche**

**1.** Verbindung, die durch die Formel (I) dargestellt wird:

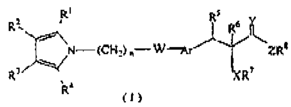

(I)

ihre tautomeren Formen, ihre Stereoisomeren, ihre pharmazeutisch annehmbaren Salze und ihre pharmazeutisch

annehmbaren Solvate,
worin eine oder mehrere der Gruppen $R^1$, $R^2$, $R^3$, $R^4$ gleich oder unterschiedlich sein können und Wasserstoff, Halogen, Perhalogenalkyl, Amino, Nitro, Cyano, Formyl, Amidino, Guanidino, substituierte oder unsubstituierte Gruppen, ausgewählt aus

- $(C_1-C_{12})$-Alkyl-Gruppen;
- linearen oder verzweigten $(C_2-C_{12})$-Alkenyl-Gruppen;
- $(C_3-C_7)$-Cycloalkyl-Gruppen;
- $(C_3-C_7)$-Cycloalkenyl-Gruppen;
- Bicycloalkyl, ausgewählt aus Bicyclooctan, Bicyclononan und Bicyclodecan; Bicycloalkenyl, ausgewählt aus Bicyclooocten, Bicyclononen und Bicyclodecen;
- $(C_1-C_{12})$-Alkoxy-Gruppen;
- Cyclo-$(C_3-C_7)$-alkoxy-Gruppen;
- Aryl-Gruppen, ausgewählt aus Phenyl-, Naphthyl-, Benzo[1,3]dioxol- oder Biphenyl-Gruppen;
- Aryloxy-Gruppen, ausgewählt aus Phenoxy-, Naphthyloxy-, Biphenyloxy-Gruppen;
- Aralkyl-Gruppen, wobei Aryl- und Alkyl-Gruppen wie vorher definiert sind;
- Aralkoxygruppen, wobei Arylgruppe wie vorher definiert ist;
- Heteroaryl-Gruppen, ausgewählt aus Pyridyl-, Thienyl-, Furyl-, Pyrrolyl-, Oxazolyl-, Thiazolyl-, Imidazolyl-, Oxadiazolyl-, Tetrazolyl-, Benzopyranyl-, Benzofvranyl-Gruppen;
- Heteroaralkyl-Gruppen, ausgewählt aus Furanmethyl-, Pyridinmethyl-, Oxazolmethyl-, Oxazolethyl-Gruppen;
- Heterocyclyl-Gruppen, ausgewählt aus Aziridinyl, Pyrrolidinyl, Piperidinyl;
- Heterocyclylalkyl-Gruppen, ausgewählt aus Pyrrolidinalkyl, Piperidinalkyl-, Morpholinalkyl, Thiomorpholinalkyl, Oxazolinalkyl;
- Heterocycloalkoxy-Gruppen, worin die Heterocyclyl- und Alkoxy-Gruppe wie vorher definiert ist;
- Heteroaryloxy-Gruppen, worin die Heteroaryl-Gruppe wie vorher definiert ist;
- Heteroaralkoxy-Gruppen, worin die Heteroaralkyl-Gruppe wie vorher definiert ist;
- Acyl-Gruppen, ausgewählt aus Acetyl-, Propionyl- oder Benzoyl-Gruppen;
- Acyloxy-Gruppen, ausgewählt aus MeCOO-, EtCOO-, PhCOO-Gruppen;
- Acylamino-Gruppen, ausgewählt aus $CH_3CONH$-, $C_2H_5CONH$-, $C_3H_7CONH$-, $C_5H_5CONH$-Gruppen;
- $(C_1-C_6)$-Monoalkylamino-Grupoen;
- $(C_1-C_6)$-Dialkylamino-Gruppen;
- Arylamino, worin die Aryl-Gruppe wie vorher definiert ist,;
- Aralkylamino-Gruppen, worin die Aryl-Gruppe wie vorher definiert ist;
- Alkoxycarbonyl, worin die Alkoxy-Gruppe wie vorher definiert ist;
- Aryloxycarbonyl, worin die Aryloxy-Gruppe wie vorher definiert ist;
- Aralkoxycarbonyl, worin die Aralkoxy-Gruppe wie vorher definiert ist;
- Heterocycloalkoxycarbonyl, worin die Heterocycloalkoxy-Gruppe wie vorher definiert ist;
- Heteroaryloxycarbonyl, worin die Heteroaryloxy-Gruppe wie vorher definiert ist;
- Heteroaralkoxycarbonyl, worin die Heteroaralkoxy-Gruppe wie vorher definiert ist;
- Hydroxyalkyl, worin die Alkyl-Gruppe wie vorher definiert ist;
- Aminoalkyl, worin die Alkyl-Gruppe wie vorher definiert ist;
- mono- oder disubstituiertem Alkylaminoalkyl, worin die Alkyl-Gruppe wie vorher definiert ist und die substituierte Gruppe ausgewählt ist aus Alkyl-, Cycloalkyl-, Alkoxy-, Cycloalkoxy-, Aryl-, Aralkyl-, Aralkoxyalkyl-, Heterocyclyl-, Heteroaryl-, Heteroaralkyl-, Acyl-, Acyloxy-, Hydroxyalkyl-, Amino-, Acyl-amino-, Arylamino-, Aminoalkyl-, Aryloxy-, Aralkoxy-, Alkylamino-, Alkoxy-alkyl-, Alkylthio- oder Thioalkyl-Gruppen;
- Alkoxyalkyl, worin die Alkyl-Gruppe wie vorher definiert ist;
- Aryloxyalkyl, worin die Alkyl-Gruppe wie vorher definiert ist;
- Aralkoxyalkyl, worin die Alkyl-Gruppe wie vorher definiert ist;
- Alkylthio- oder Thioalkyl-Gruppen, worin die Alkyl-Gruppe wie vorher definiert ist;
- Alkoxycarbonylamino, worin die Alkoxycarbonyl-Gruppe wie vorher definiert ist;
- Aryloxycarbonylamino, worin die Aryloxycarbonyl-Gruppe wie vorher definiert ist;
- Aralkyloxycarbonylamino, worin die Aralkyloxycarbonyl-Gruppe wie vorher definiert ist;
- Aminocarbonylamino, worin die Aminocarbonyl-Gruppe wie vorher definiert ist;
- Hydrazino, Hydroxylamino; Carbonsäure und ihren Derivaten, ausgewählt aus $(C_1-C_3)$-Estern, darstellen, wobei die Substituenten an $R^1$, $R^2$, $R^3$ und $R^4$ aus Alkyl-, Cycloalkyl-, Alkoxy-, Cycloalkoxy-, Aryl-, Aralkyl-, Aralkoxyalkyl-, Heterocyclyl-, Heteroaryl-, Heteroaralkyl-, Acyl-, Acyloxy-, Hydroxyalkyl-, Amino-, Acylamino-, Arylamino-, Aminoalkyl-, Aryloxy-, Aralkoxy-, Alkylamino-, Alkoxyalkyl-, Alkylthio-, Thioalkyl-Gruppen ausgewählt sind, wobei jeder der Ausdrücke wie vorher definiert ist;

n eine ganze Zahl im Bereich von 1 bis 8 ist; W für O, S oder $NR^9$ steht, worin $R^9$ Alkyl oder Aryl, wie sie vorher definiert wurden, darstellt; Ar eine substituierte oder unsubstituierte zweiwertige einzelne oder kondensierte aromatische, heteroaromatische oder heterocyclische Gruppe darstellt, wobei jeder dieser Ausdrücke aus solchen ausgewählt ist, wie sie vorher definiert wurden, die Substituenten an "Ar" aus linearem oder verzweigtem Alkyl, Alkoxy, Halogen, Halogenalkyl, Halogenalkoxy, Acyl, Amino, Acylamino, Thio oder Carbon- oder Sulfonsäuren ausgewählt sind; $R^5$ und $R^6$ beide Wasserstoff darstellen oder zusammen eine Bindung darstellen; $R^5$ und $R^6$ auch zusammen eine Hydroxy-, Alkyl-, Alkoxy-, Halogen-, Acyl-, Aralkylgruppen darstellen können, wobei jeder dieser Ausdrücke aus denen, wie sie vorher definiert wurden, ausgewählt ist; X für O oder S steht; $R^7$ Wasserstoff, Perfluoralkyl oder Gruppen, ausgewählt aus Alkyl-, Cycloalkyl-, Aryl-, Aralkyl-, Heteroaryl-, Heteroaralkyl-, Alkoxyalkyl-, Aryloxyalkyl-, Alkoxycarbonyl-, Aryloxycarbonyl-, Cycloalkyloxycarbonyl-, Alkylaminocarbonyl-, Arylaminocarbonyl-, Acyl-Gruppen, darstellt, wobei jeder dieser Ausdrükke wie vorher definiert ist; Y für O oder S steht; Z Sauerstoff oder $NR^{10}$ darstellt, worin $R^{10}$ Wasserstoff oder Gruppen, ausgewählt aus Alkyl-, Aryl-, Aralkyl-, Hydroxyalkyl-, Aminoalkyl-, Heteroaryl-, Heteroaralkyl-Gruppen, darstellt, wobei jeder dieser Ausdrücke aus denen, wie sie vorher definiert wurden, ausgewählt ist; $R^8$ Wasserstoff, Gruppen, ausgewählt aus Alkyl-, Aryl-, Aralkyl-, Heteroaryl-, Heteroaralkyl-, Hydroxyalkyl-, Alkoxyalkyl-, Alkylaminoalkyl-Gruppen, darstellt, wobei jeder dieser Ausdrücke wie vorher definiert ist;

$R^{10}$ und $R^8$ zusammen eine 5- oder 6-gliedrige substituierte oder unsubstituierte cyclische Ringstruktur, enthaltend Kohlenstoffatome oder enthaltend ein oder mehrere Heteroatom(e), ausgewählt aus O, N und S, bilden können, wobei die cyclischen Strukturen solche sind, wie sie vorher beansprucht wurden, wobei an der cyclischen Struktur, die durch $R^8$ und $R^{10}$ zusammengenommen gebildet wird, Substituenten aus Halogen, Hydroxy, Alkyl, Oxo, Aralkyl ausgewählt sind.

**2.** Verbindung gemäß Anspruch 1, wobei Ar substituierte oder unsubstituierte zweiwertige Phenylen-, Naphthylen-Gruppen darstellt und substituierte Gruppen aus linearem oder verzweigtem Alkyl, Alkoxy, Halogen, Halogenalkyl, Halogenalkoxy, Acyl, Amino, Acylamino, Thio oder Carbon- oder Sulfonsäuren ausgewählt sind.

**3.** Verbindung gemäß Anspruch 1, die ausgewählt ist aus:

(±)-Ethyl-3-{4-[2-(pyrrol-1-yl)ethoxy]phenyl}-2-ethoxypropanoat;
(+)-Ethyl-3-{4-[2-(pyrrol-1-yl)ethoxy]phenyl}-2-ethoxypropanoat;
(-)-Ethyl-3-{4-[2-(pyrrol-1-yl)ethoxy]phenyl}-2-ethoxypropanoat;
(±)-Ethyl-3-{4-[2-(2,5-dimethylpyrrol-1-yl)ethoxy]phenyl}-2-ethoxypropanoat;
(+)-Ethyl-3-{4-[2-(2,5-dimethylpyrrol-1-yl)ethoxy]phenyl}-2-ethoxypropanoat;
(-)-Ethyl-3-{4-[2-(2,5-dimethylpyrryl-1-yl)ethoxy]phenyl}-2-ethoxypropanoat;
(±)-Ethyl-3-{4-[2-(2,5-diisopropyl-3-phenylpyrrol-1-yl)ethoxy]phenyl}-2-ethoxypropanoat;
(+)-Ethyl-3-{4-[2-(2,5-diisopropyl-3-phenylpyrrol-1-yl)ethoxy]phenyl}-2-ethoxypropanoat;
(-)-Ethyl-3-{4-[2-(2,5-diisopropyl-3-phenylpyrrol-1-yl)ethoxy]phenyl}-2-ethoxypropanoat;
(±)-Ethyl-3-(4-{2-[2-isopropyl-5-(4-methoxyphenyl)pyrrol-1-yl]ethoxy}phenyl)-2-ethoxypropanoat;
(+)-Ethyl-3-(4-{2-[2-isopropyl-5-(4-methoxyphenyl)pyrrol-1-yl]ethoxy}phenyl)-2-ethoxypropanoat;
(-)-Ethyl-3-(4-{2-[2-isopropyl-5-(4-methoxyphenyl)pyrrol-1-yl]ethoxy}phenyl)-2-ethoxypropanoat;
(±)-Ethyl-3-(4-{2-[2-(4-fluorphenyl)-5-isopropylpyrrol-1-yl]ethoxy}phenyl)-2-ethoxypropanoat;
(+)-Ethyl-3-(4-{2-[z-(4-fluorphenyl)-5-isopropylpyrrol-1-yl]ethoxy}phenyl)-2-ethoxypropanoat;
(-)-Ethyl-3-(4-{2-[2-(4-fluorphenyl)-5-isopropylpyrrol-1-yl]ethoxy}phenyl)-2-ethoxypropanoat;
(±)-Ethyl-3-(4-{2-{2-(4-fluorphenyl)-5-isopropyl-3-phenylpyrrol-1-yl]ethoxy]phenyl)-2-ethoxypropanoat;
(+)-Ethyl-3-(4-{2-{2-(4-fluorphenyl)-5-isopropyl-3-phenylpyrrol-1-yl]ethoxy]phenyl)-2-ethoxypropanoat;
(-)-Ethyl-3-(4-{2-{2-(4-fluorphenyl)-5-isopropyl-3-phenylpyrrol-1-yl]ethoxy]phenyl)-2-ethoxypropanoat;
(±)-Ethyl-3-(4-{2-[2-(4-fluorphenyl)-5-phenylpyrrol-1-yl]ethoxy}phenyl)-2-ethoxy-propanoat;
(+)-Ethyl-3-(4-{2-[2-(4-fluorphenyl)-5-phenylpyrrol-1-yl]ethoxy}phenyl)-2-ethoxy-propanoat;
(-)-Ethyl-3-(4-{2-[2-(4-fluorphenyl)-5-phenylpyrrol-1-yl]ethoxy}phenyl)-2-ethoxy-propanoat;
(+)-Ethyl-3-[4-[2-[2-(2-phenyl-3-carboxy-5-(4-fluorphenyl)pyrrol-1-yl)ethoxy]phenyl-2-ethoxypropanoat;
(+)-Ethyl-3-[4-[2-[2-(2-phenyl-3-carboxy-5-(4-fluorphenyl)pyrrol-1-yl)ethoxy]phenyl-2-ethoxypropanoat;
(-)-Ethyl-3-[4-[2-[2-(2-phenyl-3-carboxy-5-(4-fluorphenyl)pyrrol-1-yl)ethoxy]phenyl-2-ethoxypropanoat;
(±)-Ethyl-3-(4-{2-[2-(4-fluorphenyl)-5-isopropyl-3-phenyl-4-phenylcarbamoylpyrrol-1-yl]ethoxy}phenyl)-2-ethoxypropanoat;
(+)-Ethyl-3-(4-{2-[2-(4-fluorphenyl)-5-isopropyl-3-phenyl-4-phenylcarbamoylpyrrol-1-yl]ethoxy}phenyl)-2-ethoxypropanoat;
(-)-Ethyl-3-(4-{2-[2-(4-fluorphenyl)-5-isopropyl-3-phenyl-4-phenylcarbamoylpyrrol-1-yl]ethoxy}phenyl)-2-ethoxypropanoat;

(±)-Ethyl-3-(4-{2-[2-(4-fluorphenyl)-5-isopropyl-3-phenyl-4-phenylcarbamoylpyrrol-1-yl]propoxy}phenyl)-2-ethoxypropanoat;
(+)-Ethyl-3-(4-{2-[2-(4-fluorphenyl)-5-isopropyl-3-phenyl-4-phenylcarbamoylpyrrol-1-yl]propoxy}phenyl)2-ethoxypropanoat;
(-)-Ethyl-3-(4-{2-[2-(4-fluorphenyl)-5-iscpropyl-3-phenyl-4-phenylcarbamoylpyrrol-1-yl]propoxy}phenyl)-2-ethoxypropanoat;
(±)-Ethyl-3-(4-{2-[2-iscpropyl-5-methylpyrrol-1-yl)ethoxy]phenyl}-2-ethoxy-propanoat;
(+)-Ethyl-3-(4-{2-[2-isopropyl-5-methylpyrrol-1-yl)ethoxy]phenyl}2-ethoxypropanoat;
(-)-Ethyl-3-(4-{2-[2-isopropyl-5-methylpyrrol-1-yl)ethoxy]phenyl0-2-ethoxypropanoat;
(±)-3-{4-[2-(Pyrrol-1-yl)ethoxy]phenyl}-2-ethoxypropansäure und ihren pharmazeutisch annehmbaren Salzen;
(+)-3-{4-[2-(Pyrrol-1-yl)ethoxy]phenyl}-2-ethoxypropansäure und ihren pharmazeutisch annehmbaren Salzen;
(-)-3-{4-[2-(Pyrrol-1-yl)ethoxy]phenyl}-2-ethoxypropansäure und ihren pharmazeutisch annehmbaren Salzen;
(±)-3-{4-[2-(2,5-Dimethylpyrrol-1-yl)ethoxy]phenyl}-2-ethoxypropansäure und ihren pharmazeutisch annehmbaren Salzen;
(+)-3-{4-[2-(2,5-Dimethylpyrrol-1-yl)ethoxy]phenyl}-2-ethoxypropansäure und ihren pharmazeutisch annehmbaren Salzen;
(-)-3-{4-[2-(2,5-Dimethylpyrrol-1-yl)ethoxy]phenyl)-2-ethoxypropansäure und ihren pharmazeutisch annehmbaren Salzen;
(±)-3-{4-[2-(2,5-Diisopropyl-3-phenylpyrrol-1-yl)ethoxy]phenyl}-2-ethoxypropansäure und ihren pharmazeutisch annehmbaren Salzen;
(+)-3-{4-[2-(2,5-Diisopropyl-3-phenylpyrrol-1-yl)ethoxy]phenyl}-2-ethoxypropansäure und ihren pharmazeutisch annehmbaren Salzen;
(-)-3-{4-[2-(2,5-Diisopropyl-3-phenylpyrrol-1-yl)ethoxy]phenyl}-2-ethoxypropansäure und ihren pharmazeutisch annehmbaren Salzen;
(±)-3-{4-[2-(2-Isopropyl-5-(4-methoxyphenyl)pyrrol-1-yl]ethoxy}phenyl)-2-ethoxy-propansäure und ihren pharmazeutisch annehmbaren Salzen;
(+)-3-{4-[2-(2-Isopropyl-5-(4-methoxyphenyl)pyrrol-1-yl]ethoxy}phenyl)-2-ethoxy-propansäure und ihren pharmazeutisch annehmbaren Salzen;
(-)-3-{4-[2-(2-Isopropyl-5-(4-methoxyphenyl)pyrrol-1-yl]ethoxy}phenyl)-2-ethoxy-propansäure und ihren pharmazeutisch annehmbaren Salzen;
(±)-3-(4-{2-[2-(4-Fluorphenyl)-5-isopropylpyrrol-1-yl]ethoxy}phenyl)-2-ethoxy-propansäure und ihren pharmazeutisch annehmbaren Salzen;
(+)-3-(4-(2-[2-(4-Fluorphenyl)-5-iscpropylpyrrol-1-yt]ethoxy}phenyl)-2-ethoxy-propansäure und ihren pharmazeutisch annehmbaren Salzen;
(-)-3-(4-{2-[2-(4-Fluorphenyl)-5-isopropylpyrrol-1-yl]ethoxy}phenyl)-2-ethoxy-propansäure und ihren pharmazeutisch annehmbaren Salzen;
(±)-3-(4-{2-[2-(4-Fluorphenyl)-5-isopropyl-3-phenylpyrrol-1-yl]ethoxy}phenyl)-2-ethoxypropansäure und ihren pharmazeutisch annehmbaren Salzen;
(+)-3-(4-{2-[2-(4-Fluorphenyl)-5-isopropyl-3-phenytpyrrol-1-yl]ethoxy}phenyl)-2-ethoxypropansäure und ihren pharmazeutisch annehmbaren Salzen;
(-)-3-(4-{2-[2-(4-Fluorphenyl)-5-isopropyl-3-phenylpyrrol-1-yl]ethoxy}phenyl)-2-ethoxypropansäure und ihren pharmazeutisch annehmbaren Salzen;
(±)-3-(4-{2-[2-(4-Fluorphenyl)-5-phenylpyrrol-1-yl]ethoxy}phenyl)-2-ethoxy-propansäure und ihren pharmazeutisch annehmbaren Salzen;
(+)-3-(4-{2-[2-(4-Fluorphenyl)-5-phenylpyrrol-1-yl]ethoxy}phenyl)-2-ethoxy-propansäure und ihren pharmazeutisch annehmbaren Salzen;
(-)-3-(4-{2-[2-(4-Fluorphenyl)-5-phenylpyrrol-1-yl]ethoxy}phenyl)-2-ethoxy-propansäure und ihren pharmazeutisch annehmbaren Salzen;
(±)-3-[4-[2-(2-Phenyl-3-carbethoxy-5-(4-fluorphenyl)pyrrol-1-yl)ethoxy]phenyl]-2-ethoxypropansäure und ihren pharmazeutisch annehmbaren Salzen;
(+)-3-[4-[2-(2-Phenyl-3-carbethoxy-5-(4-fluorphenyl)pyrrol-1-yl)ethoxy]phenyl]-2-ethoxypropansäure und ihren pharmazeutisch annehmbaren Salzen;
(-)-3-[4-[2-(2-Phenyl-3-carbethoxy-5-(4-fluorphenyl)pyrrol-1-yl)ethoxy]phenyl]-2-ethoxypropansäure und ihren pharmazeutisch annehmbaren Salzen;
(±)-3-(4-{2-[2-(4-Fluorphenyl)-5-isopropyl-3-phenyl-4-phenylcarbamoylpyrrol-1-yl]ethoxy]phenyl)-2-ethoxypropansäure und ihren pharmazeutisch annehmbaren Salzen;
(+)-3-(4-{2-[2-(4-Fluorphenyl)-5-isopropyl-3-phenyl-4-phenylcarbamoylpyrrol-1-yl]ethoxylphenyl)-2-ethoxypropansäure und ihren pharmazeutisch annehmbaren Salzen;

(-)-3-(4-{2-[2-(4-Fluorphenyl)-5-isopropyl-3-phenyl-4-phenylcarbamoylpyrrol-1-yl]ethoxy]phenyl)-2-ethoxypropansäure und ihren pharmazeutisch annehmbaren Salzen;
(+)-3-(4-{3-[2-(4-Fluorphenyl)-5-isopropyl-3-phenyl-4-phenylcarbamoylpyrrol-1-yl]propoxy}phenyl)-2-ethoxypropansäure und ihren pharmazeutisch annehmbaren Salzen;
(+)-3-(4-{3-[2-(4-Fluorphenyl)-5-isopropyl-3-phenyl-4-phenylcarbamoylpyrrol-1-yl]propoxy}phenyl)-2-ethoxypropansäure und ihren pharmazeutisch annehmbaren Salzen;
(-)-3-(4-{3-[2-(4-Fluorphenyl)-5-isopropyl-3-phenyl-4-phenylcarbamoylpyrrol-1-yl]propoxy}phenyl)-2-ethoxypropansäure und ihren pharmazeutisch annehmbaren Salzen;
(±)-Ethyl-3-{4-[2-(2-isopropyl-5-methylpyrrol-1-yl)ethoxy]phenyl}-2-ethoxypropan-säure und ihren pharmazeutisch annehmbaren Salzen;
(+)-Ethyl-3-{4-[2-(2-isopropyl-5-methylpyrrol-1-yl)ethoxy]phenyl}-2-ethoxypropan-säure und ihren pharmazeutisch annehmbaren Salzen;
(-)-Ethyl-3-{4-[2-(2-isopropyl-5-methylpyrrol-1-yl)ethoxy]phenyl}2-ethoxypropan-säure und ihren pharmazeutisch annehmbaren Salzen;

**4.** Verfahren für die Herstellung einer Verbindung der Formel (I) gemäß Anspruch 1, ausgewählt aus:

a. Umsetzen einer Verbindung der Formel (1a)

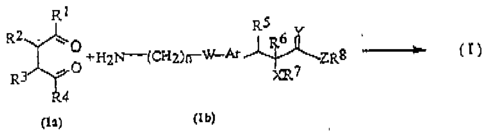

worin alle Symbole wie vorher definiert sind, mit einer Verbindung der Formel (1b), die racemisch oder chiral sein kann, worin alle Symbole wie vorher definiert sind, unter Erhalt einer Verbindung der Formel (I), worin alle Symbole wie vorher definiert sind;
b. Umsetzen einer Verbindung der Formel (1c)

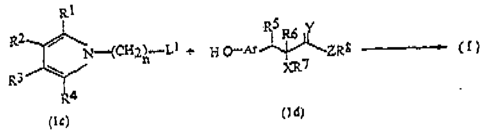

worin alle Symbole wie vorher definiert sind und $L^1$ eine Abgangsgruppe darstellt, mit einer Verbindung der Formel (1d), die racemisch oder chiral sein kann, worin alle Symbole wie vorher definiert sind;
c. Umsetzen einer Verbindung der Formel (1e)

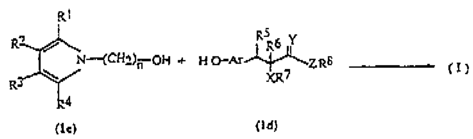

worin alle Symbole wie vorher definiert sind, mit einer Verbindung der allgemeinen Formel (1d), die racemisch oder chiral sein kann, die vorher definiert wurde;
d. Umsetzen einer Verbindung der Formel (1f)

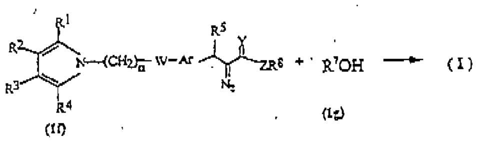

worin alle Symbole wie vorher definiert sind, mit einem Alkohol der Formel (1g), worin $R^7$ wie vorher definiert ist, ausgenommen H, um eine Verbindung der Formel (I) herzustellen, um eine Verbindung der allgemeinen

Formel (I), worin alle Symbole wie vorher definiert sind, und X "O"-Atome darstellt, herzustellen;
e) Umsetzen einer Verbindung der allgemeinen Formel (1h)

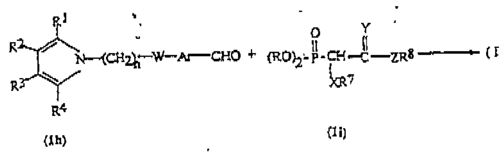

worin alle Symbole wie vorher definiert sind, mit einer Verbindung der Formel (1i), die chiral oder racemisch sein kann, worin alle Symbole wie vorher definiert sind, und R für ($C_1$-$C_8$)-Alkyl steht, um eine Verbindung der Formel (I), worin alle Symbole wie vorher definiert sind, und $R^5$ und $R^6$ zusammen eine Bindung bilden, herzustellen.

**5.** Verfahren gemäß Anspruch 4, das Durchführen eines oder mehrerer der folgenden optionalen Schritte umfasst:

i. Umwandeln einer Verbindung der Formel (I) in eine weitere Verbindung der Formel (I);
ii. Entfernen einer Schutzgruppe;
iii. Trennen des racemischen Gemisches in reine Enantiomere durch die bekannten Verfahren und
iv. Herstellen eines pharmazeutisch annehmbaren Salzes einer Verbindung der Formel (I) und/oder eines pharmazeutisch annehmbaren Solvates davon.

**6.** Verbindung der allgemeinen Formel (I), ihre tautomeren Formen, ihre Stereoisomeren, ihre Polymorphen, ihre pharmazeutisch annehmbaren Salze und ihre pharmazeutisch annehmbaren Solvate zur Herstellung eines Medikaments.

**7.** Pharmazeutische Zusammensetzung, die eine Verbindung gemäß der allgemeinen Formel (I), ihre tautomeren Formen, ihre Stereoisomeren, ihre Polymorphen, ihre pharmazeutisch annehmbaren Salze, ihre pharmazeutisch annehmbaren Solvate und pharmazeutisch annehmbare Zusammensetzungen, die sie enthalten, umfasst.

**8.** Pharmazeutische Zusammensetzung, die eine Verbindung gemäß der allgemeinen Formel (I), gemäß Anspruch 7, in der Form einer Tablette, einer Kapsel, eines Pulvers, eines Sirups, einer Lösung oder einer Suspension umfasst.

**9.** Verwendung einer Verbindung der Formel (I), wie in Anspruch 1 oder Anspruch 2 definiert, oder einer pharmazeutischen Zusammensetzung, wie in Anspruch 7 und 8 beansprucht, zur Herstellung eines Medikaments für die Behandlung und/oder Prophylaxe von Hyperglykämie, Hyperlipidämie, Bluthochdruck, kardiovaskulären Erkrankungen und bestimmten Essstörungen.

**10.** Intermediat, definiert durch die Formel (1h)

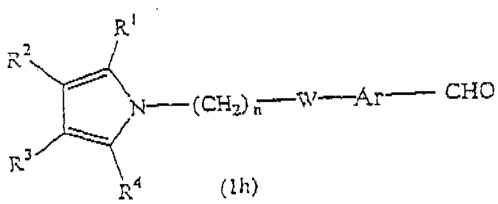

(1h)

worin eine oder mehrere Gruppen $R^1$, $R^2$, $R^3$, $R^4$, W, Ar und n wie in Anspruch 1 definiert sind, optional enthaltend eine oder mehrere Doppelbindungen und optional enthaltend ein oder mehrere Heteroatom(e), ausgewählt aus O, N oder S.

**11.** Verfahren, das für die Herstellung eines Intermediats der allgemeinen Formel (1h) bereitgestellt wird, das umfasst

a. Umsetzen der Verbindung der allgemeinen Formel (1c), worin $R^1$, $R^2$, $R^3$, $R^4$ und n wie in Anspruch 1 definiert sind,

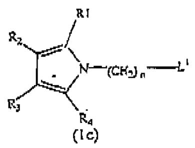
(Ic)

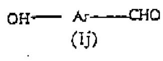
(Ij)

mit der Verbindung der allgemeinen Formel (1j), worin Ar wie in den Ansprüchen 1 und 2 definiert ist,

b. Umsetzen der Verbindung der Formel (1e), worin $R^1$, $R^2$, $R^3$, $R^4$ und n wie in Anspruch 1 definiert sind,

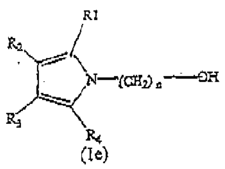
(Ie)

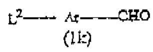
(1k)

mit Verbindung der Formel (1k), worin $L^2$ Fluor, Chlor, Brom oder Iod ist und Ar wie in den Ansprüchen 1 und 2 definiert ist.

## Revendications

**1.** Composé représenté par la formule (I) :

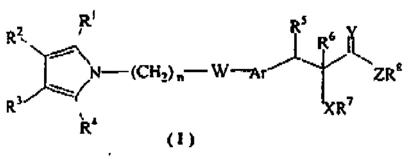
(I)

ses formes tautomères, ses stéréoisomères, ses sels pharmaceutiquement acceptables et ses solvates pharmaceutiquement acceptables, dans lequel un ou plusieurs groupes $R^1$, $R^2$, $R^3$, $R^4$ peuvent être identiques ou différents et représentent l'hydrogène, un halogène, les groupes perhalogénoalkyle, amino, nitro, cyano, formyle, amidino, guanidino, des groupes substitués ou non substitués choisis parmi

- les groupes alkyle en $C_1$-$C_{12}$ ;
- les groupes alcényle en $C_2$-$C_{12}$ linéaires ou ramifiés ;
- les groupes cycloalkyle en $C_3$-$C_7$ ;
- les groupes cycloalcényle en $C_3$-$C_7$ ;
- les groupes bicycloalkyle choisis parmi le bicyclooctane, le bicyclononane et le bicyclodécane ; un groupe bicycloalcényle choisi parmi le bicyclooctène, le bicyclononène et le bicyclodécène ;
- les groupes alcoxy en $C_1$-$C_{12}$ ;
- les groupes cycloalcoxy en $C_3$-$C_7$ ;
- les groupes aryle choisis parmi les groupes phényle, naphtyle, benzo[1,3]dioxole ou biphényle ;
- les groupes aryloxy choisis parmi les groupes phénoxy, naphtyloxy, biphényloxy ;
- les groupes aralkyle dans lesquels les groupes aryle et alkyle sont tels que définis ci-dessus ;
- les groupes aralcoxy dans lesquels le groupe aralkyle est tel que défini ci-dessus ;
- les groupes hétéroaryle choisis parmi les groupes pyridyle, thiényle, furyle, pyrrolyle, oxazolyle, thiazolyle, imidazolyle, oxadiazolyle, tétrazolyle, benzopyranyle, benzofuranyle ;
- les groupes hétéroaralkyle choisis parmi les groupes furaneméthyle, pyridineméthyle, oxazoleméthyle, oxazoléthyle ;
- les groupes hétérocyclyle choisis parmi les groupes aziridinyle, pyrrolidinyle, pipéridinyle ;
- les groupes hétérocyclylalkyle choisis parmi les groupes pyrrolidinalkyle, pipéridinalkyle, morpholinalkyle, thiomorpholinalkyle, oxazolinalkyle ;
- les groupes hétérocycloalcoxy dans lesquels le groupe hétérocyclyle et le groupe alcoxy sont tels que définis ci-dessus ;

- les groupes hétéroaryloxy dans lesquels le groupe hétéroaryle, est tel que défini ci-dessus ;
- les groupes hétéroaralcoxy dans lesquels le groupe hétéroaralkyle est tel que défini ci-dessus ;
- les groupes acyle choisis parmi les groupes acétyle, propionyle ou benzoyle ;
- les groupes acyloxy choisis parmi les groupes MeCOO, EtCOO, PhCOO ;
- les groupes acylamino choisis parmi $CH_3CONH$, $C_2H_5CONH$, $C_3H_7CONH$, $C_6H_5CONH$ ;
- les groupes monoalkylamino en $C_1$-$C_6$ ;
- les groupes dialkylamino en $C_1$-$C_6$ ;
- les groupes arylamino dans lesquels le groupe aryle est tel que défini ci-dessus ;
- les groupes aralkylamino dans lesquels le groupe aryle est tel que défini ci-dessus ;
- les groupes alcoxycarbonyle dans lesquels le groupe alcoxy est tel que défini ci-dessus ;
- les groupes aryloxycarbonyle dans lesquels le groupe aryloxy est tel que défini ci-dessus ;
- les groupes aralcoxycarbonyle dans lesquels le groupe aralcoxy est tel que défini ci-dessus ;
- les groupes hétérocycloalcoxycarbonyle dans lesquels le groupe hétérocycloalcoxy est tel que défini ci-dessus ;
- les groupes hétéroaryloxycarbonyle dans lesquels le groupe hétéroaryloxy est tel que défini ci-dessus ;
- les groupes hétéroaralcoxycarbonyle dans lesquels le groupe hétéroaralcoxy est tel que défini ci-dessus ;
- les groupes hydroxyalkyle dans lesquels le groupe alkyle est tel que défini ci-dessus ;
- les groupes aminoalkyle dans lesquels le groupe alkyle est tel que défini ci-dessus ;
- les groupes alkylaminoalkyle mono- ou disubstitués dans lesquels le groupe alkyle est tel que défini ci-dessus et le groupe substitué est choisi parmi les groupes alkyle, cycloalkyle, alcoxy, cycloalcoxy, aryle, aralkyle, aralcoxyalkyle, hétérocyclyle, hétéroaryle, hétéroaralkyle, acyle, acyloxy, hydroxyalkyle, amino, acylamino, arylamino, aminoalkyle, aryloxy, aralcoxy, alkylamino, alcoxyalkyle, alkylthio, ou thioalkyle ;
- les groupes alcoxyalkyle dans lesquels le groupe alkyle est tel que défini ci-dessus ;
- les groupes aryloxyalkyle dans lesquels le groupe alkyle est tel que défini ci-dessus ;
- les groupes aralcoxyalkyle dans lesquels le groupe alkyle est tel que défini ci-dessus ;
- les groupes alkylthio ou thioalkyle dans lesquels le groupe alkyle est tel que défini ci-dessus ;
- les groupes alcoxycarbonylamino dans lesquels le groupe alcoxycarbonyle est tel que défini ci-dessus ;
- les groupes aryloxycarbonylamino dans lesquels le groupe aryloxycarbonyle est tel que défini ci-dessus ;
- les groupes aralkyloxycarbonylamino dans lesquels le groupe aralkyloxycarbonyle est tel que défini ci-dessus ;
- les groupes aminocarbonylamino dans lesquels le groupe aminocarbonyle est tel que défini ci-dessus ;
- les groupes hydrazino, hydroxylamino ; l'acide carboxylique et ses dérivés choisis parmi les esters en $C_1$-$C_3$, dans lesquels les substituants sur $R^1$, $R^2$, $R^3$ et $R^4$ sont choisis parmi les groupes alkyle, cycloalkyle, alcoxy, cycloalcoxy, aryle, aralkyle, aralcoxyalkyle, hétérocyclyle, hétéroaryle, hétéroaralkyle, acyle, acyloxy, hydroxyalkyle, amino, acylamino, arylamino, aminoalkyle, aryloxy, aralcoxy, alkylamino, alcoxyalkyle, alkylthio, thioalkyle, dans lesquels chacun des termes est tel que défini ci-dessus ;

n est un entier valant 1 à 8 ; W représente O, S ou $NR^9$ dans lequel $R^9$ représente un groupe alkyle ou aryle tel que défini ci-dessus ; Ar représente un groupe aromatique, hétéroaromatique ou hétérocyclique simple ou condensé divalent substitué ou non substitué, chacun de ces termes étant choisi parmi ceux définis ci-dessus, les substituants sur « Ar » sont choisis parmi les groupes alkyle, alcoxy, halogéno, halogénoalkyle, halogénoalcoxy, acyle, amino, acylamino, thio linéaires ou ramifiés ou les acides carboxyliques ou sulfoniques ; $R^5$ et $R^6$ représentent à la fois l'hydrogène, ou représentent ensemble une liaison ; $R^5$ et $R^6$ peuvent aussi représenter les groupes hydroxy, alkyle, alcoxy, halogéno, acyle, aralkyle, chacun de ces termes étant choisi parmi ceux définis ci-dessus ;
X représente O ou S ; $R^7$ représente l'hydrogène, un groupe perfluoroalkyle ou des groupes choisis parmi les groupes alkyle, cycloalkyle, aryle, aralkyle, hétéroaryle, hétéroaralkyle, alcoxyalkyle, aryloxyalkyle, alcoxycarbonyle, aryloxycarbonyle, cycloalkyloxycarbonyle, alkylaminocarbonyle, arylaminocarbonyle, acyle, chacun de ces termes étant tel que défini ci-dessus ; Y représente O ou S ; Z représente l'oxygène ou $NR^{10}$, dans lequel $R^{10}$ représente l'hydrogène ou des groupes choisis parmi les groupes alkyle, aryle, aralkyle, hydroxyalkyle, aminoalkyle, hétéroaryle, hétéroaralkyle, chacun de ces termes étant choisi parmi ceux définis ci-dessus ; $R^8$ représente l'hydrogène, des groupes choisis parmi les groupes alkyle, aryle, aralkyle, hétéroaryle, hétéroaralkyle, hydroxyalkyle, alcoxyalkyle, alkylaminoalkyle, chacun de ces termes étant tel que défini ci-dessus ;
$R^{10}$ et $R^8$ peuvent former ensemble une structure de noyau cyclique substituée ou non substituée à 5 ou 6 chaînons contenant des atomes de carbone ou contenant un ou plusieurs hétéroatomes choisis parmi O, N et S, les structures cycliques étant celles formées ci-dessus, dans lesquelles, sur la structure cyclique formée par $R^8$ et $R^{10}$ pris ensemble, les substituants sont choisis parmi l'halogène, les groupes hydroxy, alkyle, oxo, aralkyle.

**2.** Composé selon la revendication 1, dans lequel Ar représente des groupes phénylène, naphtylène divalents substitués ou non substitués et les groupes substitués sont choisis parmi les groupes alkyle, alcoxy, halogéno, halogénoalkyle, halogénoalcoxy, acyle, amino, acylamino, thio linéaires ou ramifiés ou les acides carboxyliques ou sulfoniques.

**3.** Composé selon la revendication 1, qui est choisi parmi :

le (±) éthyl 3-{4-[2-(pyrrol-1-yl)éthoxy]phényl}-2-éthoxypropanoate,
le (+) éthyl 3-{4-[2-(pyrrol-1-yl)éthoxy]phényl}-2-éthoxypropanoate,
le (-) éthyl 3-{4-[2-(pyrrol-1-yl)éthoxy]phényl}-2-éthoxypropanoate,
le (±) éthyl 3-{4-[2-(2,5-diméthylpyrrol-1-yl)éthoxy]phényl)-2-éthoxypropanoate,
le (+) éthyl 3-{4-[2-(2,5-diméthylpyrrol-1-yl)éthoxy]phényl}-2-éthoxypropanoate,
le (-) éthyl 3-{4-[2-(2,5-diméthylpyrrol-1-yl)éthoxy]phényl}-2-éthoxypropanoate,
le (±) éthyl 3-{4-[2-(2,5-diisopropyl-3-phénylpyrrol-1-yl)éthoxy]phényl}-2-éthoxypropanoate,
le (+) éthyl 3-{4-[2-(2,5-diisopropyl-3-phénylpyrrol-1-yl)éthoxy]phényl}-2-éthoxypropanoate,
le (-) éthyl 3-{4-[2-(2,5-diisopropyl-3-phénylpyrrol-1-yl)éthoxy]phényl}-2-éthoxypropanoate,
le (±) éthyl 3-(4-{2-[2-isopropyl-5-(4-méthoxyphényl)pyrrol-1-yl)éthoxy}phényl)-2-éthoxypropanoate,
le (+) éthyl 3-(4-{2-[2-isopropyl-5-(4-méthoxyphényl)pyrrol-1-yl}éthoxy}phényl)-2-éthoxypropanoate,
le (-) éthyl 3-(4-{2-[2-isopropyl-5-(4-méthoxyphényl)pyrrol-1-yl)éthoxy}phényl)-2-éthoxypropanoate,
le (±) éthyl 3-(4-{2-[2-(4-fluorophényl)-5-isopropylpyrrol-1-yl]éthoxy}phényl)-2-éthoxypropanoate,
le (+) éthyl 3-(4-{2-[2-(4-fluorophényl)-5-isopropylpyrrol-1-yl]éthoxy}phényl)-2-éthoxypropanoate,
le (-) éthyl 3-(4-{2-[2-(4-fluorophényl)-5-isopropylpyrrol-1-yl]éthoxy}phényl)-2-éthoxypropanoate,
le (±) éthyl 3-(4-{2-[2-(4-fluorophényl)-5-isopropyl-3-phénylpyrrol-1-yl]éthoxy}phényl)-2-éthoxypropanoate,
le (+) éthyl 3-(4-{2-[2-(4-fluorophényl)-5-isopropyl-3-phénylpyrrol-1-yl]éthoxy}phényl)-2-éthoxypropanoate,
le (-) éthyl 3-(4-{2-[2-(4-fluorophényl)-5-isopropyl-3-phénylpyrrol-1-yl]éthoxy}phényl)-2-éthoxypropanoate,
le (±) éthyl 3-(4-{2-[2-(4-fluorophényl)-5-phénylpyrrol-1-yl]éthoxy}phényl)-2-éthoxypropanoate,
le (+) éthyl 3-(4-{2-[2-(4-fluorophényl)-5-phénylpyrrol-1-yl]éthoxy}phényl)-2-éthoxypropanoate,
le (-) éthyl 3-(4-{2-[2-(4-fluorophényl)-5-phénylpyrrol-1-yl]éthoxy}phényl)-2-éthoxypropanoate,
le (±) éthyl 3-[4-[2-[2-(2-phényl-3-carboxy-5-(4-fluorophényl)pyrrol-1-yl)éthoxy]phényl-2-éthoxypropanoate,
le (+) éthyl 3-[4-[2-[2-(2-phényl-3-carboxy-5-(4-fluorophényl)pyrrol-1-yl)éthoxy]phényl-2-éthoxypropanoate,
le (-) éthyl 3-[4-[2-[2-(2-phényl-3-carboxy-5-(4-fluorophényl)pyrrol-1-yl)éthoxylphényl-2-éthoxypropanoate,
le (±) éthyl 3-(4-{2-[2-(4-fluorophényl)-5-isopropyl-3-phényl-4-phénylcarbamoylpyrrol-1-yl]éthoxy}phényl)-2-éthoxypropanoate,
le (+) éthyl 3-(4-{2-[2-(4-fluorophényl)-5-isopropyl-3-phényl-4-phénylcarbamoylpyrrol-1-yl]éthoxy}phényl)-2-éthoxypropanoate,
le (-) éthyl 3-(4-{2-[2-(4-fluorophényl)-5-isopropyl-3-phényl-4-phénylcarbamoylpyrrol-1-yl]éthoxy}phényl)-2-éthoxypropanoate,
le (±) éthyl 3-(4-{2-[2-(4-fluorophényl)-5-isopropyl-3-phényl-4-phénylcarbamoylpyrrol-1-yl]propoxy}phényl)-2-éthoxypropanoate,
le (+) éthyl 3-(4-{3-[2-(4-fluorophényl)-5-isopropyl-3-phényl-4-phénylcarbamoylpyrrol-1-yl]propoxy}phényl)-2-éthoxypropanoate,
le (-) éthyl 3-(4-{3-[2-(4-fluorophényl)-5-isopropyl-3-phényl-4-phénylcarbamoylpyrrol-1-yl]propoxy}phényl)-2-éthoxypropanoate,
le (±) éthyl 3-(4-{2-[2-isopropyl-5-méthylpyrrol-1-yl)éthoxy]phényl}-2-éthoxypropanoate,
le (+) éthyl 3-{4-[2-(2-isopropyl-5-méthylpyrrol-1-yl)éthoxy]phényl}-2-éthoxypropanoate,
le (-) éthyl 3-{4-[2-[2-isopropyl-5-méthylpyrrol-1-yl)éthoxy]phényl}-2-éthoxypropanoate,
l'acide (±) 3-{4-[2-(pyrrol-1-yl)éthoxy]phényl}-2-éthoxypropanoïque et ses sels pharmaceutiquement acceptables,
l'acide (+) 3-{4-[2-(pyrrol-1-yl)éthoxy]phényl}-2-éthoxypropanoïque et ses sels pharmaceutiquement acceptables,
l'acide (-) 3-{4-[2-(pyrrol-1-yl)éthoxy]phényl}-2-éthoxypropanoïque et ses sels pharmaceutiquement acceptables,
l'acide (±) 3-{4-[2-(2,5-diméthylpyrrol-1-yl)éthoxy]phényl}-2-éthoxypropanoïque et ses sels pharmaceutiquement acceptables,
l'acide (+) 3-{4-[2-(2,5-diméthylpyrrol-1-yl)éthoxy]phényl}-2-éthoxypropanoïque et ses sels pharmaceutiquement acceptables,
l'acide (-) 3-{4-[2-(2,5-diméthylpyrrol-1-yl)éthoxy]phényl}-2-éthoxypropanoïque et ses sels pharmaceutiquement acceptables,
l'acide (±) 3-{4-[2-(2,5-diisopropyl-3-phénylpyrrol-1-yl)éthoxy]phényl}-2-éthoxypropanoïque et ses sels pharmaceutiquement acceptables,
l'acide (+) 3-{4-[2-(2,5-diisopropyl-3-phénylpyrrol-1-yl)éthoxy]phényl}-2-éthoxypropanoïque et ses sels pharmaceutiquement acceptables,
l'acide (-) 3-{4-[2-(2,5-diisopropyl-3-phénylpyrrol-1-yl)éthoxy]phényl}-2-éthoxypropanoïque et ses sels pharmaceutiquement acceptables,
l'acide (±) 3-(4-{2-[2-isopropyl-5-(4-méthoxyphényl)pyrrol-1-yl]éthoxy}phényl)-2-éthoxypropanoïque et ses sels pharmaceutiquement acceptables,
l'acide (+) 3-(4-{2-[2-isopropyl-5-(4-méthoxyphényl)pyrrol-1-yl]éthoxy}phényl)-2-éthoxypropanoique et ses sels pharmaceutiquement acceptables,
l'acide (-) 3-(4-{2-[2-isopropyl-5-(4-méthoxyphényl)pyrrol-1-yl]éthoxy}phényl)-2-éthoxypropanoïque et ses sels pharmaceutiquement acceptables,
l'acide (±) 3-(4-{2-[2-(4-fluorophényl)-5-isopropylpyrrol-1-yl]éthoxy}phényl)-2-éthoxypropanoïque et ses sels pharmaceutiquement acceptables,
l'acide (+) 3-(4-{2-[2-(4-fluorophényl)-5-isopropylpyrrol-1-yl]éthoxy}phényl)-2-éthoxypropanoïque et ses sels pharmaceutiquement acceptables,
l'acide (-) 3-(4-{2-[2-(4-fluorophényl)-5-isopropylpyrrol-1-yl]éthoxy}phényl)-2-éthoxypropanoïque et ses sels pharmaceutiquement acceptables,
l'acide (±) 3-(4-(2-[2-(4-fluorophényl)-5-isopropyl-3-phénylpyrrol-1-yl]éthoxy)phényl)-2-éthoxypropanoïque et ses sels pharmaceutiquement acceptables,
l'acide (+) 3-(4-(2-[2-(4-fluorophényl)-5-isopropyl-3-phénylpyrrol-1-yl]éthoxy)phényl)-2-éthoxypropanoïque et ses sels pharmaceutiquement acceptables,
l'acide (-) 3-(4-(2-[2-(4-fluorophényl)-5-isopropyl-3-phénylpyrrol-1-yl]éthoxy)phényl)-2-éthoxypropanoïque et ses sels pharmaceutiquement acceptables,
l'acide (±) 3-(4-(2-[2-(4-fluorophényl)-5-phénylpyrrol-1-yl]éthoxy)phényl-2-éthoxypropanoïque et ses sels pharmaceutiquement acceptables,
l'acide (+) 3-(4-{2-[2-(4-fluorophényl)-5-phénylpyrrol-1-yl]éthoxy}phényl)-2-éthoxypropanoïque et ses sels pharmaceutiquement acceptables,
l'acide (-) 3-(4-{2-[2-(4-fluorophényl)-5-phénylpyrrol-1-yl]éthoxy}phényl)-2-éthoxypropanoïque et ses sels pharmaceutiquement acceptables,
l'acide (±) 3-[4-[2-(2-phényl-3-carbéthoxy-5-(4-fluorophényl)pyrrol-1-yl)éthoxy]phényl]-2-éthoxypropanoïque et ses sels pharmaceutiquement acceptables,
l'acide (+) 3-[4-[2-(2-phényl-3-carbéthoxy-5-(4-fluorophényl)pyrrol-1-yl)éthoxy]phényl]-2-éthoxypropanoïque et ses sels pharmaceutiquement acceptables,
l'acide (-) 3-[4-[2-(2-phényl-3-carbéthoxy-5-(4-fluorophényl)pyrrol-1-yl)éthoxy]phényl]-2-éthoxypropanoïque et ses sels pharmaceutiquement acceptables,
l'acide (±) 3-(4-{2-[2-(4-fluorophényl)-5-isopropyl-3-phényl-4-phénylcarbamoylpyrrol-1-yl]éthoxy}phényl)-2-éthoxypropanoïque et ses sels pharmaceutiquement acceptables,
l'acide (+) 3-(4-{2-[2-(4-fluorophényl)-5-isopropyl-3-phényl-4-phénylcarbamoylpyrrol-1-yl]éthoxy}phényl)-2-éthoxypropanoïque et ses sels pharmaceutiquement acceptables,
l'acide (-) 3-(4-{2-[2-(4-fluorophényl)-5-isopropyl-3-phényl-4-phénylcarbamoylpyrrol-1-yl]éthoxy}phényl)-2-éthoxypropanoïque et ses sels pharmaceutiquement acceptables,
l'acide (±) 3-(4-{3-[2-(4-fluorophényl)-5-isopropyl-3-phényl-4-phénylcarbamoylpyrrol-1-yl]propoxy}phényl)-2-éthoxypropanoïque et ses sels pharmaceutiquement acceptables,
l'acide (+) 3-(4-{3-[2-(4-fluorophényl)-5-isopropyl-3-phényl-4-phénylcarbamoylpyrrol-1-yl]propoxy}phényl)-2-éthoxypropanoïque et ses sels pharmaceutiquement acceptables,
l'acide (-) 3-(4-{3-[2-(4-fluorophényl)-5-isopropyl-3-phényl-4-phénylcarbamoylpyrrol-1-yl]propoxy}phényl)-2-éthoxypropanoïque et ses sels pharmaceutiquement acceptables,
l'acide (±) 3-{4-[2-(2-isopropyl-5-méthylpyrrol-1-yl)éthoxy]phényl}-2-éthoxypropanoïque et ses sels pharmaceutiquement acceptables,
l'acide (+) 3-{4-[2-(2-isopropyl-5-méthylpyrrol-1-yl)éthoxy]phényl}-2-éthoxypropanoïque et ses sels pharmaceutiquement acceptables,
l'acide (-) 3-{4-[2-(2-isopropyl-5-méthylpyrrol-1-yl)éthoxy]phényl}-2-éthoxypropanoïque et ses sels pharmaceutiquement acceptables.

**4.** Procédé pour préparer un composé de formule (I) selon la revendication 1 choisi parmi ;

a. la réaction d'un composé de formule (la),

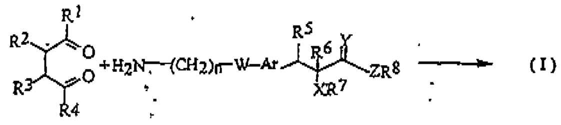

(1a) (1b)

dans laquelle tous les symboles sont tels que définis ci-dessus, avec un composé de formule (1b) qui peut être racémique ou chiral, formule dans laquelle tous les symboles sont tels que définis ci-dessus pour donner un composé de formule (I) dans laquelle tous les symboles sont tels que définis ci-dessus ;

b. la réaction d'un composé de formule (1c),

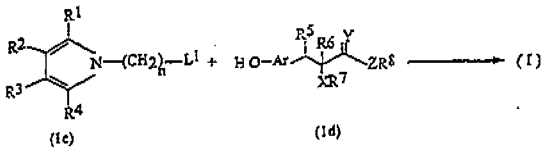

(1c) (1d)

dans laquelle tous les symboles sont tels que définis ci-dessus et $L^1$ représente un groupe labile, avec un composé de formule (1d) qui peut être racémique ou chiral, formule dans laquelle tous les symboles sont tels que définis ci-dessus ;

c. la réaction du composé de formule (1e),

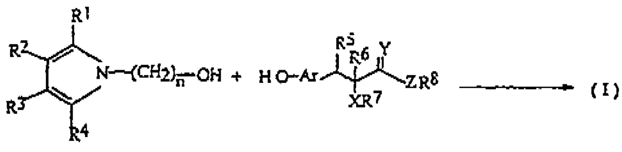

(1c) (1d)

dans laquelle tous les symboles sont tels que définis ci-dessus avec un composé de formule générale (1d) qui peut être racémique ou chiral, défini ci-dessus ;

d. la réaction d'un composé de formule (1f)

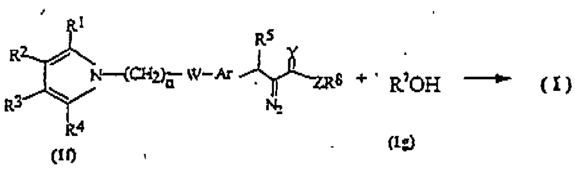

(1f) (1g)

dans laquelle tous les symboles sont tels que définis ci-dessus avec un alcool de formule (1g), dans laquelle $R^7$ est tel que défini ci-dessus, sauf H, pour produire un composé de formule (I), pour produire un composé de formule générale (I), dans laquelle tous les symboles sont tels que définis ci-dessus et X représente des atomes de « O » ;

e. la réaction d'un composé de formule générale (1h),

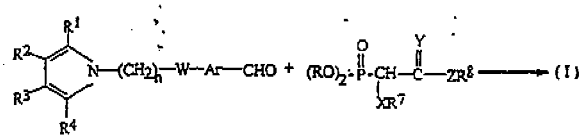

(1h) (1i)

dans laquelle tous les symboles sont tels que définis ci-dessus, avec un composé de formule (Ii) qui peut être chiral ou racémique, formule dans laquelle tous les symboles sont tels que définis ci-dessus et R représente

un groupe alkyle en $C_1$-$C_8$ pour donner un composé de formule (I), dans laquelle tous les symboles sont tels que définis ci-dessus et $R^5$ et $R^6$ forment ensemble une liaison.

**5.** Procédé selon la revendication 4, comprenant la réalisation d'une ou plusieurs étapes facultatives suivantes comprenant :

i. la transformation d'un composé de formule (I) en un autre composé de formule (I) ;
ii. l'élimination de tout groupe protecteur ;
iii. la résolution du mélange racémique en énantiomères purs grâce aux procédés connus et
iv. la préparation d'un sel pharmaceutiquement acceptable du composé de formule (I) et/ou d'un de ses solvates pharmaceutiquement acceptables.

**6.** Composé de formule générale (I), ses formes tautomères, ses stéréoisomères, ses polymorphes, ses sels pharmaceutiquement acceptables et ses solvates pharmaceutiquement acceptables pour préparer un médicament.

**7.** Composition pharmaceutique qui comprend un composé de formule générale (I), leurs formes tautomères, leurs stéréoisomères, leurs polymorphes, leurs sels pharmaceutiquement acceptables, leurs solvates pharmaceutiquement acceptables et les compositions pharmaceutiquement acceptables les contenant.

**8.** Composition pharmaceutique qui comprend un composé de formule générale (I), selon la revendication 7, sous la forme d'un comprimé, d'une gélule, d'une poudre, d'un sirop, d'une solution ou d'une suspension.

**9.** Utilisation d'un composé de formule (I) selon la revendication 1 ou la revendication 2 ou composition pharmaceutique selon les revendications 7 et 8, pour fabriquer un médicament destiné au traitement et/ou à la prophylaxie de l'hyperglycémie, de l'hyperlipidémie, de l'hypertension, des maladies cardiovasculaires et de certains troubles alimentaires.

**10.** Intermédiaire défini par la formule (1h),

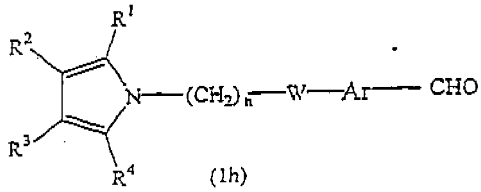

(1h)

dans laquelle un ou plusieurs groupes $R^1$, $R^2$, $R^3$, $R^4$, W, Ar et n sont tels que définis dans la revendication 1, contenant facultativement une ou plusieurs doubles liaisons et contenant facultativement un ou plusieurs hétéroatomes choisis parmi O, N ou S.

**11.** Procédé prévu pour la préparation d'un intermédiaire de formule générale (1h), qui comprend :

a. la réaction du composé de formule générale (1c) dans laquelle $R^1$, $R^2$, $R^3$, $R^4$ et n sont tels que définis dans la revendication 1,

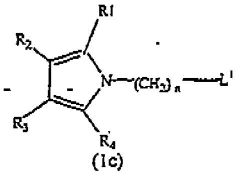

(1c)

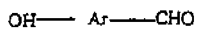

(1j)

avec le composé de formule générale (lj), dans laquelle Ar est tel que défini dans les revendications 1 et 2,
b. la réaction du composé de formule (1e), dans laquelle $R^1$, $R^2$, $R^3$, $R^4$ et n sont tels que définis dans la revendication 1,

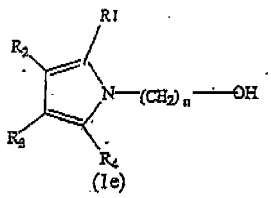

(Ie)

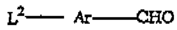

(1k)

avec le composé de formule générale (1k), dans laquelle $L^2$ est le fluor, le chlore, le brome ou l'iode et Ar est tel que défini dans les revendications 1 et 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9802159 A
- WO 9736579 A
- WO 9725042 A
- EP 0753298 A
- US 5306726 A
- US 5985884 A
- US 6054453 A
- EP 903343 A
- WO 9119702 A
- WO 9401420 A
- WO 9413650 A
- WO 9503038 A
- WO 9517394 A
- WO 9604260 A
- WO 9604261 A
- WO 9633998 A
- WO 9828534 A
- WO 9908501 A
- WO 9916758 A
- WO 9919313 A
- WO 9920614 A
- WO 0023417 A
- WO 0023445 A
- WO 0023451 A
- WO 95177394 A
- WO 00540414 A


### Non-patent literature cited in the description


- *New Engl. J. Med.,* 1976, vol. 295, 369-377
- **STAMPFER et al.** *N. Engl. J. Med.,* 1991, vol. 325, 373-381
- **MILLER.** *Br. Med. J.,* 1981, vol. 282, 1741-1744
- **PICARDO et al.** *Arteriosclerosis,* 1986, vol. 6, 434-441
- **MACIKINNON et al.** *J. Biol. Chem.,* 1986, vol. 261, 2548-2552
- *Endocrinology,* 1994, vol. 135, 798-800
- *Cell,* 1995, vol. 83, 803-812
- *Trends Endocrine. Metabolism,* 1993, vol. 4, 291-296
- *Current. Biol.,* 1995, vol. 5, 618-621
- *Cell,* 1994, vol. 79, 1147-1156
- *Cell,* 1996, vol. 79, 377-389
- *Molecular Cell,* 1998, 465-470
- *Carcinogenesis,* 1998, 1949-1953
- *Proc. Natl. Acad. Sci.,* 1997, vol. 94, 237-241
- *Cancer Research,* 1998, vol. 58, 3344-3352
- *Proc. Natl. Acad. Sci.,* 1996, vol. 93, 5793-5796
- **PAAL C.** *Ber.,* vol. 18, 367
- **KNORR, L.** *Ber.,* vol. 18, 299
- Principles of Asymmetric synthesis. Tetrahedron series. vol. 14, 311-316
- **JAQUES et al.** Enantiomers, Racemates and Resolution. Wiley Interscience, 1981
- Remington: the Science and Practice of Pharmacy. 1995
- **WIELAND, O.** Methods of Enzymatic analysis. 1963, 211-214
- **TRINDER, P.** *Ann. Clin. Biochem.,* 1969, vol. 6, 24-27
- **PETIT, D. ; BONNEFIS, M. T. ; REY, C ; INFANTE, R.** Effects of ciprofibrate on liver lipids and lipoprotein synthesis in normal and hyperlipidemic rats. *Atherosclerosis,* 1988, vol. 74, 215-225